# EUROPÄISCHE PATENTSCHRIFT

(11) **EP 1 407 034 B1**
(45) Veröffentlichungstag und Bekanntmachung des Hinweises auf die Patenterteilung: **07.09.2016**
(21) Anmeldenummer: 02760212.7
(22) Anmeldetag: 02.07.2002
(51) Int. Cl.: C12N 15/90, C12N 15/82, C12N 5/00, A01K 67/027

(54) **REKOMBINATIONSSYSTEME UND VERFAHREN ZUM ENTFERNEN VON NUKLEINSÄURESEQUENZEN AUS DEM GENOM EUKARYOTISCHER ORGANISMEN**
RECOMBINATION SYSTEMS AND A METHOD FOR REMOVING NUCLEIC ACID SEQUENCES FROM THE GENOME OF EUKARYOTIC ORGANISMS
SYSTEMES DE RECOMBINAISON ET PROCEDES POUR RETIRER DES SEQUENCES D'ACIDE NUCLEIQUE DU GENOME D'ORGANISMES EUCARYOTES

(30) Priorität: 04.07.2001 DE 10131786
(43) Veröffentlichungstag der Anmeldung: 14.04.2004
(73) Patentinhaber: SunGene GmbH, 06466 Gatersleben (DE); Institut F. Pflanzengenetik, 06466 Gatersleben (DE)
(72) Erfinder: PUCHTA, Holger, 06449 Aschersleben (DE); BIESGEN, Christian, 06484 Quedlinburg (DE)
(74) Vertreter: BASF IP Association
(86) Internationale Anmeldenummer: PCT/EP2002/007281
(87) Internationale Veröffentlichungsnummer: WO 2003/004659

(56) Entgegenhaltungen:
- US-A- 5 527 695
- US-A- 5 792 632
- PUCHTA H. ET AL.: "Two different but related mechanisms are used in plants for the repair of genomic double-strand breaks by homologous recombination" PROCEEDINGS OF THE NATIONAL ACADEMY OF SCIENCES, USA, Bd. 93, Mai 1996 (1996-05), Seiten 5055-5060, XP002236852
- SIEBERT R. ET AL.: "Efficient Repair of Genomic Double-Strand Breaks by Homologous Recombination between Directly Repeated Sequences in the Plant Genome" THE PLANT CELL, Bd. 14, Mai 2002 (2002-05), Seiten 1121-1131, XP002236853
- AGGARWAL A.K. ET AL.: "Novel site-speific DNA endonucleases" CURRENT OPINION IN STRUCTURAL BIOLOGY, Bd. 8, 1998, Seiten 19-25, XP002236854
- DONOHO GREG ET AL: "Analysis of gene targeting and intrachromosomal homologous recombination stimulated by genomic doubler-strand breaks in mouse embryonic stem cells", MOLECULAR AND CELLULAR BIOLOGY, AMERICAN SOCIETY FOR MICROBIOLOGY, WASHINGTON, US, vol. 18, no. 7, 1 July 1998 (1998-07-01), pages 4070-4078, XP002427409, ISSN: 0270-7306
- PUCHTA H: "Removing selectable marker genes: taking the shortcut.", TRENDS IN PLANT SCIENCE JUL 2000 LNKD- PUBMED:10871898, vol. 5, no. 7, July 2000 (2000-07), pages 273-274, ISSN: 1360-1385
- ZUBKO ELENA ET AL: "Intrachromosomal recombination between attP regions as a tool to remove selectable marker genes from tobacco transgenes", NATURE BIOTECHNOLOGY, NATURE PUBLISHING GROUP, NEW YORK, NY, US, vol. 18, no. 4, 1 April 2000 (2000-04-01), pages 442-445, XP002198606, ISSN: 1087-0156, DOI: DOI:10.1038/74515
- EPINAT J-C ET AL: "A novel engineered meganuclease induces homologous recombination in yeast and mammalian cells", NUCLEIC ACIDS RESEARCH, OXFORD UNIVERSITY PRESS, SURREY, GB, vol. 31, no. 11, 1 June 2003 (2003-06-01), pages 2952-2962, XP002248751, ISSN: 0305-1048, DOI: DOI:10.1093/NAR/GKG375
- BRENNEMAN M. ET AL.: "Stimulation of intrachromosomal homologous recombination in human cells by electroporation with site-specific endonucleases.", PNAS, vol. 93, no. 8, April 1996 (1996-04), pages 3608-3612, XP002233940,
- JOHNSON, R.D. & JASIN, M.: BIOCHEMICAL SOCIETY TRANSACTIONS, vol. 29, no. 2, May 2001 (2001-05), pages 196-201, XP008110938,
- TRAVER B E ET AL: "Homing endonucleases catalyze double-stranded DNA breaks and somatic transgene excision in Aedes aegypti", INSECT MOLECULAR BIOLOGY, vol. 18, no. 5, October 2009 (2009-10), pages 623-633, ISSN: 0962-1075
- MENGISTE T ET AL: 'PROSPECTS FOR THE PRECISE ENGINEERING OF PLANT GENOMES BY HOMOLOGOUS RECOMBINATION' BIOLOGICAL CHEMISTRY, WALTER DE GRUYTER GMBH & CO, BERLIN, DE Bd. 380, Nr. 7/8, 01 Januar 1999, Seiten 749 - 758, XP000885763 DOI: 10.1515/BC.1999.095 ISSN: 1431-6730

### SEQUENZPROTOKOLL

<110> SunGene GmbH & Co. KGaA
<120> Systeme und Verfahren zum Entfernen von Nukleinsäuresequenzen aus der chromosomalen DNA eukaryotischer Organismen
<130> NAE502_2001
<140>
   <141>
<160>
<170> PatentIn Ver. 2.1
<210>
   <211> 78B
   <212> DNA
   <213> Saccharomyces cerevisiae
<220>
   <221> CDS
   <222> (62)..(766)
   <223> open reading frame coding for I-SceI
<400> 1
<210>
   <211> 235
   <212> PRT
   <213> Saccharomyces cerevisiae
<400> 2
<210> 3
   <211> 746
   <212> DNA
   <213> Chlamydomonas applanata
<220>
   <221> CDS
   <222> (54)..(737)
   <223> open reading frame of I-ChuI with nuclear location signal
<220>
   <221> misc_feature
   <222> (54)..(83)
   <223> coding for nuclear location signal
<400> 3
   ctcgagtacc tagaatacaa agaagaggaa gaagaaactc tatagaagaa gcc atg     56
                                                              Met
                                                               1
   ggt cca aag aaa aag aga aag gtt atc atg tca tta aca caa caa caa    104
   Gly Pro Lys Lys Lys Arg Lys Val Ile Met Ser Leu Thr Gln Gln Gln
               5                   10                  15
   aaa gac tta att ttc gga tct cta ctg ggt gat gga aat tta caa act    152
   Lys Asp Leu Ile Phe Gly Ser Leu Leu Gly Asp Gly Asn Leu Gln Thr
           20                  25                  30
   ggt tca gta ggt agg act tgg cgc tat cga gcg ctc cat aaa agt gag    200
   Gly Ser Val Gly Arg Thr Trp Arg Tyr Arg Ala Leu His Lys Ser Glu
       35                  40                  45
   cat cag aca tac tta ttt cat aag tat gaa atc tta aag ccg ctt tgt    248
   His Gln Thr Tyr Leu Phe His Lys Tyr Glu Ile Leu Lys Pro Leu Cys
   50                  55                  60                  65
   ggc gaa aat act ctc cca aca gaa agt ata gtg ttc gac gaa aga aca    296
   Gly Glu Asn Thr Leu Pro Thr Glu Ser Ile Val Phe Asp Glu Arg Thr
                   70                  75                  80
   aac aag gag gtt aaa cgt tgg ttt ttc aac aca tta acc aat cct tcc    344
   Asn Lys Glu Val Lys Arg Trp Phe Phe Asn Thr Leu Thr Asn Pro Ser
               85                  90                  95
   tta aaa ttc ttc gca gac atg ttc tac aca tat gac caa aac aca caa    392
   Leu Lys Phe Phe Ala Asp Met Phe Tyr Thr Tyr Asp Gln Asn Thr Gln
           100                 105                 110
   aaa tgg gtt aaa gat gta cct gta aag gtt caa aca ttc tta act cct    440
   Lys Trp Val Lys Asp Val Pro Val Lys Val Gln Thr Phe Leu Thr Pro
       115                 120                 125
   caa gct tta gca tac ttt tat ata gac gat gga gcg tta aaa tgg ctt    488
   Gln Ala Leu Ala Tyr Phe Tyr Ile Asp Asp Gly Ala Leu Lys Trp Leu
   130                 135                 140                 145
   aat aag tct aac gct atg caa att tgt act gaa agt ttc agt caa ggg    536
   Asn Lys Ser Asn Ala Met Gln Ile Cys Thr Glu Ser Phe Ser Gln Gly
                   150                 155                 160
   ggc acg att cgg atc caa aaa gca cta aaa acg ctc tat aat att gat    584
   Gly Thr Ile Arg Ile Gln Lys Ala Leu Lys Thr Leu Tyr Asn Ile Asp
               165                 170                 175
   aca acg ttg aca aaa aaa act cta caa gac ggc aga att ggc tat cgt    632
   Thr Thr Leu Thr Lys Lys Thr Leu Gln Asp Gly Arg Ile Gly Tyr Arg
           180                 185                 190
   ata gct att cct gaa gcc agt agc ggt gct ttt cgt gaa gtc att aaa    680
   Ile Ala Ile Pro Glu Ala Ser Ser Gly Ala Phe Arg Glu Val Ile Lys
       195                 200                 205
   cct ttt cta gtt gat tgt atg aga tac aaa gtt tct gat ggc aat aaa    728
   Pro Phe Leu Val Asp Cys Met Arg Tyr Lys Val Ser Asp Gly Asn Lys
   210                 215                 220                 225
   ggc cac ctt tagctcgag                                              746
   Gly His Leu
<210> 4
   <211> 228
   <212> PRT
   <213> Chlamydomonas applanata
<400> 4
   Met Gly Pro Lys Lys Lys Arg Lys Val Ile Met Ser Leu Thr Gln Gln
   1               5                   10                  15
   Gln Lys Asp Leu Ile Phe Gly Ser Leu Leu Gly Asp Gly Asn Leu Gln
               20                  25                  30
   Thr Gly Ser Val Gly Arg Thr Trp Arg Tyr Arg Ala Leu His Lys Ser
           35                  40                  45
   Glu His Gln Thr Tyr Leu Phe His Lys Tyr Glu Ile Leu Lys Pro Leu
       50                  55                  60
   Cys Gly Glu Asn Thr Leu Pro Thr Glu Ser Ile Val Phe Asp Glu Arg
   65                  70                  75                  80
   Thr Asn Lys Glu Val Lys Arg Trp Phe Phe Asn Thr Leu Thr Asn Pro
                   85                  90                  95
   Ser Leu Lys Phe Phe Ala Asp Met Phe Tyr Thr Tyr Asp Gln Asn Thr
               100                 105                 110
   Gln Lys Trp Val Lys Asp Val Pro Val Lys Val Gln Thr Phe Leu Thr
           115                 120                 125
   Pro Gln Ala Leu Ala Tyr Phe Tyr Ile Asp Asp Gly Ala Leu Lys Trp
       130                 135                 140
   Leu Asn Lys Ser Asn Ala Met Gln Ile Cys Thr Glu Ser Phe Ser Gln
   145                 150                 155                 160
   Gly Gly Thr Ile Arg Ile Gln Lys Ala Leu Lys Thr Leu Tyr Asn Ile
                   165                 170                 175
   Asp Thr Thr Leu Thr Lys Lys Thr Leu Gln Asp Gly Arg Ile Gly Tyr
               180                 185                 190
   Arg Ile Ala Ile Pro Glu Ala Ser Ser Gly Ala Phe Arg Glu Val Ile
           195                 200                 205
   Lys Pro Phe Leu Val Asp Cys Met Arg Tyr Lys Val Ser Asp Gly Asn
       210                 215                 220
   Lys Gly His Leu
   225
<210> 5
   <211> 582
   <212> DNA
   <213> Chlamydomonas reinhardtii
<220>
   <221> CDS
   <222> (55)..(573)
   <223> openreading frame coding for I-CreI with nuclear location signal
<220>
   <221> misc_feature
   <222> (55)..(84)
   <223> coding for nuclear location signal
<400> 5
   ctcgagtacc tagaatacaa agaagaggaa gagaaacctc taccagaaga agcc atg    57
                                                                Met
                                                                1
   ggt cca aag aaa aag aga aag gtt atc atg aat aca aaa tat aat aaa   105
   Gly Pro Lys Lys Lys Arg Lys Val Ile Met Asn Thr Lys Tyr Asn Lys
                   5                   10                  15
   gag ttc tta ctc tac tta gca ggg ttt gta gac ggt gac ggt agc ata   153
   Glu Phe Leu Leu Tyr Leu Ala Gly Phe Val Asp Gly Asp Gly Ser Ile
               20                  25                  30
   atc gct caa att aag cct aat cag tct tat aaa ttt aag cat cag cta   201
   Ile Ala Gln Ile Lys Pro Asn Gln Ser Tyr Lys Phe Lys His Gln Leu
           35                  40                  45
   tca ctc gcg ttc caa gtc acg caa aag aca cag aga cgt tgg ttt tta   249
   Ser Leu Ala Phe Gln Val Thr Gln Lys Thr Gln Arg Arg Trp Phe Leu
       50                  55                  60                  65
   gac aaa tta gtg gat gaa att ggg gtt ggt tat gta aga gat agg ggt   297
   Asp Lys Leu Val Asp Glu Ile Gly Val Gly Tyr Val Arg Asp Arg Gly
                       70                  75                  80
   agc gtt tcg gat tat att cta agc gaa atc aag cct ttg cat aat ttt   345
   Ser Val Ser Asp Tyr Ile Leu Ser Glu Ile Lys Pro Leu His Asn Phe
                   85                  90                  95
   tta aca caa cta caa cct ttt cta aaa cta aaa caa aaa caa gca aat   393
   Leu Thr Gln Leu Gln Pro Phe Leu Lys Leu Lys Gln Lys Gln Ala Asn
               100                 105                 110
   tta gtt tta aaa att att gaa caa ctt ccg tca gca aaa gaa tcc ccg   441
   Leu Val Leu Lys Ile Ile Glu Gln Leu Pro Ser Ala Lys Glu Ser Pro
           115                 120                 125
   gac aaa ttc tta gaa gtt tgt aca tgg gtg gat caa att gca gct ctg   489
   Asp Lys Phe Leu Glu Val Cys Thr Trp Val Asp Gln Ile Ala Ala Leu
       130                 135                 140                 145
   aat gat tcg aag acg cgt aaa aca act tct gaa acc gtt cgt gct gtg   537
   Asn Asp Ser Lys Thr Arg Lys Thr Thr Ser Glu Thr Val Arg Ala Val
                   150                 155                 160
   cta gac agt tta agt gaa aaa aag aaa tcg tcc ccg tagctcgag         582
   Leu Asp Ser Leu Ser Glu Lys Lys Lys Ser Ser Pro
               165                 170
<210> 6
   <211> 173
   <212> PRT
   <213> Chlamydomonas reinhardtii
<400> 6
   Met Gly Pro Lys Lys Lys Arg Lys Val Ile Met Asn Thr Lys Tyr Asn
   1 5 10 15
   Lys Glu Phe Leu Leu Tyr Leu Ala Gly Phe Val Asp Gly Asp Gly Ser
   20 25 30
   Ile Ile Ala Gln Ile Lys Pro Asn Gln Ser Tyr Lys Phe Lys His Gln
   35 40 45
   Leu Ser Leu Ala Phe Gln Val Thr Gln Lys Thr Gln Arg Arg Trp Phe
   50 55 60
   Leu Asp Lys Leu Val Asp Glu Ile Gly Val Gly Tyr Val Arg Asp Arg
   65 70 75 80
   Gly Ser Val Ser Asp Tyr Ile Leu Ser Glu Ile Lys Pro Leu His Asn
   85 90 95
   Phe Leu Thr Gln Leu Gln Pro Phe Leu Lys Leu Lys Gln Lys Gln Ala
   100 105 110
   Asn Leu Val Leu Lys Ile Ile Glu Gln Leu Pro Ser Ala Lys Glu Ser
   115 120 125
   Pro Asp Lys Phe Leu Glu Val Cys Thr Trp Val Asp Gln Ile Ala Ala
   130 135 140
   Leu Asn Asp Ser Lys Thr Arg Lys Thr Thr Ser Glu Thr Val Arg Ala
   145 150 155 160
   Val Leu Asp Ser Leu Ser Glu Lys Lys Lys Ser Ser Pro
   165 170
<210> 7
   <211> 546
   <212> DNA
   <213> Chlamydomonas segnis
<220>
   <221> CDS
   <222> (52)..(537)
   <223> open readings frame coding for I-CpaI with nuclear location signal
<220>
   <221> misc_feature
   <222> (52)..(81)
   <223> coding for nuclear location signal
<400> 7
   ctcgagtacc tagaatacaa gaagagaaga agaacctcta cagaagaagc c atg ggt  57
                                                            Met Gly
                                                              1
   cca aag aaa aag aga aag gtt atc atg gac att aat cct caa tgg att  105
   Pro Lys Lys Lys Arg Lys Val Ile Met Asp Ile Asn Pro Gln Trp Ile
           5                   10                  15
   aca ggt ttc gta gat ggg gaa ggt tgt ttt agt gta agt att ctt aga  153
   Thr Gly Phe Val Asp Gly Glu Gly Cys Phe Ser Val Ser Ile Leu Arg
       20                  25                  30
   aat aat tcg ttg cgc tat ggc cat cag ctt caa cca gaa ttc gta gtg  201
   Asn Asn Ser Leu Arg Tyr Gly His Gln Leu Gln Pro Glu Phe Val Val
   35                  40                  45                  50
   acc caa cat aaa tta gat gca aat gtt tta tat gca tta aaa gac tac  249
   Thr Gln His Lys Leu Asp Ala Asn Val Leu Tyr Ala Leu Lys Asp Tyr
                   55                  60                  65
   ttt aaa gtt gga tca gtc gtt gtg aat cat ggg gaa cgg ctt tgc tat  297
   Phe Lys Val Gly Ser Val Val Val Asn His Gly Glu Arg Leu Cys Tyr
               70                  75                  80
   aaa gtc aaa aat att gat cac ttt ata acc gtc att ata cca ttt ttc  345
   Lys Val Lys Asn Ile Asp His Phe Ile Thr Val Ile Ile Pro Phe Phe
           85                  90                  95
   gaa aaa cat gag cta aaa aca aaa aga aga att gaa ttt ctt cga ttt  393
   Glu Lys His Glu Leu Lys Thr Lys Arg Arg Ile Glu Phe Leu Arg Phe
       100                 105                 110
   cga aaa atc tgc ttg ctg tta aaa gca ggt aga cat tta gaa tcg cag  441
   Arg Lys Ile Cys Leu Leu Leu Lys Ala Gly Arg His Leu Glu Ser Gln
   115                 120                 125                 130
   gaa gga ttc gag aaa gtg ttg gat tta gca aaa aaa ctc cgt atc aat  489
   Glu Gly Phe Glu Lys Val Leu Asp Leu Ala Lys Lys Leu Arg Ile Asn
                   135                 140                 145
   gag aaa aac tac cag gaa tct atc aaa cgt ttt gaa gaa act ggc gag  537
   Glu Lys Asn Tyr Gln Glu Ser Ile Lys Arg Phe Glu Glu Thr Gly Glu
               150                 155                 160
   taactcgag                                                         546
<210> 8
   <211> 162
   <212> PRT
   <213> Chlamydomonas segnis
<400> 8
   Met Gly Pro Lys Lys Lys Arg Lys Val Ile Met Asp Ile Asn Pro Gln
   1 5 10 15
   Trp Ile Thr Gly Phe Val Asp Gly Glu Gly Cys Phe Ser Val Ser Ile
   20 25 30
   Leu Arg Asn Asn Ser Leu Arg Tyr Gly His Gln Leu Gln Pro Glu Phe
   35 40 45
   Val Val Thr Gln His Lys Leu Asp Ala Asn Val Leu Tyr Ala Leu Lys
   50 55 60
   Asp Tyr Phe Lys Val Gly Ser Val Val Val Asn His Gly Glu Arg Leu
   65 70 75 80
   Cys Tyr Lys Val Lys Asn Ile Asp His Phe Ile Thr Val Ile Ile Pro
   85 90 95
   Phe Phe Glu Lys His Glu Leu Lys Thr Lys Arg Arg Ile Glu Phe Leu
   100 105 110
   Arg Phe Arg Lys Ile Cys Leu Leu Leu Lys Ala Gly Arg His Leu Glu
   115 120 125
   Ser Gln Glu Gly Phe Glu Lys Val Leu Asp Leu Ala Lys Lys Leu Arg
   130 135 140
   Ile Asn Glu Lys Asn Tyr Gln Glu Ser Ile Lys Arg Phe Glu Glu Thr
   145 150 155 160
   Gly Glu
<210> 9
   <211> 793
   <212> DNA
   <213> Chlamydomonas segnis
<220>
   <221> CDS
   <222> (53)..(784)
   <223> open reading frame coding for T-CpaII with nuclear location signal
<220>
   <221> misc_feature
   <222> (53)..(82)
   <223> coding for nuclear location signal
<400> 9
   ctcgagtacc tagaaacaaa gaagaggaag aagaaactct acagaagaag cc atg ggt  58
                                                            Met Gly
                                                              1
   cca aag aaa aag aga aag gtt atc atg acc gat tct aaa tct aga aac  106
   Pro Lys Lys Lys Arg Lys Val Ile Met Thr Asp Ser Lys Ser Arg Asn
               5                  10                  15
   aac aat aat ttt tta agc aat aat ctt tta cct ttg acc gat gac gag  154
   Asn Asn Asn Phe Leu Ser Asn Asn Leu Leu Pro Leu Thr Asp Asp Glu
       20                  25                  30
   aag gct tta att gcg ggg aca ctt tta ggg gat gct cat att caa aag  202
   Lys Ala Leu Ile Ala Gly Thr Leu Leu Gly Asp Ala His Ile Gln Lys
   35                  40                  45                  50
   cgt ggt gat agc tat agg cta aaa ata gct cat ggc ttg gat cat gaa  250
   Arg Gly Asp Ser Tyr Arg Leu Lys Ile Ala His Gly Leu Asp His Glu
                   55                  60                  65
   gag ctt gtc gtc tgg aag tat aac cgt tta atc agg ttg tgt caa aca  298
   Glu Leu Val Val Trp Lys Tyr Asn Arg Leu Ile Arg Leu Cys Gln Thr
               70                  75                  80
   aca caa ccc cca agg gtg gaa acc tac tca aca aag tta aag tct ggc  346
   Thr Gln Pro Pro Arg Val Glu Thr Tyr Ser Thr Lys Leu Lys Ser Gly
           85                  90                  95
   gta ttg cct caa ggg gtt gtt ttc tat acc tcg tcc gga aag tat tta  394
   Val Leu Pro Gln Gly Val Val Phe Tyr Thr Ser Ser Gly Lys Tyr Leu
       100                 105                 110
   aaa gag act tat gac ctt ttt tat aaa caa act gca gac ggt cgg agg  442
   Lys Glu Thr Tyr Asp Leu Phe Tyr Lys Gln Thr Ala Asp Gly Arg Arg
   115                 120                 125                 130
   gta aaa aca ata aca cag gag ttg atc gac agt tta ccc aag cat cca  490
   Val Lys Thr Ile Thr Gln Glu Leu Ile Asp Ser Leu Pro Lys His Pro
                   135                 140                 145
   ttg gtc tta gca gcc ttt ttt atg gac gat ggt agt gtt cgg tcc gac  538
   Leu Val Leu Ala Ala Phe Phe Met Asp Asp Gly Ser Val Arg Ser Asp
               150                 155                 160
   tgt tat tca gga aag att gca acg cca ggg ttt gct ggt aaa gaa gaa  586
   Cys Tyr Ser Gly Lys Ile Ala Thr Pro Gly Phe Ala Gly Lys Glu Glu
           165                 170                 175
   agc cag ttg ttg tgt aac tat cta cac agt tgg gat gtt caa gca aac      634
   Ser Gln Leu Leu Cys Asn Tyr Leu His Ser Trp Asp Val Gln Ala Asn
       180                 185                     190
   gta gtt gct cat aaa aaa gca aac aat cag tat tac att ggg ctc cca      682
   Val Val Ala His Lys Lys Ala Asn Asn Gln Tyr Tyr Ile Gly Leu Pro
   195                 200                 205                 210
   gca aaa aca ttt ggt cgc ttt att aac att att gaa ccc tac gtt aga      730
   Ala Lys Thr Phe Gly Arg Phe Ile Asn Ile Ile Glu Pro Tyr Val Arg
                   215                 220                 225
   gaa gtt cct gct tta tgt tat aaa tta aac gaa tca aga aaa ccc cgt      778
   Glu Val Pro Ala Leu Cys Tyr Lys Leu Asn Glu Ser Arg Lys Pro Arg
               230                 235                 240
   aac gac tgactcgag                                                    793
   Asn Asp
<210> 10
   <211> 244
   <212> PRT
   <213> Chlamydomonas segnis
<400> 10
   Met Gly Pro Lys Lys Lys Arg Lys Val Ile Met Thr Asp Ser Lys Ser
   1 5 10 15
   Arg Asn Asn Asn Asn Phe Leu Ser Asn Asn Leu Leu Pro Leu Thr Asp
   20 25 30
   Asp Glu Lys Ala Leu Ile Ala Gly Thr Leu Leu Gly Asp Ala His Ile
   35 40 45
   Gln Lys Arg Gly Asp Ser Tyr Arg Leu Lys Ile Ala His Gly Leu Asp
   50 55 60
   His Glu Glu Leu Val Val Trp Lys Tyr Asn Arg Leu Ile Arg Leu Cys
   65 70 75 80
   Gln Thr Thr Gln Pro Pro Arg Val Glu Thr Tyr Ser Thr Lys Leu Lys
   85 90 95
   Ser Gly Val Leu Pro Gln Gly Val Val Phe Tyr Thr Ser Ser Gly Lys
   100 105 110
   Tyr Leu Lys Glu Thr Tyr Asp Leu Phe Tyr Lys Gln Thr Ala Asp Gly
   115 120 125
   Arg Arg Val Lys Thr Ile Thr Gln Glu Leu Ile Asp Ser Leu Pro Lys
   130 135 140
   His Pro Leu Val Leu Ala Ala Phe Phe Met Asp Asp Gly Ser Val Arg
   145 150 155 160
   Ser Asp Cys Tyr Ser Gly Lys Ile Ala Thr Pro Gly Phe Ala Gly Lys
   165 170 175
   Glu Glu Ser Gln Leu Leu Cys Asn Tyr Leu His Ser Trp Asp Val Gln
   180 185 190
   Ala Asn Val Val Ala His Lys Lys Ala Asn Asn Gln Tyr Tyr Ile Gly
   195 200 205
   Leu Pro Ala Lys Thr Phe Gly Arg Phe Ile Asn Ile Ile Glu Pro Tyr
   210 215 220
   Val Arg Glu Val Pro Ala Leu Cys Tyr Lys Leu Asn Glu Ser Arg Lys
   225 230 235 240
   Pro Arg Asn Asp
<210> 11
   <211> 35
   <212> DNA
   <213> Künstliche Sequenz
<220>
   <223> Beschreibung der künstlichen Sequenz: oligonucleotide primer
<400> 11
   cggctcgagc tacggggacg atttcttttt ttcac 35
<210> 12
   <211> 120
   <212> DNA
   <213> Künstliche Sequenz
<220>
   <223> Beschreibung der künstlichen Sequenz: oligonucleotide primer
<400> 12
   cggctcgagt acctagaata caaagaagag gaagaagaaa cctctacaga agaagccatg 60
   ggtccaaaga aaaagagaaa ggttatcatg aatacaaaat ataataaaga gttcttactc 120
<210> 13
   <211> 116
   <212> DNA
   <213> Künstliche Sequenz
<220>
   <223> Beschreibung der künstlichen Sequenz: oligonucleotide primer
<400> 13
   cggctcgagt acctagaata caaagaagag gaagaagaaa cctctacaga agaagccatg 60
   ggtccaaaga aaaagagaaa ggttatcatg gacattaatc ctcaatggat tacagg 116
<210> 14
   <211> 33
   <212> DNA
   <213> Künstliche Sequenz
<220>
   <223> Beschreibung der künstlichen Sequenz: oligonucleotide primer
<400> 14
   cggctcgagt tactcgccag tttcttcaaa acg 33
<210> 15
   <211> 113
   <212> DNA
   <213> Künstliche Sequenz
<220>
   <223> Beschreibung der künstlichen Sequenz: oligonucleotide primer
<400> 15
   cggctcgagt acctagaata caaagaagag gaagaagaaa cctctacaga agaagccatg 60
   ggtccaaaga aaaagagaaa ggttatcatg accgattcta aatctagaaa caa 113
<210> 16
   <211> 35
   <212> DNA
   <213> Künstliche Sequenz
<220>
   <223> Beschreibung der künstlichen Sequenz: oligonucleotid primer
<400> 16
   cggctcgagc taaaggtggc ctttattgcc atcag 35
<210> 17
   <211> 114
   <212> DNA
   <213> Künstliche Sequenz
<220>
   <223> Beschreibung der künstlichen Sequenz: oligonucleotide primer
<400> 17
   cggctcgagt acctagaata caaagaagag gaagaagaaa cctctacaga agaagccatg 60
   ggtccaaaga aaaagagaaa ggttatcatg tcattaacac aacaacaaaa agac 114
<210> 18
   <211> 35
   <212> DNA
   <213> Künstliche Sequenz
<220>
   <223> Beschreibung der künstlichen Sequenz: oligonucleotide primer
<400> 18
   cggctcgagc taaaggtggc ctttattgcc atcag 35
<210> 19
   <211> 66
   <212> DNA
   <213> Künstliche Sequenz
<220>
   <223> Beschreibung der künstlichen Sequenz: oligonucleotide primer
<400> 19
   cggctctaga gcggccgcct agggataaca gggtaataga atcccacaaa aatctgagct 60
   taacag 66
<210> 20
   <211> 65
   <212> DNA
   <213> Künstliche Sequenz
<220>
   <223> Beschreibung der künstlichen Sequenz: oligonucleotide primer
<400> 20
   cggctctaga ctattaccct gttatcccta ggcccgatct agtaacatag atgacaccgc 60
   gcgcg 65
<210> 21
   <211> 34
   <212> DNA
   <213> Künstliche Sequenz
<220>
   <223> Beschreibung der künstlichen Sequenz: oligonucleotide primer
<400> 21
   cggaagcttc gtcaccaatc ccaattcgat ctac 34
<210> 22
   <211> 34
   <212> DNA
   <213> Künstliche Sequenz
<220>
   <223> Beschreibung der künstlichen Sequenz: oligonucleotide primer
<400> 22
   cggaagcttc cacttgcaaa gtcccgctag tgcc 34
<210> 23
   <211> 34
   <212> DNA
   <213> Künstliche Sequenz
<220>
   <223> Beschreibung der künstlichen Sequenz : oligonucleotide primer
<400> 23
   cggaagcttc gtcaccaatc ccaattcgat ctac 34
<210> 24
   <211> 34
   <212> DNA
   <213> Künstliche Sequenz
<220>
   <223> Beschreibung der künstlichen Sequenz : oligonucleotide primer
<400> 24
   cggaagcttc cacttgcaaa gtcccgctag tgcc 34
<210>
   <211> 62
   <212> DNA
   <213> Künstliche Sequenz
<220>
   <223> Beschreibung der künstlichen Sequenz: oligonucleotide primer
<400> 25
<210> 26
   <211> 62
   <212> DNA
   <213> Künstliche Sequenz
<220>
   <223> Beschreibung der künstlichen Sequenz: oligonucleotide primer
<400> 26
   ctaggatgag ccgtcatcga ggtgccaaac cttcagatta aaatgtctca cgacgttttg 60
   ta 62
<210> 27
   <211> 75
   <212> DNA
   <213> Künstliche Sequenz
<220>
   <223> Beschreibung der künstlichen Sequenz: oligonucleotide primer
<400> 27
   ctagtccgaa aacgccgtga gacatattgg ttacgatcct aaggtagcga aattcacccg 60
   gtaactctgt gccag 75
<210> 28
   <211> 75
   <212> DNA
   <213> Künstliche Sequenz
<220>
   <223> Beschreibung der künstlichen Sequenz: oligonucleotide primer
<400> 28
   ctagctggca cagagttacc gggtgaattt cgctacctta ggatcgtaac caatatgtct 60
   cacggcgttt tcgga 75
<210> 29
   <211> 7
   <212> PRT
   <213> Künstliche Sequenz
<220>
   <223> Beschreibung der künstlichen Sequenz: nuclear location sequence
<400> 29
   Pro Lys Thr Lys Arg Lys Val
   1 5
<210> 30
   <211> 7
   <212> PRT
   <213> Künstliche Sequenz
<220>
   <223> Beschreibung der künstlichen Sequenz: nuclear location sequence
<400> 30
   Pro Lys Lys Lys Arg Lys Val
   1 5

## Beschreibung

Die Erfindung betrifft Rekombinationssysteme und Verfahren, zum Entfernen von Nukleinsäuresequenzen aus dem Genom pflanzlicher Organismen, sowie transgene Pflanzen, die diese Systeme enthalten.

Ziel der biotechnologischen Arbeiten an Organismen ist unter anderem die Gewinnung von kommerziell verwertbaren Informationen über die Funktion bestimmter Gene und Genprodukte sowie die Aufklärung von Biosynthesewegen oder Krankheitsmechanismen. Die so gewonnenen Informationen können vielfältig eingesetzt werden. Sie dienen beispielsweise der Erzeugung neuer Medikamente, der Entwicklung von alternativen, biotechnologischen Produktionsverfahren oder der Erzeugung modifizierten Pflanzen. Ziel biotechnologischer Arbeiten an Pflanzen ist die Herstellung von Pflanzen mit vorteilhaften, neuen Eigenschaften zum Beispiel zur Steigerung der landwirtschaftlichen Produktivität, zur Qualitätssteigerung bei Nahrungsmitteln oder zur Produktion bestimmter Chemikalien oder Pharmazeutika (Dunwell JM, J Exp Bot. 2000;51 Spec No:487-96).

Bei der Herstellung transgene Organismen ist aufgrund der geringen Effizienz der verwendeten Methoden (wie beispielsweise der stabilen Transformation oder insbesondere der homologen Rekombination) eine Selektion der in der gewünschten Weise modifizierten Organismen erforderlich. Die Herstellung transgener Pflanzen kann durch eine Reihe von Techniken erreicht werden (Übersicht: Potrykus I. and Spangenberg G. ed. (1995) Gene transfer to plants. Springer, Berlin). Vor allem der mittels *Agrobacterium tumefaciens* vermittelte Gentransfer und die Beschießung von Pflanzenzellen mit der "Particle Gun" spielen hierbei eine wichtige Rolle. Ein wesentliches Problem ist die Tatsache, dass transgene DNA, sobald sie in einen Organismus stabil eingeführt wurden, nur schwer wieder zu entfernen ist. Die bei der Transformation zur Selektion verwendeten Antibiotika- oder Herbizidresistenzgene werden in den transgenen Pflanzen belassen, was erheblich zu der mangelnden Akzeptanz dieser "Genfood" Produkte bei den Konsumenten beiträgt.

Es wird deshalb seit längerem versucht, Techniken zu entwickeln, mittels derer Fremd-DNA an spezifischen Stellen ins Pflanzengenom integriert bzw. wieder herausgeschnitten werden kann (Ow DW and Medberry SL (1995) Crit Rev in Plant Sci 14:239-261).

Verschiedene Systeme zum gezielten Entfernen von transgen eingefügten Nukleinsäuresequenzen sind dem Fachmann bekannt. Sie basieren auf der Verwendung sequenzspezifischer Rekombinasen und zweier Erkennungssequenzen besagter Rekombinasen, die die zu entfernende Sequenz flankieren. Einwirkung der Rekombinase auf dieses Konstrukt führt zum Herausschneiden der flankierten Sequenz, wobei eine der Erkennungssequenzen im Genom des Organismus verbleibt. Verschiedene sequenzspezifische Rekombinationssystemen sind beschrieben wie das Cre/lox-System des Bacteriophagen P1 (Dale EC und Ow DW (1991) Proc Natl Acad Sci USA 88:10558-10562; Russell SH et al. (1992) Mol Gen Genet 234: 49-59; Osborne BI et al. (1995) Plant J. 7, 687-701), das FLP/FRT System der Hefe (Kilby NJ et al. (1995) Plant J 8:637-652; Lyznik LA et al. (1996) Nucleic Acids Res 24:3784-3789), die Gin Rekombinase des Mu Phagen, die Pin Rekombinase aus E. coli oder das R/RS System des pSR1 Plasmids (Onouchi H et al. (1995) Mol. Gen. Genet. 247:653-660.; Sugita Ket al. (2000) Plant J. 22:461-469).
Hier interagiert die Rekombinase (beispielsweise Cre oder FLP) spezifisch mit ihren jeweiligen Rekombinätionssequenzen (34 bp lox-Sequenz bzw. 47 bp FRT-Seqüenz), um die zwischengelagerten Sequenzen zu deletieren oder zu invertieren. Berichte über gelungene Anwendungen dieser Systeme in Pflanzen sind limitiert. So konnte die Gruppe von David Ow zeigen, dass ein für die Pflanzentransformation verwendeter Selektionsmarker, der von zwei lox Sequenzen umgeben war, durch Cre-Expression aus dem Pflanzengenom wieder herausgeschnitten werden kann (Dale EC und Ow DW (1991) Proc Natl Acad Sci USA 88:10558-10562). Ein Nachteil der sequenzspezifischen Rekombinationssysteme ist die Reversibilität der Reaktion, d.h. es herrscht ein Gleichgewicht zwischen Exzision und Integration des entsprechenden Markergens. Dies führt häufig dazu, dass Mutationen selektioniert werden, d. h. sobaldeine Mutation die weitere Interaktion der lox-Erkennungssequenzen mit dem Enzym blockiert, wird das (ungewollte) Produkt dem Gleichgewicht entzogen und fixiert. Neben dem Cre-lox System trifft das auch auf die anderen sequenzspezifische Rekombinase zu (s.o.). Nachteilig ist ferner die Tatsache, dass eine der Erkennungssequenzen der Rekombinase im Genom verbleibt, dieses also modifiziert wird. Dies kann Auswirkungen auf die Eigenschaften des Organismus haben, wenn beispielsweise durch die Erkennungssequenz Leseraster oder genetische Kontrollelemente wie Promotoren oder Enhancer verändert oder zerstört werden. Ferner schließt die im Genom verbleibende Erkennungssequenz eine weitere Verwendung des Rekombinationsystems, beispielsweise für eine zweite genetische Modifikation, aus, da Wechselwirkungen mit den nachfolgend eingeführten Erkennungssequenzen nicht ausgeschlossen werden können. Größere Chromosomale Umlagerungen oder Deletionen können die Folge sein.

Zubko et al. beschreiben ein System zur Deletion von Nukleinsäuresequenzen aus dem Genom von Tabak, wobei die zu deletierende Sequenz von zwei 352 bp langen attP Erkennungssequenzen des Bakteriophagen Lambda flankiert ist. Die Deletion der flankierten Region erfolgt unabhängig von der Expression von Helferproteinen in zwei von elf transgene Tabaklinien durch spontane, intrachromosomale Rekombination zwischen den attP Erkennungsregionen. Das Verfahren weist Nachteile dahirigehend auf, als die Rekombination bzw. die Deletion nicht gezielt zu einem bestimmten Zeitpunkt induziert werden kann, sondern spontan erfolgt. Dass das Verfahren nur bei einem kleinen Teil der Linien funktionierte, deutet darauf hin, dass in den betreffenden Fällen der jeweilige Integrationslokus zur Instabilität neigt (Puchta H (2000) Trends in Plant Sci 5:273-274) .In der selben Veröffentlichung wird spekuliert, dass die beobachtete Instabilität entweder darauf beruht, dass die verwendeten attP Erkennungsregionen per se Rekombinationen auslösen, oder dass die Transgene in den wenigen Lininen in denen die spontane Rekombination auftrat, in chromosomale Regionen inseriert waren, die eine erhöhte Rekombinationsfrequenz aufweisen. In beiden Fällen wäre die beschriebene Technik nicht breit anwendbar, da sie entweder auf die Verwendung von attP Erkennungsregionen oder auf zufällige Intertiönen an speziellen und weitgehend unbekannte chromosomalen Regionen angewiesen wären.

WO 96/14408 beschreibt auf Seite 12 in der Legende zu Abbildung 32 ein Verfahren zur Entfernung eines genetischen Locus, bei dem je eine Erkennungssequenz der Homing-Restriktionsendonuklease I-SceI an dem jeweiligen Ende der zu deletierenden Sequenz insertiert wird. Die Behandlung mit der Endonuklease führt hier zu Doppelstrangbrüchen an beiden Enden der zu deletierenden Sequenz. Die freien Enden verbinden sich dann durch "Rekombination". Die hier zitierte "Rekombination" kann - wie auch aus der Abbildung ersichtlich - nur eine illegitime sein (beispielsweiseein "non-homologous end-joining" (NHEJ) Ereignis), da an den beiden verbleibenden Enden der genomischen DNA keine homologen Sequenzen vorhanden sind. Eine illegitime Rekombination führt jedoch zu unvorhersagbaren Rekombinationsereignissen. Dies kann Auswirkungen auf die Eigenschaften des Organismus haben, wenn dadurch beispielsweise Leseraster oder genetische Kontrollelemente wie Promotoren oder Enhancer verändert oder zerstört werden. Das System erfordert zwei Erkennungssequenzen, die das zu deletierende Fragment flankieren.

Die Erzeugung sequenzspezifischer Doppelstrangbrüche mit Hilfe von Restriktionsenzymen in eukaryontischen Genomen, wie Hefe (Haber JE (1995) Bioassays17:609-620), Säugerzellen (JasinM (1996) Trends Genet. 12:224-228) oder Pflanzen (PuchtaH (1999a) Methods Mol Biol 113:447-451) ist beschrieben.

Beschrieben ist die Induktion einer intramolekularen Rekombination auf einer Plasmid DNA in Xenopus Oozyten durch sequerizspezifische Spaltung mit der Endonuklease I-SceI (Segal DJ und Caroll D (1995) Proc Natl Acad Sci USA 92:806-810) oder durch synthetische, chimäre Nukleasen (Bibikova M et al. (2001) Mol Cell Biol 21 (1) 289-297). Ziel ist die gezielte Rekombination zwischen zwei homologen Sequenzen, zwischen denen eine entsprechende Nuklease-Schnittstelle lokalisiert ist. In beiden Fällen handelt es sich um extrachromosomale Rekombinationsereignisse, bei denen jeweils nur ein Teil der extrachromosomalen Plasmid-DNA homolog rekombiniert.

Posfai et al. beschreiben ein Verfahren zum Austausch von Genen in dem Prokaryoten E.coli (Posfai G et al. (1999) Nucleic Acids Res 27 (22) :4409-4415) . Dabei kommtes im E.coli Genom zu einer Rekombinätion zwischen dem endogerien und dem mutierten Gen, die durch einen Schnitt mit dem Restriktionsenzym I-SceI induziert wird. Ziel und Aufgabe war der Austausch eines endogenen Gens gegen ein mutiertes Transgen. Rekombinationen in E.coli verlaufen deutlich einfacherer und mit höherer Effizienz als in höheren Eukaryonten (zum Beispiel beschrieben bei Kuzminov A (1999) Microbiol Mol Biol Rev. 63(4):751-813).

Dürrenberger et al. beschreiben die Induktion einer Rekombination in Chloroplasten der einzelligen Grünalge Chlamydoinonas reinhardtii unter Verwendung der I-SceI Homing-Endonuklease (Dürrenberger F et al: (1996) Nucleic Acid Res 24 (17) : 3323-3331). Die Rekombination erfolgt zwischen dem endogenen 23S-Gen und einer insertierten 23S-cDNA, die eine I-SceI Schnittstelle enthält. Doppelstrangbrüche werden durch "Mating" des entsprechenden transgenen Organismus mit einem I-SceI exprimierenden Organismus induziert. Rekombinationen in Chloroplasten verlaufen deutlich einfacherer undmit höherer Effizienz als in der chromosomalen DNA höheren Eukaryonten. So ist die homolöge Rekombination anscheinend sogar der bevorzugte, normale Weg der DNA Integration in Plastiden (Chloroplasten) (beschrieben bei: Heifetz PB und Tuttle AM (2001) Curr Opinion Plant Biol 4:157-161). Plastiden haben anscheinend ein spezielles System, das ihnen homologe Rekombination im Unterschied zum Zellkern ermöglicht und die gezielte Einführung von Fremd-DNA erleichtert (Heifetz PB (2000) Biochimie 82:655-666).

Die "Gene Targeting" Technik, bei der eine gezielte Integration in die chromosomale DNA des Wirtsorganismus durch homologe Rekombination erreicht werden soll, funktioniert mit akzeptabler Effizienz nur bei Prokaryonten und Hefe. Die Erzeugung entsprechender transgener Organismen ist nur bei wenigen Spezies (wie beispielsweise der Maus) und dort nur mit hohem Aufwand möglich (siehe auch Kanaar R Hoeijmakers JH (1997) Genes Funct 1 (3) : 165-174).Diebestehende,geringen Effizienz der homologen Rekombination (ca. 1:1x10⁶) wird hier durch den Einsatz aufwendiger, ausgeklügelter und auf die jeweilige Spezie beschränkter Selektionstechniken (wie beispielsweise der "ES"-Zelltechnologie) kompensiert. In anderen Spezies - vor allem aber in höheren Pflanzen - sind derartige Technologien bis heute nicht etabliert (Mengiste T und Paszkowski J (1999) Biol Chem. 380:749-758; Vergunst AC und Hooykaas PJJ (1999) Crit Rev Plant Sci 18:1-31; Puchta H (1999). Methods Mol Biol 113:447-451; Hohn B und Puchta H (1999) Proc Natl Acad Sci USA 96:8321-8323) Versuche bei Pflanzen eine homologe Rekombination zu erreichen, führen in den meisten Fällen zu zufälligen, nicht-homologen ("illegitimen") Insertionsereignissen. Dabei wird die eingebrachte DNA an einer oder mehreren vorher nicht bestimmbaren Stellen im Pflanzengenom integriert. Die Integration erfolgt mittels illegitimer Rekombination (Roth DB und Wilson JH (1988) Illegitimate recombination in mammalian cells. In "Genetic recombination", R. Kucherlapati and G.R. Smith Edts., American Society of Micorbiology, Washington, USA; S.621-635.) und nicht in Sequenzbereichen, die homolog zur transferierten DNA sind (Puchta H und Hohn B (1996) Trends Plant Sci. 1:340-348). Die Problematik der mangelnden Effizienz der homologen Rekombination, die vor allem bei Pflanzen gravierend ist, ist dem Fachmann allgemein bekannt. Die Ursachen sind Gegenstand aktueller Forschung (Übersichtsartikel: Mengiste T und Paszkowski J (1999) Biological Chemistry 380(7-8):749-58). Die Steigerung der Effizienz der homologen Rekombination ist ein lange bestehendes, bislang ungelöstes Bedürfnis in der Pflanzenbiotechnologie.

Eine weiteres, lange bestehendes Bedürfnis der biotechnologischen Forschung, das durch alle bislang etablierten Systeme nicht gelöst wird, ist die Bereitstellung von Systemen und Verfahren, die eine gezielte Entfernung von Nukleinsäuresequenzen aus der chromosomalen DNA.eines eukaryotischen Organismus ermöglichen und eine mehrfache Anwendung auf den gleichen Organismus gestatten. Beispielsweise ist es ein Ziel der pflanzlichen Biotechnologie, bereits gezüchtete Hochleistungssorten mittels gentechnologischer Methoden weiter zu verbessern. Dabei ist es besonders wichtig, überschüssige Transgensequenzen wie Selektionsmarker nach der Transformation zu entfernen. Darüberhinaus böten Verfahren zur vorhersagbaren Eliminationen von Sequenzen beispielsweise aus der chromosomalen DNA eines Organismus weitere wissenschaftlich und wirtschaftlich hochinteressante Anwendungen im Bereich des "genetic engeneering".

Es stellte sich also die Aufgabe, Systeme und Verfahren zu entwickeln, die eine vorhersagbare Elimination definierter Nukleinsäuresequenzen aus der chromosomalen DNA eines eukaryotischen Organismus ermöglichen und eine mehrfache, sukzessive Anwendung auf den gleichen Organismus gestatten.

Diese Ausgabe wurde durch Bereitstellung des erfindungsgemäßen Rekombinationssystems in überraschender Weise gelöst.

Ein erster Gegenstand der Erfindung betrifft ein Rekombinationssystem zum Entfernen einer DNA-Sequenz aus der chromosomalen DNA einer pflanzlichen Zelle oder eines pflanzlichen Organismus, dadurch gekennzeichnet, dass
I) ein transgenes Rekombinationskonstrukt insertiert in die chromosomale DNAeiner pflanzlichen Zelle oder eines pflanzlichen Organismus, das eine Sequenz enthält bestehend in 5'/3' -Richtung aus
   a1) einer ersten Homologiesequenz A und
   b1) mindestens eine Erkennungssequenz zur gezielten Induktion von DNA-Doppelstrangbrüchen und
   c) eines Expressions Kassette als weitere Nukleinsäursequenz und
   a2) einer zweiten Homologiesequenz B, wobei die Homolögiesequenzen A und B eine ausreichende Länge und ausreichende Homologie aufweisen, um eine homologe Rekombination zwischen den Homologiesequenzen A und B zu gewährleisten,
   und wobei besagte Expressionskassette und besagte Erkennüngssequenz (en) zur gezielten Induktion von DNA-Doppelstrangbrüchen infolge einer homologen Rekombination zwischen A und B aus der chromosomalen DNA entfernt werden
   und
II) ein Enzym geeignet zur Induktion von DNA-Doppelstrangbrüchen an der Erkennungssequenz (b1) zur gezielten Induktion von DNA-Doppelstrangbrüchen oder eine Nukleinsäuresequenz kodierend für ein Enzym geeignet zur Induktion von DNA-Doppelstrangbrüchen an der Erkennungssequenz (b1)
in einer pflanzlichen Zelle oder einem pflanzlichen Organismus zusammen vorliegen zusammen vorliegen und das zur Induktion von DNA-Doppelstrangbrüchen geeignete Enzym ausgewählt ist aus der Gruppe bestehend aus Homing-Endonukleasen, Gruppe II Intron Endonukleasen, Rekombinasen, Transposasen, Chimäre Nukleasen.

Ein weiterer Gegenstand der Erfindung betrifft ein Verfahren zum Entfernen einer DNA-Sequenz aus der chromosomalen DNA einer pflanzlichen Zelle oder pflanzlichen Organismus, dadurch gekennzeichnet, dass
I) ein transgenes Rekombinationskonstrukt inseriert in die chromosomale DNA eines eukaryotischen Organismus, das eine Sequenz enthält bestehend in 5'/3'-Richtung aus
   a1) einer ersten Homologiesequenz A und
   b1) mindestens eine Erkennungssequenz zur gezielten Induktion von DNA-Döppelstrangbrüchen und
   c) einer Expressionskassette als weitere Nukleinsäuresequenz
   a2) einer zweiten Homologiesequenz B, wobei die Homologiesequenzen A und B eine ausreichende Länge und ausreichende Homologie aufweisen, um eine homologen Rekombination zu gewährleisten und
   und
II) ein Enzym geeignet zur Induktion von DNA-Doppelstrangbrüchen an der Erkennungssequenz (b1) zur gezielten Induktion von DNA-Doppelstrangbrüchen
in einer pflanzlichen Zelle oder pflanzlichen Organismus zusammengebracht werden, und die Induktion von DNA-Doppelstrangbrüchen an der (den)Erkennungssequenz (en) zur gezielten Induktion von DNA-Doppelstrangbrüchen sowie die homologe Rekombination zwischen den Homologiesequenzen A und B erfolgt und wobeibesagte Expressionskassette und besagte Erkennungssequenz(en) zur gezielten Induktion von DNA-Doppelstrangbrüchen infolge einer homologen Rekombination zwischen A und B aus der chromosomalen DNA entfernt werden und das zur Induktion von DNA-Doppelstrangbrüchen geeignete Enzym ausgewählt ist aus der Gruppe bestehend aus Homing-Endonukleasen, Gruppe II Intron Endonukleasen, Rekombinasen, Transposasen, Chimäre Nukleasen.

Die Erfindung ermöglicht es, aus der chromosomalen DNA eines Organismus Sequenzen (beispielsweise Selektionsmarker wie Antibiotika- oder Herbizidresistenzgene) in einer exakt vorhersagbaren Weise zu deletieren. Dabei wird die zu eliminierende Sequenz mit Erkennungssequenzen zur gezielten Induktion von DNA-Doppelstrangbrüchen (beispielsweise Erkennungssequenzen von Homing-Endonukleasen, Gruppe II Intron Endonukleasen, Rekombinasen, Transposasen, oder Chimäre Nukleasen) flankiert und mit homologen Sequenzen im Bereich der Schnittstellen kombiniert. Die Induktion eines Doppelstrangbruchs erfolgt durch Homing-Endonukleasen, Gruppe II Intron Endonukleasen, Rekombinasen, Transposasen, oder Chimäre Nukleasen an der Erkennungssequenz zur gezielten Induktion von DNA-Doppelstrangbrüchen, was infolge die homologe Rekombination von am Bruch gelegenen homologer Sequenzen und damit Deletion etwaiger zwischen den Sequenzen lokalisierten Nukleinsäuresequenzen auslöst. Die Erkennungssequenz zur gezielten Induktion von DNA-Doppelstrangbrüchen wird dabei ebenfalls deletiert, wodurch das Verfahren für weitere kontrollierte genetische Veränderungen wiederholt verwendet werden kann.

Überraschenderweise erfolgt diese induzierte, homologe Rekombination im Kontrast zu den bisherigen Erfahrungen auf dem Gebiet der homologen Rekombination - auch bei Pflanzen - mit hoher Effizienz und Präzision. Die Häufigkeit ist mit der der parallelen nicht-homologen Ereignisse (zum Beispiel "non-homologous end-joining" Ereignisse) vergleichbar (vgl. Beispiel 5). Dies ist ein bemerkenswerter Befund, der im Widerspruch zu den bisherigen Beobachtungen steht, wonach die homologe Rekombination - vor allem bei Pflanzen - eine untergeordnete, fast zu vernachlässigende Häufigkeit im Vergleich zu den "illegitimen" Ereignissen hat.

Deletiert werden die zwischen den Homologiesequenzen A und B lokalisierten Sequenzen. Im Unterschied zu Systemen wie beispielsweise dem cre/lox- oder FRT/FLP-System ist man für die Rekombination nicht an bestimmte Sequenzen gebunden. Dem Fachmann ist bekannt, dass jede Sequenz mit einer anderen homolog rekombinieren kann, wenn eine ausreichende Länge und Homologie vorliegt. Durch die sequenzspezifische Induktion der Doppelst rangbrüche wird die Effizienz der homologen Rekombination zwischen den Homologiesequenzen A und B erheblich gesteigert, wenn nicht gar erst ermöglicht.

"Transgen" meint in Bezug auf das Rekombinationskonstrukt alle solche durch gentechnische Methoden zustande gekommen Konstruktionen, in denen sich entweder
a) mindestens eine der Homologiesequenzen A oder B, oder
b) mindestens eine Erkennungsseguenz zur gezielten Induktion von DNA-Doppelstrangbrüchen, oder.
c) (a) und (b)
sich nicht in ihrer natürlichen, genetischen Umgebung (beispielsweise an ihrem natürlichen chromosomalen Locus) befinden oder durch gentechnische Methoden modifiziert wurden, wobei die Modifikation beispielhaft Substitutionen, Additionen, Deletionen, Inversion oder Insertionen eines oder mehrerer Nukleotidreste umfassen kann.

" pflanzlichen Zelle oder pflanzlichen Organismus "meint allgemein jede pflanzlichen Zelle oder pflanzlichen Organismus sowie von diesen abgeleitete Zellen, Gewebe, Teile oder Vermehrungsgut (wie Samen oder Früchte), in denen bei gleichzeitigem Vorliegen des Rekombinationskonstruktes und des Enzyms geeignet zur Induktion von DNA-Doppelstrangbrüchen an der Erkennungssequenz zur gezielten Induktion von DNA-Doppelstrangbrüchen in einem Reaktionsraum (beispielsweise einer Zelle oder einem Kompartiment derselben) eine Induktion von Doppelstrangbrüchen an der Erkennungssequenz zur gezielten Induktion von DNA-Doppelstrangbrüchen sowie die homologe Rekombination zwischen den Homologiesequenzen A und B erfolgen kann. Umfasst sind in einer besonders bevorzugten Ausführungsform Kompartimente einer pflanzlichen Zelle, wie beispielsweise der Zellkern.

Besonders bevorzugt umfasst sind solche pflanzlichen Zellen oder pflanzlichen Organismen, die einen mehrzelligen pflanzlichen Organismus darstellen oder von diesem abgeleitet sind, sowie Zellen, Gewebe, Teile oder Vermehrungsgut (wie Samen oder Früchte) derselben. Ganz besonders bevorzugt Bevorzugte Gattungen und Arten sind weiter unten aufgeführt.

"Ausreichende Länge" meint in Bezug auf die Homologiesequenzen A und B bevorzugt Sequenzen von einer Länge von mindestens 20 Basenpaaren, bevorzugt mindestens 50 Basenpaaren, besonders bevorzugt von mindestens 100 Basenpaaren, ganz besonders bevorzugt von mindestens 250 Basenpaaren, am meistens bevorzugt von mindestens 500 Basenpaaren.

"Ausreichende Homologie" meint in Bezug auf die Homologiesequenzen A und B bevorzugt Sequenzen die eine Homologie innerhalb dieser Homologiesequenzen aufweisen von mindestens 70 %, bevorzugt 80 %, vorzugsweise mindestens 90 %, besonders bevorzugt mindestens 95 %, ganz besonders bevorzugt mindestens 99 %, am meisten bevorzugt 100% über eine Länge von mindestens 20 Basenpaaren, bevorzugt mindestens 50 Basenpaaren, besonders bevorzugt von mindestens 100 Basenpaaren, ganz besonders bevorzugt von mindestens 250 Basenpaaren, am meistens bevorzugt von mindestens 500 Basenpaaren.

Unter Homologie zwischen zwei Nukleinsäure wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2,912 | Average Mismatch:-2,003 |

In einer bevorzugten Ausführungsform ist zwischen den Homologiesequenzen A und B nur eine Erkennungssequenz zur gezielten Induktion von DNA-Doppelstrangbrüchen lokalisiert, so dass das in dem erfindungsgemäßen Rekombinationssystem bzw. Verfahren zum Einsatz kommende Rekombinationskonstrukt wie folgt in 5'/3'-Richtung aufgebaut ist aus
a1) einer ersten Homologiesequenz A und
b1) einer Erkennungssequenz zur gezielten Induktion von DNA-Doppelstrangbrüchen und
c) einer weiteren Nukleinsäuresequenz und
a2) einer zweiten Homologiesequenz B, wobei die Homologiesequenzen A und B eine ausreichende Länge und ausreichende Homologie aufweisen, um eine homologe Rekombination zu gewährleisten.

In einer bevorzugten Ausführungsform ist zwischen den Homologiesequenzen A und B eine weitere Nukleinsäuresequenz lokalisiert, so dass das in dem erfindungsgemäßen Rekombinationssystem bzw. Verfahren zum Einsatz kommende Rekombinationskonstrukt wie folgt in 5'/3'-Richtung aufgebaut ist aus
a1) einer ersten Homologiesequenz A und
b1) einer Erkennungssequenz zur gezielten Induktion von DNA-Doppelstra-ngbrüchen und
c) einer weiteren Nukleinsäuresequenz und
a2) einer zweiten Homologiesequenz B, wobei die Homologiesequenzen A und B eine ausreichende Länge und ausreichende Homologie aufweisen, um eine homologe Rekombination zu gewährleisten.

Die Erkennungssequenz zur gezielten Induktion von DNA-Doppelstrangbrüchen kann auch hinter oder in der weiteren Nukleinsäuresequenz lokalisiert sein.

In einer weiteren bevorzugten Ausführungsform ist hinter die weitere Nukleinsäuresequenz eine zweite Erkennungssequenz zur gezielten Induktion von Doppelstrangbrüchen vorhanden. Vor allem bei weiter auseinanderliegenden Homologiesequenzen A und B bzw. längeren weiteren Nukleinsäuresequenzen ist diese Ausführungsform vorteilhaft, da die Effizienz der Rekombination gesteigert wird. Das in dem erfindungsgemäßen Rekombinationssystem bzw. Verfahren zum Einsatz kommende Rekombinationskonstrukt ist nach dieser Ausführungsform wie folgt in 5'/3'-Richtung aufgebaut aus
a1) einer ersten Homologiesequenz A und
b1) einer ersten Erkennungssequenz zur gezielten Induktion von DNA-Doppelstrangbrüchen and
c) einer weiteren Nukleinsäuresequenz und
b2) einer zweiten Erkennungssequenz zur gezielten Induktion von DNA-Doppelstrangbrüchen und
a2) einer zweiten Homologiesequenz B, wobei die Homologiesequenzen A und B eine ausreichende Länge und ausreichende Homologie aufweisen, um eine homologe Rekombination zu gewährleisten.

Ferner können neben der zweiten Erkennungssequenz zur gezielten Induktion von DNA-Doppelstrangbrüchen noch weitere Erkennungssequenzen zwischen den Homologiesequenzen A und B vorhanden sein. Die einzelnen Erkennungssequenzen (zum Beispiel b1 oder b2) zur gezielten Induktion von DNA-Doppelstrangbrüchen können identisch oder unterschiedlich sein, d.h. sie können als Erkennungssequenz für ein einzelnes Enzym zur gezielten Induktion von DNA-Doppelstrangbrüchen fungieren oder auch für verschiedene. Dabei ist die Ausführungsform bevorzugt, bei der die Erkennungssequenzen zur gezielten Induktion von DNA-Doppelstrangbrüchen als Erkennungssequenz für ein einzelnes Enzym zur gezielten Induktion von DNA-Doppelstrangbrüchen fungieren.

Dem Fachmann sind verschiedene Wege bekannt, um zu einem der erfindungsgemäßen Rekombinationskonstrukte zu gelangen. Die Herstellung kann mittels gängiger Rekombination- und Klonierungstechniken realisiert werden, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Bermän und L.Wi, Enquist, Experiments with Gene Fusions, Cold Spring Harbor Laboratory, Cold Spring Harbor, NY (1984) und in Ausubel, F.M. et al., Current Protocols in Molekular Biology, Greene Publishing Assoc. and Wiley Interscience (1987) beschrieben sind.

Bevorzugt wird das erfindungsgemäße Rekombinationskonstrukt durch Aneinanderfügung der oben aufgeführten wesentlichen Bestandteile des Rekombinationskonstrukt in der genannten Reihenfolge unter Verwendung dem Fachmann geläufiger Rekombinations- und Klonierungstechniken hergestellt und dann in die chromosomale DNA eines Wirtsorganismus eingeführt.

Dem Fachmann ist jedoch bewusst, dass er zu dem erfindungsgemäßen Rekombinationskonstrukt auch auf andere Weise gelangen kann. So kann der Wirtsorganismus bereits eines oder mehr der wesentlichen Bestandteile des Rekombinationskonstruktes enthalten.
Das erfindungsgemäße Rekombinationskonstrukt wird dann durch Einführung eines weiteren oder mehr der wesentlichen Bestandteile des Rekombinationskonstruktes in der richtigen Position zu den bereits vorhandenen Bestandteilen in dem besagten Organismus erzeugt. So kann beispielsweise der Ausgangsorganismus bereits eine der Homologiesequenzen A oder B enthalten. Enthält der Organismus bereits eine Homologiesequenz A, so entsteht durch Einführung eines Konstruktes bestehend aus einer Erkennungssequenz zur gezielten Induktion von DNA-Doppelstrangbrüchen und einer zweiten Homologiesequenz B hinter die Homologiesequenz A eines der erfindungsgemäßen Rekombinationskonstrukte.

Ferner sind dem Fachmann verschiedene Wege bekannt, wie das erfindungsgemäße Rekombinationskonstrukt in die chromosomale DNA pflanzlichen Zelle oder pflanzlichen Organismus eingeführt werden kann. Dabei kann die Insertion gerichtet (d.h. an einem definiertem Insertionsort) oder ungerichtet (d.h. zufällig) erfolgen. Entsprechende Techniken sind dem Fachmann bekannt und weiter unten beispielhaft beschrieben.

"Enzym geeignet zur Induktion von DNA-Doppelstrangbrüchen an der Erkennungssequenz zur gezielten Induktion von DNA-Doppelstrangbrüchen" (infolge "DSBI-Enzym" für "double strand-break inducing enzyme") meint solche Enzyme, die in der Lage sind, sequenzspezifisch Doppelstrangbrüche in doppelsträngige DNA zu erzeugen. Beispielsweise aber nicht einschränkend seien zu nennen:
1. Homing-Endonukleasen wie weiter unten im Detail beschrieben.
2. Rekombinasen (wie beispielsweise Cre/lox; R-RS; FLP/FTR wie oben beschrieben)
3. Transposasen zum Beispiel die P-Element Transposase (Kaufman PD und Rio DC (1992) Cell 69(1) : 27-39) oder AcDs (Xiao YL und Peterson T (2000) Mol Gen Genet 263(1):22-29). Im Prinzip sind alle Transposasen oder Integrasen geeignet, solange sie eine Sequenzspezifität haben (Haren L et al. (1999) Annu Rev Microbiol. 1999;53:245-281; Beall EL, Rio DC (1997) Genes Dev. 11 (16) : 2137-2151).
4. Chimäre Nukleasen wie weiter unten im Detail beschrieben.
5. Gruppe II Intron Endonukleasen. Durch Modifikationen der Intronsequenz können Gruppe II Introns zu einer nahezu beliebigen Sequenz in einer Doppelstrang-DNAdirigiert werden. In diese können die Gruppe II Introns dann mittels eines reversen Spleißmechanismus inserieren (Mohr et al. (2000) Genes & Development 14:559-573; Guo et al. (2000) Science 289:452- 457). Während dieses reversen Spleißmechanismus wird in die Ziel-DNA ein Doppelstrangbruch eingeführt, wobei die ausgeschnittene Intron-RNA den Sinn-Strang spaltet, während der Proteinanteil der Gruppe II Intron Endonuklease denn Gegensinn-Strang hydrolysiert (Guo et al. (1997) EMBO J 16: 6835- 6848) . Will man wie in der vorliegenden Erfindung lediglich den Doppelstrangbruch induzieren und kein vollständiges reverses Spleißen erzielten, kann man zum Beispiel Gruppe II Intron Endonukleasen benutzen, denen die reverse Tränskriptaseaktivität fehlt. Dadurch wird nicht das Erzeugen des Doppelsträngbruches verhindert, aber der reverse Spleißmechanismus kann nicht vollständig ablaufen.

Sowohl natürliche als auch künstlich hergestellte Enzyme sind geeignet.

Bevorzugt sind all solche DSBI-Enzyme, deren Erkennungssequenz bekannt ist und die entweder in Form ihrer Proteine (beispielsweise durch Aufreinigung) gewonnen oder unter Verwendung ihrer Nukleinsäuresequenz exprimiert werden können.

Besonders bevorzugt sind Homing-Endonukleasen, die keine oder nur wenige Erkennungssequenzen - neben den im transgenen Rekombinationskonstrukt vorhandenen Erkennungssequenzen - in der chromosomalen DNA-Sequenz eines bestimmten, pflanzlichen Organismus haben. Dies vermeidet weitere Doppelstrangbrüche an unerwünschten Loci im Genom.

Homing-Endonukleasen (Übersicht: (Belfort M und Roberts RJ (1997) Nucleic Acids Res 25: 3379-3388; JasinM (1996) Trends Genet. 12:224-228; Internet: http://rebase.neb.com/rebase/rebase.homing.html) haben aufgrund ihrer langen Erkennungssequenzen meist keine oder nur wenige weitere Erkennungssequenzen in der chromosomalen DNA eukaryotischer Organismen.

Die für derartige Homing-Endonukleasen kodierenden Sequenzen können beispielsweise aus dem Chloroplastengenom von Chlamydomonas isoliert werden (Turmel M et al. (1993) J Mol Biol 232: 446-467). Sie sind klein (18 bis 26 kD), weisen in ihrem offenen Leseraster (ORF) eine "coding usage" auf, die direkt für nukleäre Expression in Eukaryonten (Monnat RJ Jr et al. (1999) Biochem Biophys Res Com 255:88-93) geeignet ist. Ganz besonders bevorzugt sind die Homing-Endonukleasen I-SceI (WO96/14408), I-SceII (Sarguiel B et al. (1990) Nucleic Acids Res 18:5659-5665), I-SceII (Sarguiel B et al. (1991) Mol Gen Genet. 255:340-341), I-CeuI (Marshall (1991) Gene 104:241-245), I-CreI (Wang J et al. (1997) Nucleic Acids Res 25: 3767-3776), I-ChuI (Cote V et al.(1993) Gene 129:69-76), I-TevI (Chu et al. (1990) Proc Natl Acad Sci USA 87:3574-3578; Bell-Pedersen et al. (1990) Nucleic Acids Res18:3763-3770), I-TevII (Bell-Pedersen et al. (1990) Nucleic Acids Res18:3763-3770), I-TevIII (Eddy et al. (1991) GenesDev.5:1032-1041), Endo SceI (Kawasaki et al. (1991) J Biol Chem 266:5342-5347), I-CpäI (Turmel M et al. (1995a) Nucleic Acids Res 23:2519-2525) und I-CpaII (Turmel M et al. (1995b) Mol. Biol. Evol. 12, 533-545) isoliert.

Weitere Homing-Endonukleasen sind unter der oben angegebenen Internet-Adresse aufgeführt, zu nennen sind beispielsweise Homing-Endonukleasen wie F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI-, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-Sce.I, PI-TfuI, PI-TfuII, PI-ThyI, PI-TliI, PI-TliII.

Bevorzugt sind dabei die Homing-Endonukleasen, deren Gensequenzen bereits bekannt sind wie beispielsweise F-SceI, I-CeuI, I-ChuI, I-DmoI, I-CpaI, I-CpaII, I-CreI, I-CsmI, F-TevI, F-TevII, I-TevI, I-TevII, I-AniI, I-CvuI, I-DdiI, I-HmuI, I-HmuII, I-LlaI, I-NanI, I-MsoI, I-NitI, I-NjaI, I-PakI, I-PorI, I-PpoI, I-ScaI, I-Ssp6803I-, PI-PkoI, PI-PkoII, PI-PspI, PI-TfuI, PI-TliI.

Ganz besonders bevorzugt sind kommerziell erhältliche Homing-Endonukleasen wie I-CeuI, I-SceI, I-DmoI, I-PpoI, PI-PspI oder PI-SceI.

Die Enzyme können in der dem Fachmann geläufigen Art und Weise aus ihren Herkunftsörganismen auf gereinigt und/oder die für sie kodierende Nukleinsäuresequenz kloniert werden. Die Sequenzen verschiedener Enzyme sind in der GenBank hinterlegt.

Ganz besonders bevorzugt sind die Homing-Endonukleasen I-SceI, I-CpaI, I-CpaII, I-CreI und I-ChuI. Am meisten bevorzugt sind die Homing-Endonukleasen gemäß SEQ ID NO: 2, 4, 6, 8 oder 10.

Als künstliche DSBI-Enzyme seien beispielhaft chimäre Nukleasen zu nennen, die sich aus einer unspezifischen Nukleasedomäne und einer sequenzspezifischen DNA-Bindungsdomäne bestehend aus Zinkfingern zusammensetzen (Bibikova M et al. (2001) Mol Cell Biol. 21:289-297). Diese DNA-bindenden Zinkfingerdomänen können an jede beliebige DNA-Sequenzangepasst werden. Entsprechende Verfahren zur Erstellung entsprechender Zinkfingerdomänen sind beschrieben und dem Fachmann bekannt (Beerli RR et al., Proc Natl Acad Sci USA. 2000; 97 (4):1495-1500; Beerli RR, et al., J Biol Chem 2000; 275(42):32617-32627; Segal DJ and Barbas CF 3rd., Curr Opin Chem Biol 2000; 4 (1) :34-39; Kang JS and Kim JS, J Biol Chem 2000; 275(12):8742-8798; Beerli RR et al., Proc Natl Acad Sci USA 1998; 95(25):14628-14633; Kim JS et al., Proc Natl Acad Sci USA 1997; 94(8):3616-3620; Klug A, J Mol Biol 1999; 293 (2)::215-218; Tsai SY et al., Adv Drug Deliv Rev 1998; 30 (1-3):23-31; Mapp AK et al., Proc Natl Acad Sci USA 2000; 97 (8):3930-3935; Sharrocks AD et al., Int J Biochem Cell Biol 1997; 29(12):1371-1387; Zhang L et al., J Biol Chem 2000; 275(43):33850-33860).

Bevorzugt wird das DSBI-Enzym als Fusionsprotein mit einer Kernlokalisationssequenz (NLS) exprimiert. Diese NLS-Sequenz ermöglicht einen erleichterten Transport in den Kern und steigert die Effizienz des Rekombinationssystems. Verschiedene NLS-Sequenzen sind dem Fachmann bekannt und unter anderem beschrieben bei Jicks GR und Raikhel NV (1995) Annu. Rev. Cell Biol. 11:155-188. Bevorzugt für pflanzliche Organismen ist beispielsweise die NLS-Sequenz des SV40 "large antigen". Ganz besonders bevorzugt sind die nachfolgenden NLS-Sequenzen:

| | |
|---|---|
| NLS1: | N-Pro-Lys-Thr-Lys-Arg-Lys-Val-C (SEQ ID NO: 29) |
| NLS2: | N-Pro-Lys-Lys-Lys-Arg-Lys-Val-C (SEQ ID NO: 30) |

Die in den Ausführungsbeispielen verwendeten Homing-Endonukleäsen gemäß SEQ ID NO: 4, 6, 8 oder 10 stellen Fusionsproteine aus den nativen Nukleasen und der NLS2 Kernlokalisationssequenz dar.

Aufgrund der geringen Größe vieler DSBI-Enzyme (wie beispielsweise der Homing-Endonukleasen) ist jedoch eine NLS-Sequenz nicht zwingend erforderlich. Diese Enyzme können die Kernporen auch ohne die Unterstützung passieren. Belegt wird dies durch die Effizienz der verwendeten Homing-Endonuklease gemäß SEQ ID NO: 2, die keine Kernlokalisationssequenz umfasst.

In einer weiteren bevorzugten Ausführungsform kann die Aktivität des DSBI-Enzyms induziert werden. Entsprechende Verfahren sind für sequenzspezifische Rekombinasen beschrieben (Angrand PO et al. (1998) Nucl. Acids Res. 26(13):3263-3269; Logie C und Stewart AF (1995) Proc Natl Acad Sci USA 92(13):5940-5944; Imai T et al. (2001) Proc Natl. Acad Sci USA 98(1):224-228). Bei diesen Verfahren werden Fusionsproteine aus dem DSBI-Enzym und der Ligandenb-indedomäne eines Steroidhormonrezeptors (z.B. des humanen Androgenrezeptors, oder mutierte Varianten des humanen Estrogenrezeptors wie dort beschrieben) eingesetzt. Die Induktion kann mit Liganden wie beispielsweise Estradiol, Dexamethason, 4-Hydroxytamoxifen oder Raloxifen erfolgen.

Manche DBSI-Enyzme sind als Dimer (Homo- oder Heterodimer) aktiv (I-CreI bildet ein Homodimer; I-SecIV bildet ein Heterodimer (Wernette CM (1998) Biochemical & Biophysical Research Communications 248(1):127-333)). Eine Dimerisisierung kann induzierbar gestaltet werden, indem beispielsweise die natürlichen Dimerisierungsdomänen gegen die Bindungsdomäne eines niedermolekularen Liganden ausgetauscht werden. Zugäbe eines dimeren Liganden bewirkt dann Dimerisierung des Fusionsproteins. Entsprechende induzierbare Dimerisierungsverfahren als auch die Herstellung der dimeren Liganden sind beschrieben (Amara JF et al. (1997) Proc Natl Acad Sci USA 994 (20) : 10618-1623; Muthuswamy SK et al. (1999) Mol Cell Biol 19 (10): 6845-685; Schultz LW und Clardy J (1998) Bioorg Med Chem Lett. 8(1):1-6; Keenan T et al. (1998) Bioorg Med Chem. 6(8):1309-1335).

"Erkennungssequenz zur gezielten Induktion von DNA-Doppelstrangbrüchen" meint allgemein solche Sequenzen, die unter den Bedingungen in der jeweils verwendeten pflanzlichen Zelle oder pflanzlichen Organism die Erkennung und Spaltung durch das DSBI-Enzym erlauben. Beispielhaft aber nicht einschränkend seien dabei in nachfolgender Tabelle 1 die Erkennungssequenzen für die jeweiligen aufgeführten DSBI-Enzyme genannt.

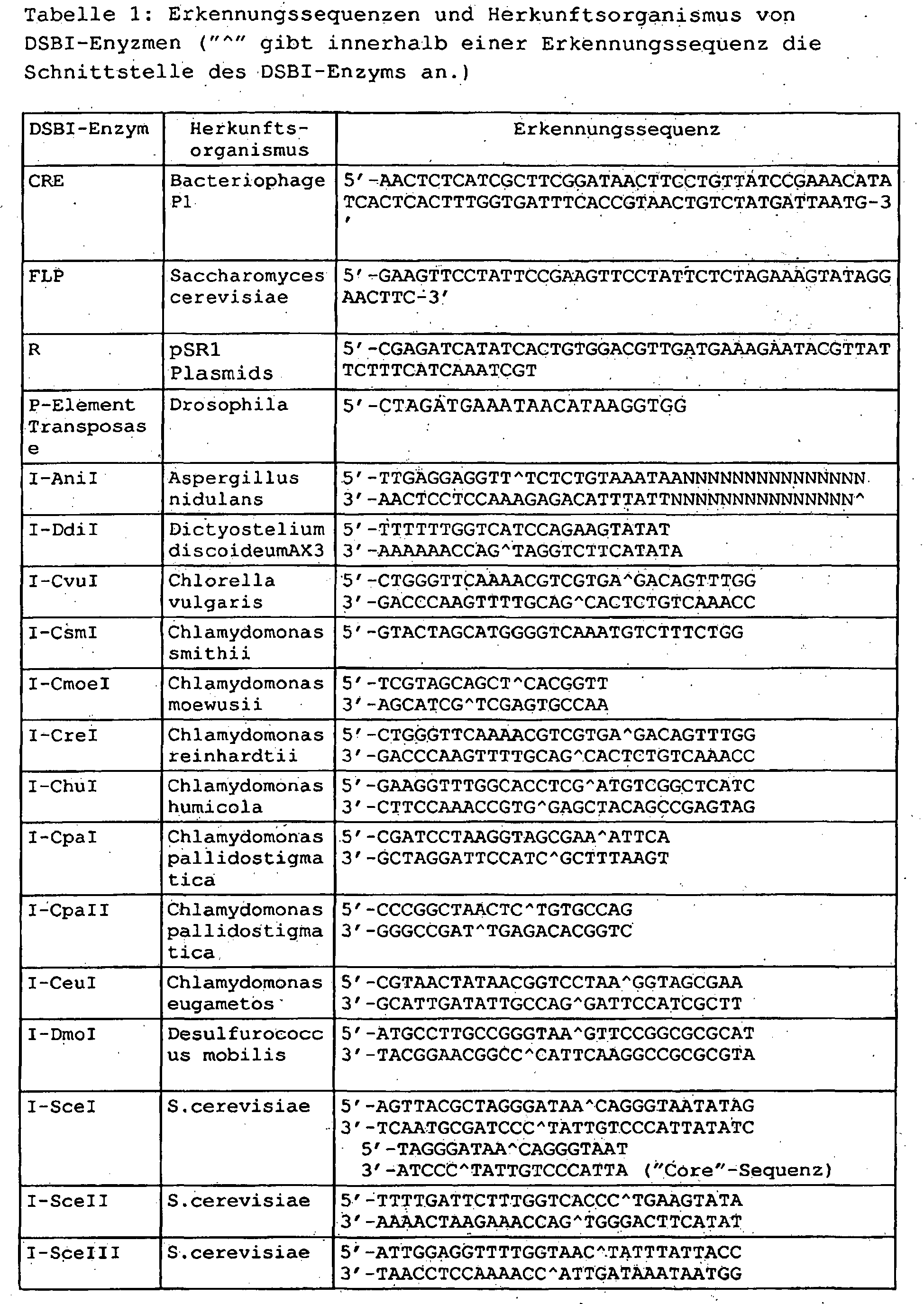

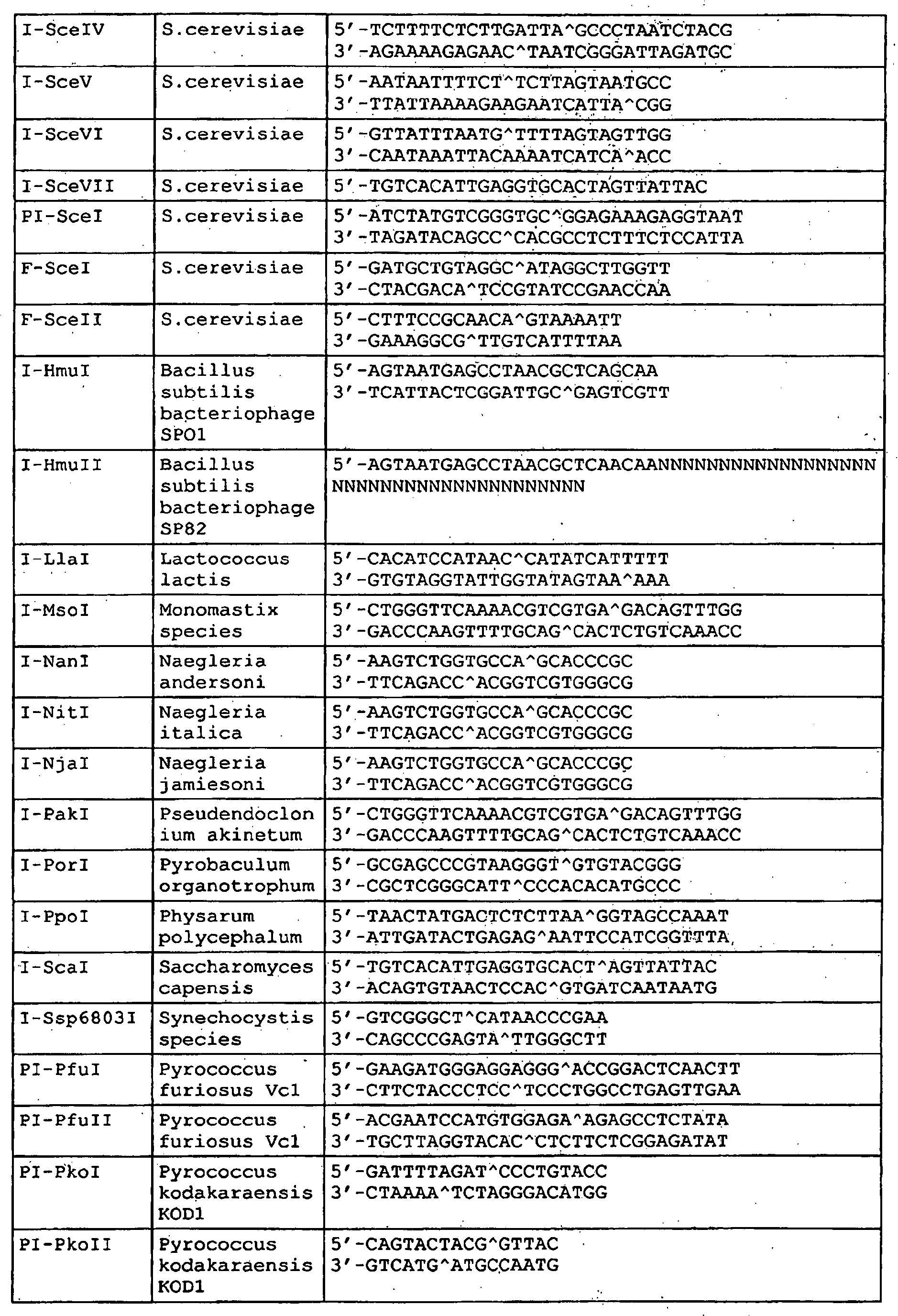

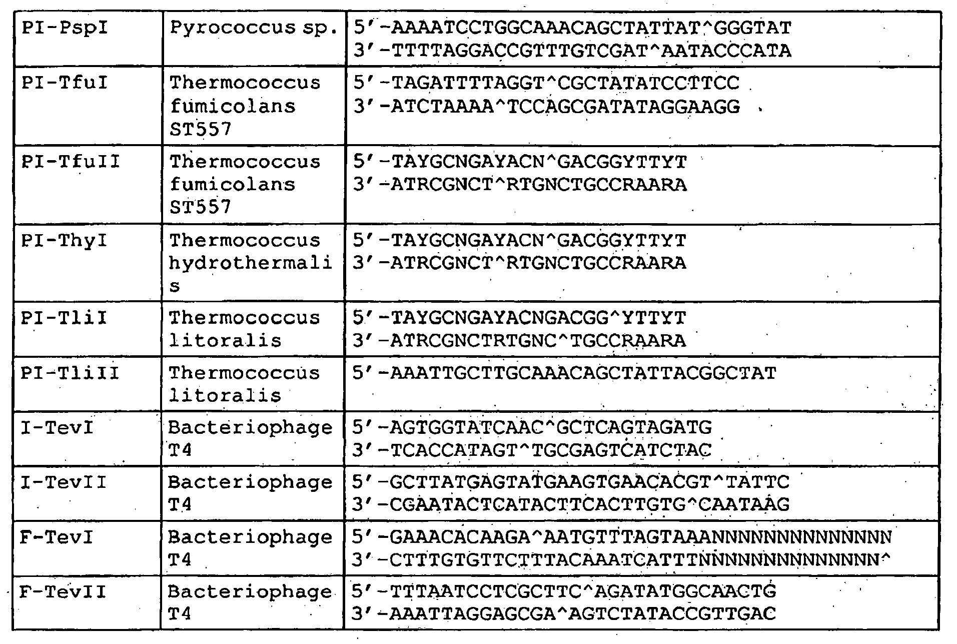

Dabei sind auch kleinere Abweichungen (Degenerationen) der Erkennungssequenz umfasst, die dennoch eine Erkennung und Spaltung durch das jeweilige DSBI-Enzym ermöglichen. Derartige Abweichungen - auch in Zusammenhang mit unterschiedlichen Rahmenbedingungen wie beispielsweise Calcium oder Magnesium-Konzentration-sind beschrieben (Argast GM et al. (1998) J Mol Biol 280: 345-353). Ferner sind Kernsequenzen ("Core"-Sequenzen) dieser Erkennungssequenzen umfasst. Es ist bekannt, dass auch die inneren Anteile der Erkennungssequenzen für einen induzierten Doppelstrangbruch genügen und das die äußeren nicht unbedingt relevant sind, jedoch die Effizienz der Spaltung mitbestimmen können. So kann beispielsweise für I-SceI eine 18bp-"Core"-Sequenz definiert werden.

Die Zusammenführung von Rekombinationskonstrukt und DSBI-Enzym zu einem der erfindungsgemäßen Rekombinationssystemen bzw. Verfahren kann auf verschiedene dem Fachmann geläufige Weisen realisiert werden. So können die Rekombinationskonstrukte und das DSBI-Enzym beispielsweise folgender Maßen in einem Organismus, einer Zelle, Zellkompartiment oder einem Gewebe zusammengebracht werden:
1.) Es werden auf dem üblichen Weg Organismen hergestellt, die die Rekombinationskassette in die chromosomale DNA insertiert tragen. Beispielsweise können entsprechende Pflanzen bevorzugt durch Agrobakterien-vermittelte Transformation hergestellt werden. Die die Rekombinatiönskassette enthaltenden Primärtransformanten werden für die Transformation mit einer Expressionskassette, die die Expression des DSBI-Enzym gewährleistet, eingesetzt oder in geeigneter Weise bis zur Homozygotie geführt und dienen dann als Wirtsorganismus (Wirtspflanze) für die Transformation mit einer Expressionskassette, die die Expression des DSBI-Enzym gewährleistet. Ausgehend von diesen Wirtspflanzen können beispielsweise in vitro Kulturen, wie z.B. Kallus- oder embryogene Kulturen angelegt, etabliert und zur Transformation verwendet werden. Die Transformation mit der Expressionskassette für das DSBI-Enzym kann jeweils stabil oder transient erfolgen.
2.) Es werden auf dem üblichen Weg sogenannte Masterorganismen hergestellt, die das entsprechende Gen für das DSBI-Enzym (bzw. eine Expressionskassette, die die Expression des DSBI-Enzym gewährleistet) tragen und exprimieren. Beispielsweise können entsprechende Masterpflanzen bevorzugt durch Agrobakterien-vermittelte Transformation hergestellt werden. Die das DSBI-Enzym exprimierenden Primärtransformanten werden für die Transformation mit dem Rekombinationskonstrukt eingesetzt oder in geeigneter Weise bis zur Homozygotie geführt und dienen dann als Master- bzw. Wirtsorganismus (Masterpflanze), in die die Rekombinationskonstrukte eingeführt werden. Ausgehend von diesen Masterpflanzen können beispielsweise in vitro Kulturen, wie z.B. Kallus- oder embryogene Kulturen angelegt, etabliert und zur Transformation verwendet werden.
3.) Das Gen kodierend für das DSBI-Enzym (bzw. eine Expressionskassette, die die Expression des DSBI-Enzym gewährleistet) kann in einen Vektor, der bereits die Rekombinationskassette trägt, integriert werden und dadurch zeitgleich mit dem Zielgen in Pflanzenzellen eingeführt werden. Bevorzugt wird das Gen kodierend für das DSBI-Enzym zwischen die Homologiesequenzen insertiert und somit nach Erfüllung seiner Funktion aus der chromosomalen DNA deletiert. Ganz besonders bevorzugt erfolgt in diesem Fall die Expression des DSBI-Enzym induzierbar (beispielsweise unter Kontrolle eines der unten beschriebenen induzierbaren Promotoren), entwicklungsabhängig unter Verwendung eines entwicklungsabhängigen Promotors oder es werden DSBI-Enzyme eingesetzt, deren Aktivität induzierbar ist, um ein Schneiden des Rekombinationskonstruktes gleich nach der Transformation und vor der Insertion in das Genom zu vermeiden.
4.) Die Expressionskassette, die die Expression des DSBI-Enzym gewährleistet, kann mit Hilfe der Co-Transformation zeitgleich mit dem Rekombinationskonstrukt, aber auf einem separaten Vektor in die pflanzlichen Zellen transformiert werden. Die Co-Transformation kann jeweils stabil oder transient erfolgen. Bevorzugt erfolgt in diesem Fall die Expression des DSBI-Enzyms induzierbar (beispielsweise unter Kontrolle eines der unten beschriebenen induzierbaren Promotoren), entwicklungsabhängig unter Verwendung eines entwicklungsabhängigen Promotors oder es werden DSBI-Enzyme eingesetzt, deren Aktivität induzierbar ist, um ein Schneiden des Rekombinationskonstruktes gleich nach der Transformation und vor der Insertion in das Genom zu vermeiden.
5.) Pflanzen, die das DSBI-Enzym exprimieren, können auch als Kreuzungspartner dienen. In den Nachkommen der Kreuzung zwischen pflanzlichen Organismen, die das DSBI-Enzym exprimieren, einerseits und pflanzlichen Organismen, die das Rekombinationskonstrukt tragen, andererseits kommt es zu den erwünschten Doppelstrangbrüchen und der Rekombination zwischen den Homologiesequenzen, wobei ggf. die zwischen den Homologiesequenzeh lokalisierten Sequenzen deletiert werden.
6.) Die Expression des DSBI-Enzyms ist auch in einem transienten Transformationsansatz, bei dem die Möglichkeiten 2 bis 4 genutzt werden können, denkbar.
7.) Das DSBI-Enzym kann auch direkt beispielsweise über Mikroinjektion, Partikel-Bombardierung (biolistische Verfahren), Polyethylenglykol-Transfektion oder Liposomen-vermittelte Transfektion, in pflanzliche Zellen eingebracht werden, die das transgene Rekombinationskonstrukt beinhalten bzw. tragen. Diese Ausführungsform ist vorteilhaft, da hier keine DSBI-Enzym kodierenden Sequenzen im Genom verbleiben können. Ein entsprechendes Verfahren ist beispielsweise beschrieben bei Segal DJ et al. (1995) Proc Natl Acad Sci USA 92:806-810.
8.) Das DSBI-Enzym kann auch durch Einführung der für das DSBI-Enzym kodierenden, in vitro erzeugten mRNA in pflanzlichen Zellen (beispielsweise über Mikroinjektion, Partikel-Bombardierung (biolistische Verfahren) oder Liposomen-vermittelte Transfektion) erzeugt werden. Diese Ausführungsform ist vorteilhaft, da hier keine DSBI-Enzym kodierenden Sequenzen im Genom verbleiben können.
9.) Das DSBI-Enzym kann als Fusionsprotein mit dem VirE2 oder VirF Protein eines Agrobakterium in Pflanzenzellen eingeschleust werden. Entsprechende Verfahren sind beispielsweise für die Cre-Rekombinase beschrieben (Vergunst AC et al. (2000) Science. 290: 979-982). Liegt die Expressionskassette für das Fusionsprotein außerhalb der "Border"-Sequenzen wird sie nicht in das pflanzliche Genom insertiert. Diese Ausführungsform ist vorteilhaft, da hier keine DSBI-Enzym kodierenden Sequenzen im Genom verbleiben können.

Die Realisierung des erfindungsgemäßen Rekombinationssystems bzw. Verfahrens kann in intakten pflanzlichen Organismen als auch in von diesen abgeleiteten Teilen, Zellen oder Vermehrungsgut erfolgen, besonders bevorzugt in intakten Pflanzen als auch in jedem Pflanzengewebe bzw. pflanzlichen in vitro Kulturen einschließlich Kallus. Auch eine in vitro Anwendung unter Einsatz von beispielsweise Weizenkeimextrakt ist denkbar.

Wie oben beschrieben kann das DSB1-Enzym unter Verwendung einer Expressionskassette, die die DNA kodierend für ein DSBI-Enzym enthält, und in eine pflanzlichen Zelle oder pflanzlichen Organismeingebracht wird, erzeugt werden. Dabei enthält die Expressionskassette für das DSBI-Enzym bevorzugt eine Nukleinsäuresequenz kodierend für ein DSBI-Enzym gemäß SEQ ID NO: 2, 4, 6, 8 oder 10 oder ein funktionelles Äquivalent derselben, das in der Lage ist in doppelsträngige DNA unter Nützung der im wesentlichen gleichen Erkennungssequenz DNA-Doppelstrangbrüche zu erzeugen. Im wesentlichen gleiche Erkennungssequenzen meint solche Erkennungssequenzen, die zwar Abweichungen von der für das jeweilige Enzym als optimal gefundenen Erkennungssequenz aufweisen, jedoch eine Spaltung durch dasselbe noch erlauben. Ganz besonders bevorzugt enthalten die Expressionskassetten für das DSBI-Enzym eine Nukleinsäuresequenz gemäß SEQ ID NO: 1, 3, 5, 7 oder 9.

Expressionskassette meint - zum Beispiel in Bezug auf die Expressionkassette für das DSBI-Enzym - solche Konstruktionen bei denen die zu exprimierende DNA in funktioneller Verknüpfung mit mindestens einem genetischen Kontrollelement steht, dass ihre Expression (d.h. Transkription und oder Translation) ermöglicht oder reguliert. Dabei kann die Expression zum Beispiel stabil oder tränsient, konstitutiv oder induzierbar erfolgen. Für die Einführung stehen dem Fachmann verschiedene unten aufgeführte direkte (z.B. Transfektion, Partikelbeschuss, Mikroinjektion) oder indirekte Verfahren (z.B. Agrobakterieninfektion, Virusinfektion) zur Verfügung, die weiter unten aufgeführt werden.

Unter einer funktionellen Verknüpfung versteht man allgemein eine Anordnung in der eine genetische Kontrollsequenz ihre Funktion in Bezug auf eine Nukleinsäuresequenz - beispielsweise kodierend für ein DSBI-Enzym - ausüben kann. Funktion kann dabei beispielsweise die Kontrolle der Expression d.h. Transkription und/oder Translation der Nukleinsäuresequenz - beispielsweise kodierend für ein DSBI-Enzym - bedeuten. Kontrolle umfasst dabei beispielsweise die Initiierung, Steigerung, Steuerung oder Suppression der Expression d.h. Transkription und ggf. Translation. Die Steuerung wiederum kann beispielsweise gewebe- und oder zeitspezifisch erfolgen. Sie kann auch induzierbar zum Beispiel durch bestimmte Chemikalien, Stress, Pathogene etc. sein.

Unter einer funktionellen Verknüpfung versteht man zum Beispiel die sequentielle Anordnung eines Promoters, der zu exprimierenden Nukleinsäuresequenz - beispielsweise kodierend für ein DSBI-Enzym - und ggf. weiterer regulativer Elemente wie zum Beispiel einem Terminator derart, dass jedes der regulativen Elemente seine Funktion bei der Expression der Nukleinsäuresequenz - beispielsweise kodierend für ein DSBI-Enzym - erfüllen kann.

Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu exprimierende Nukleirisäuresequenz - beispielsweise kodierend für ein DSBI-Enzym - hinter eine als Promoter fungierende Sequenz positioniert wird, so dass beide Sequenzen kovalent miteinander verbunden sind. Bevorzugt ist dabei der Abstand zwischen der Promotersequenz und der Nukleinsäuresequenz - beispielsweise kodierend für ein DSBI-Enzym - geringer als 200 Basenpaare, besonders bevorzugt kleiner als 100 Basenpaare, ganz besonders bevorzugt kleiner als 50 Basenpaare.

Dem Fachmann sind verschiedene Wege bekannt, um zu einer solchen Expressionskassette zu gelangen. Die Herstellung erfolgt beispielsweise bevorzugt durch direkte Fusion einer als Promoter fungierenden Nukleinsäuresequenz mit einer zu exprimierenden Nukleotidsequenz - beispielsweise kodierend für ein DSBI-Enzym. Die Herstellung einer funktionellen Verknüpfung kann mittels gängiger Rekombinations- und Klonierungstechniken realisiert werden, wie sie beispielsweise in T. Maniatis, E. F. Fritsch und J. Sambrook, Molecular Cloning: A Laboratory Manual, Cold Spring Harbor Laboratory, Cold Spring Harbor, NY, (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Eine Expressionskassette kann aber auch so konstruiert werden, das die zu exprimierende Nukleinsäuresequenz (z.B. kodierend für ein DSBI-Enzym) beispielsweise mittels homologer Rekombination oder auch durch zufällige Insertion unter Kontrolle eines endogenen genetischen Kontrollelementes, beispielsweise eines Promotors, gebracht wird. Solche Konstruktionen sind ebenfalls als Expressionskassetten im Rahmen der Erfindung zu verstehen.

Dem Fachmann ist ferner bekannt, dass Nukleinsäuremoleküle auch unter Verwendung künstlicher Transkriptionsfaktoren vom Typ der Zinkfingerproteine zur Expression gebracht werden können (Beerli RR et al. (2000) Proc Natl Acad Sci USA 97 (4) :1495-500) . Diese Faktoren können an jeden beliebigen Sequenzbereich adaptiert werden und erlauben eine Expression unabhängig von bestimmten Promotorsequenzen.

Der Begriff der "genetischen Kontrollsequenzen" ist breit zu verstehen und meint all solche Sequenzen, die einen Einfluss auf das Zustandekommen oder die Funktion der erfindungsgemäßen Expressionskassette haben. Genetische Kontrollsequenzen gewährleisten zum Beispiel die Transkription und gegebenenfalls Translation in prokaryotischen oder eukaryotischen Organismen. Vorzugsweise umfassen die erfindungsgemäßen Expressionskässetten 5'-stromaufwärts von der jeweiligen zu exprimierenden Nukleinsäuresequenz einen Promotor und 3'-stromabwärts eine Terminatorsequenz als zusätzliche genetische Kontrollsequenz, sowie gegebenenfalls weitere übliche regulative Elemente, und zwar jeweils funktionell verknüpft mit der zu exprimierenden Nukleinsäuresequenz.

Genetische Kontrollsequenzen sind beispielsweise beschrieben bei "Goeddel; Gene Expression Technology: Methods in Enzymology 185, Academic Press, San Diego, CA (1990)" oder "Gruber and Crosby, in: Methods in Plant Molecular Biology and Biotechnolgy, CRC Press, Boca Raton, Florida, eds. :Glick and Thompson, Chapter 7, 89-108" sowie den dort aufgewiesenen Zitaten.

Beispiele für derartige Kontrollsequenzen sind Sequenzen, an die Induktoren oder Repressoren binden und so die Expression der Nukleinsäure regulieren. Zusätzlich zu diesen neuen Kontrollsequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette kann aber auch einfacher aufgebaut sein, das heißt, es werden keine zusätzlichen Regulationssignale vor die vorstehend erwähnten Gene insertiert und der natürliche Promotor mit seiner Regulation wird nicht entfernt. Stattdessen wird die natürliche Kontrollsequenz so mutiert, dass keine Regulation mehr erfolgt und die Genexpression gesteigert wird. Diese veränderten Promotoren können auch allein vor die natürlichen Gene zur Steigerung der Aktivität gebracht werden.

Je nach nachstehend näher beschriebenen Wirtsorganismus oder Ausgangsorganismus, der durch Einbringen der Expressionskassetten oder Vektoren in einen genetisch veränderten oder trahsgeneh Organismus überführt wird, eignen sich verschiedene Kontrollsequenzen.

Vorteilhafte Kontrollsequenzen für die erfindungsgemäßen Expressionskassetten oder Vektoren sind beispielsweise in Promotoren wie cos-, tac-, trp-, tet-, phoA-, tat-, lpp-, lac-, lacIq-, T7-, 15-, T3-, gal-, trc-, ara-, SP6-, λ-PR- oder im λ-PL-Promotor enthalten, die vorteilhafterweise in gram-negativen Bakterien Anwendung finden.

Weitere vorteilhafte Kontrollsequenzen sind beispielsweise in den gram-positiven Promotoren amy und SPO2, in den Hefe- oder Pilzpromotoren ADC1, MFa , AC, P-60, CYC1, GAPDH, TEF, rp28, ADH oder in den Pflanzenpromotoren CaMV/35S (Franck et al. (1980) Cell 21:285-294), PRP1 (Martini N et al. (1993) Mol Gen Genet. 236(2-3):179-186), SSU, OCS, LEB4, USP, STLS1, B33, NOS; FBPaseP (WO 98/18940) oder im Ubiquitin- oder Phaseolin-Promotor enthalten.

Bevorzugt ist grundsätzlich jeder Promotor, der die Expression von Genen, insbesondere Fremdgenen, in Pflanzen steuern kann. Bevorzugt sind Promotoren, die eine konstitutive Expression in Pflanzen ermöglichen (Benfey et al. (1989) EMBO J. 8:2195-2202). Vorzugsweise verwendet man insbesondere einen pflanzlichen Promotor oder einen Promotor, der einem Pflanzenvirus entstammt. Besonders bevorzugt ist der Promotor des 35S-Transkriptes des Blumenkohlmosaikvirus (Franck et al. (1980) Cell 21:285-294; Odell et al. (1985) Nature 313:810-812; Shewmäker et al. (1985) Virology 140:281-288; Gardner et al. 1986, Plant Mol. Biol. 6, 221-228) oder den 19S CaMV Promotor (US 5, 352, 605 andWO 84/02913) . Dieser Promotor enthält bekanntlich unterschiedliche Erkennungssequenzen für transkriptionale Effektoren, die in ihrer Gesamtheit zu einer permanenten und konstitutiven Expression des eingeführten Gens führen (Benfey et al. (1989) EMBO J 8:2195-2202). Ein weiterer geeigneter konstitutiver Promotor ist der "Rubisco small sübunit (SSU)"-Promotor (US 4,962,028). Ein weiteres Beispiel eines geeigneten Promotors ist der LeguminB-Promotor (GenBank Acc.-No.: X03677) . Weitere bevorzugte konstitutive Promotoren sind zum Beispiel der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), die Promotoren der vakuolärer ATPase Untereinheiten oder der Promotor eines prolinreichen Proteins aus Weizen (WO 91/13991).

Die Expressionskassetten können auch einen induzierbaren, bevorzugt einen chemisch induzierbaren Promotor enthalten (Aoyama T und Chua NH (1997) Plant J 11: 605-612; Caddick MX et al. (1998) Nat. Biotechnol 16:177-180; Rewiew: Gatz, Annu Rev Plant Physiol Plant Mol Biol 1997, 48 : 89-108), durch den die Expression des exogenen Gens in der Pflanzen zu einem bestimmten Zeitpunkt gesteuert werden kann. Derartige Promotoren, wie z.B. der PRP1 Promotor (Ward et al., Plant. Mol. Biol. 22 (1993) , 361-366) , ein durch Salicylsäure induzierbarer (WO 95/19443), ein durch Benzolsulfonamid-induzierbarer (EP-A-0388186), ein durch Tetrazyklin-induzierbarer (Gatz et al., (1992) Plant J. 2, 397-404), ein durch Abscisinsäure-induzierbarer (EP-A 335528), ein durch Salicylsäüre induzierbarer (WO 95/19443) bzw. ein durch Ethanol-(Salter MG et al. (1998) Plant J. 16:127-132) oder Cyclohexanon-induzierbarer (WO 93/21334) Promotor können ebenfalls verwendet werden.

In einer besonders bevorzugten Ausführungsform wird vor allem die für das DSBI-Enzym kodierende Nukleinsäure unter Kontrolle eines induzierbaren Promotors exprimiert. Damit wird eine kontrollierte, steuerbare Expression und Deletion - beispielsweise in Pflanzenerreicht und etwaige Probleme durch eine konstitutive Expression eines DSBI-Enzyms vermieden.

Ferner sind Promotoren bevorzugt, die durch biotischen oder abiotischen Stress induziert werden wie beispielsweise der pathogen-induzierbare Promotor des PRP1-Gens (Ward et al., Plant Mol Biol 1993, 22: 361-366), der hitzeinduzierbare hsp80-Promoter aus Tomate (US 5,187,267), der kälteinduzierare alpha-Amylase Promoter aus der Kartoffel (WO 96/12814) oder der verwundungsinduzierte pinII-Promoter (EP375091).

Bevorzugt sind ferner Promotoren mit Spezifitäten für die Antheren, Ovarien, Pollen, Meristem, Blüten, Blätter, Stengel, Wurzeln und Samen.

Ein entwicklungsabhängig regulierter Promotor ist unter anderem bei Baerson et al. beschrieben (Baerson SR, Lamppa GK (1993) Plant Mol Biol 22(2):255-67).

Besonders sind solche Promotoren bevorzugt, die die Expression in Geweben oder Pflanzenteilen sicherstellen, in denen die Biosynthese von Stärke und/oder Ölen bzw. dessen Vorstufen stattfindet oder in denen die Produkte vorteilhafterweise akkumuliert werden. Der Biosyntheseort der Stärke sind die Chloroplasten der Blätter bzw. die Amyloplasten der Speicherorgane wie Samen, Früchte oder Knollen. In diesen Organen sind es v.a. die Zellen des Endosperms oder die Kotyledonen des Embryos, in denen die Synthese abläuft. Bevorzugte Promotoren sind insofern neben den oben genannten konstitutiven Promotoren, insbesondere samenspezifische Promotoren wie zum Beispiel der Promotor des Phaseolins (US 5,504,200, Bustos MM et al., Plant Cell. 1989; 1 (9) : 839-53), des 2S Albumingens (Joseffson LG et al. (1987) J Biol Chem 262: 12196-12201), des Legumins (Shirsat A et al. (1989) MolGenGenet. 215(2) : 326-331), des USP (unknown seed protein; Bäumlein H et al. (1991) Molecular & General Genetics 225 (3) :459-67) des Napin Gens (US 5,608,152; Stalberg K, et al. (1996) L. Planta 199: 515-519), des Saccharosebindeproteins (WO 00/26388) oder der LeguminB4-Promotor (LeB4; Bäumlein H et al. (1991) Mol Gen Genet 225:121-128; Baeumlein et al. (1992) Plant Journal 2(2):233-239; Fiedler U et al. (1995) Biotechnology (NY) 13 (10):1090-1093), derIns Oleosin-Promoter aus Arabidopsis (WO9895461), der Bce4-Promoter aus Brassica (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2 oder lpt1-Gen (WO 95/15389, WO 95/23230) oder die Promotoren beschrieben in WO 99/16890 (Promoteren des Hordein-Gens, des Glutelin-Gens, des Oryzin-Gens, des Prolamin-Gens, des Gliadin-Gens, des Glutelin-Gens, des Zein-Gens, des Kasirin-Gens oder des Secalin-Gens).

Bevorzugt als genetische Kontrollelemente sind ferner pollenspezifische Promotoren wie beispielsweise der Promotor des B. campestris bgp1 gene (GenBank Acc.-No: X68210; Xu H et al. (1993) Mol Gen Genet 239(1-2):58-65; WO 94/13809), des Oryza sativa ory s 1 Gens (GenBank Acc.-NO. : AJ012760; Xu H et al. (1995) Gene 164 (2):255-259), des pollen-spezifischen Mais Gens ZM13 (Hamilton DA et al. (1998) Plant Mol Biol 38 (4) : 663-669; US 5,086,169), des B.napus GensBp10 (GenBankAcc. -No. : X64257: Albani D (1992) Plant J2 (3) :331-342; US 6, 013, 859).

Ferner sind bevorzugt der Lcg1 Promotor für eine zellspezifische Expression in den männlichen Gameten (WO 99/05287; XU H et al. (1999) Proc. Natl. Acad. Sci. USA Vol. 96:2554-2558) und der Promotor des AtDMC1 Gens (Klimyuk VI et al.(1997) Plant J. 11(1):1-14).

Weitere geeignete Promotoren sind beispielsweise spezifische Promotoren für Knollen, Speicherwurzeln oder Wurzeln, wie beispielsweise der Patatin Promotor Klasse I (B33), der Promotor des Cathepsin D Inhibitors aus Kartoffel, der Promotor der Stärke Synthase (GBSS1) oder der Sporamin Promotor sowie fruchtspezifische Promotoren, wie beispielsweise der fruchtspezifische Promotor aus Tomate (EP-A 409625).

Weiterhin geeignete Promotoren sind solche, die eine blattspezifische Expression gewährleisten. Zu nennen sind der Promotor der cytosolischen FBPase aus Kartoffel (WO 98/18940), der SSU Promotor (small subunit) der Rubisco (Ribulose-1,5-bisphosphatcarboxylase) oder der ST-LSI Promotor aus Kartoffel (Stockhaus et al. (1989) EMBO J8 (9) : 2445-2451). Bevorzugt sind ferner Promotoren, die eine Expression in Samen und pflanzlichen Embryonen steuern.

Weitere geeignete Promotoren sind beispielsweise fruchtreifungsspezifische Promotoren, wie beispielsweise der fruchtreifungs-spezifische Promotor aus Tomate (WO 94/21794), blütenspezifische Promotoren, wie beispielsweise der Phytoen Synthase Promotor (WO 92/16635) oder der Promotor des P-rr Gens (WO 98/22593) oder ein anderer Nodien-spezifischer Promotor wie in EP-A 249676 können vorteilhaft verwendet werden.

Prinzipiell können alle natürlichen Promotoren mit ihren Regülationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen auch weitere Promotoren, Promotorelemente oder Minimalpromotoren, die die expressions steuern den Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (LamEundChuaNH (1991) JBiol Chem 266(26) :17131 -17135) und Hitzestress (Schoffl F et al. (1989) Molecular & General Genetics 217 (2-3) : 246-53) beschrieben.

Es können ferner weitere Promotoren funktionell mit der zu exprimierendenNukleinsäuresequenz verknüpft sein, die eine Expression in weiteren Pflanzengeweben oder in anderen Organismen, wie zumBeispiel *E*.*coli* Bakterien ermöglichen. Als Pflanzenpromotoren kommen im Prinzip alle oben beschriebenen Promotoren in Frage.

Genetische Kontrollsequenzen umfassen ferner auch die 5'-untranslatierte Region, Introns oder die nichtkodierende 3'-Region von Genen. Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Sie können ferner die Gewebespezifität fördern (Rouster Jet al., PlantJ. 1998, 15: 435-440. ) .

Umgekehrt unterdrückt die 5' -untranslatierte Region des opaque-2 Gens die Expression. Eine Deletion der entsprechenden Region führt zu einer Erhöhung der Genaktivität (Lohmer S et al., Plant Cell 1993, 5:65-73).

Genetische Kontrollsequenzen können auch Ribosomenbindungssequenzen zur Initiation der Translation umfassen. Dies ist vor allem dann bevorzugt, wenn von der zu exprimierenden Nukleinsäuresequenz entsprechende Sequenzen nicht bereitgestellt werden oder diese mit dem Expressionssystem nicht kompatibel sind.

Die Expressionskassette kann vorteilhafterweise eine oder mehrere sogenannte "Enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen insertiert werden, wie weitere regulatorische Elemente oder Terminatoren. Die transgen zu exprimierenden Nukleinsäuresequenzen können in einer oder mehreren Kopien im Genkonstrukt enthalten sein.

Genetische Kontrollsequenzen meint ferner Sequenzen, die für Fusionsproteine bestehend aus einer Signalpeptidsequenz kodieren.

Als genetische Kontrollsequenzen geeignete Polyadenylierungssignale sind pflanzliche Polyadenylierungssignale, vorzugsweise solche, die im wesentlichen T-DNA Polyadenylierungssignale aus *Agrobacterium tumefaciens,* insbesondere des Gens 3 der T-DNA (Octopin Synthase) des Ti-Plasmids pTiACHS entsprechen (Gielen et al., EMBO J. 3 (1984), 835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Wie oben erwähnt können die erfindungsgemäßen Rekombinationskonstrukte weitere Nukleinsäuresequenzen umfassen. Solche Nukleinsäuresequenzen können bevorzugt Expressionskassetten darstellen. Beispielhaft aber nicht einschränkend für die in den Expressionskonstrukten zu exprimierenden DNA-Sequenzen seien zu nennen:
i) Positive Selektionsmarker:
   Selektionsmarker sind in der Regel erforderlich, um erfolgreich homolog rekombinierte oder transformierte Zellen zu selektionieren. Der mit dem Expressionskonstrukt eingebrachte selektionierbaren Marker verleiht den erfolgreich rekombinierten oder transformierten Zellen eine Resistenz gegen ein Biozid (zum Beispiel ein Herbizid wie Phosphinothricin, Glyphosat oder Bromoxynil), einen Metabolismusinhibitor
   wie 2-Desoxyglucose-6-phosphat (WO 98/45456) oder ein Antibiotikum, wie zum Beispiel Tetracycline, Ampicillin, Kanamycin, G 418, Neomycin Bleomycin oder Hygromycin, verleiht. Der Selektionsmarker erlaubt die Selektion der transformierten Zellen von untransformierten (McCormick et as., Plant Cell Reports 5 (1986), 81-84). Besonders bevorzugte Selektionsmarker sind solche die eine Resistenz gegen. Herbizide verleihen. Beispielhaft als Selektionsmarker seien genannt:
      - DNA Sequenzen, die für Phosphinothricinacetyltransferasen (PAT) kodieren, welche die freie Aminogruppe des Glutaminsynthaseinhibitors Phosphinothricin (PPT) acetyliert und damit eine Detoxifizierung des PPT erreicht (de Block et al. 1987, EMBO J. 6, 2513-2518) (auch Bialophosr resistenzgen (bar) genannt)
      - 5-Enolpyruvylshikimat-3-phosphatsynthasegene (EPSP Synthasegene), die eine Resistenz gegen Glyphosatr (N-(phosphonomethyl)glycin) verleihen,
      - das für das Glyphosatr degradierende Enzyme kodierende gox Gen (Glyphosatoxidoreduktase),
      - das deh Gen (kodierend für eine Dehalogenäse, die Dalaponr inaktiviert),
      - Sülfonylurea- und Imidazolinon inaktivierende Acetolactatsynthasen
      - bxn Gene, die für Bromoxynilr degradierende Nitrilaseenzyme kodieren
      - das Kanamycin- bzw. G418- Resistenzgen (NPTII) . Das NPTII Gen codiert für eine Neomycinphosphotransferase, die durch eine Phosphorylierungsreaktion die inhibierende Wirkung von Kanamycin, Neomycin, G418 und Paromomycin reduziert.
      - das DOG^{R}1-Gen. Das Gen DOG^{R}1 wurde aus der Hefe Saccharomyces cerevisiae isoliert (EP 0 8.07 836) . Es codiert für eine 2-Desoxyglukose-6-phosphat Phosphatase, die Resistenz gegenüber 2-DOG verleiht (Randez-Gil et al. 1995, Yeast 11, 1233-1240).
ii) Negative Selektionsmarker ermöglichen beispielsweise die Selektion von Organismen mit erfolgreich deletierten Sequenzen, die das Markergen umfassen (Koprek T et al . (1999) The Plant Journal 19 (6) :719-726) . TK thymidine kinase (TK) and diphtheria toxin A fragment (DT-A), codA Gen kodierend für eine Cytosindeaminase (Gleve AP et al. (1999) Plant Mol Biol. 40(2) : 223-35; Pereat RI et al. (1993) Plant Mol. Biol 23 (4) : 793-799; Stougaard J; (1993) Plant J 3:755-761), das Cytochrom P450 Gen (Koprek et al. (1999) Plant J. 16:719-726), Gene kodierend für eine Haloalkan Dehalogenase (Naested H (1999) Plant J. 18: 571-576) , das iaaH Gen (Sundaresan V et al. (1995) Genes & Development 9: 1797-1810) oder das tms2 Gen (Fedoroff NV & Smith DL 1993, Plant J 3: 273- 289).
iii) Reportergene, die für leicht quantifizierbare Proteine kodieren und über Eigenfarbe oder Enzymaktivität eine Bewertung der Transförmationseffizienz, des Expressionsortes oder -zeitpunktes gewährleisten. Ganz besonders bevorzugt sind dabei Gene kodierend für Reporter-Proteine (siehe aueh Schenborn E, Groskreutz D. Mol Biotechnol. 1999; 13(1) : 29-44) wie
   - "green fluorescence protein" (GFP) (Chui WL et al., Curr Biol 1996, 6: 325-330; Leffel SM et al., Biotechniques. 23 (5) : 9912-8, 1997; Sheen et al. (1995) Plant Journal 8 (5) : 777-784; Haselöff et al. (1997) Proc Natl Acad Sci USA 94 (6) 2122-2127; Reichel et al.(1996) Proc Natl Acad Sci USA 93 (12) : 5888-5893; Tian et al. (1997) Plant Cell Rep 16: 267-271; WO 97/41228).
   - Chloramphenicoltransferase,
   - Luziferase (Millar et al., Plant Mol Biol Rep 1992 10: 324-414; Ow et al. (1986) Science, 234:856-859); erlaubt Bioluminescenzdetektion.
   - β-Galactosidase, kodiert für ein Enzym für das verschiedenen chromogene Substrate zur Verfügung stehen.
   - β-Glucuronidase (GUS) (Jefferson et al., EMBO J. 1987, 6, 3901-3907) oder das uidA Gen, das ein Enzym für verschiedene chromogene Substrate kodiert.
   - R-Locus Genprodukt:Protein, das die Produktion von Anthocyaninpigmenten (rote Färbung) in pflanzlichen Gewebe reguliert und so eine direkte Analyse der Promoteraktivität ohne Zugabe zusätzlicher Hilfsstoffe oder chromogener Substrate ermöglicht (Dellaporta et al., In: Chromosome Structure and Function: Impact of New Concepts, 18th Stadler - Genetics Symposium, 11 : 263-282, 1988).
   - β-Lactamase (Sutcliffe (1978). Proc Natl Acad Sci USA 75:3737-3741), Enzym für verschiedene chromogene Substrate (z.B. PADAC, eine chromogenes Cephalosporin).
   - xylE Genprodukt (Zukowsky et al. (1983) Proc Natl Acad Sci USA 80 : 1101-1105), Catecholdioxygenase, die chromogene Catechole umsetzen kann.
   - Alpha-Amylase (Ikuta et al. (1990) Bio/technol. 8: 241-242) .
   - Tyrosinase (Katz et al. (1983) J Gen Microbiol 129:2703-2714), Enzym, das Tyrosin zu DOPA und Dopaquinon oxidiert, die infolge das leicht nachweisbare Melanin bilden.
   - Aequorin (Prasher et al. (1985) Biochem Biophys Res Commun 126(3):1259-1268), kann in der Calcium-sensitiven Bioluminescenzdetektion verwendet werden.

Das Rekombinationskonstrukt und die gegebenenfalls von ihnen abgeleiteten Vektoren können weitere Funktionselemente enthalten. Der Begriff der weitere Funktionselemente ist breit zu verstehen. Bevorzugt sind all solche Elemente gemeint, die einen Einfluss auf Herstellung, Vermehrung, Funktion, Nutzen oder Wert des erfindungsgemäßen Rekombinationssystems, Rekombinationskonstruktes oder diese beinhaltende Zellen oder Organismen haben. Beispielhaft jedoch nicht einschränkend seien für die weiteren Funktionselemente zu nennen:
iv) Replikationsursprünge, die eine Vermehrung der erfindungsgemäßen Expressionskassetten oder Vektoren in zum Beispiel E. coli gewährleisten. Beispielhaft seien genannt ORI (origin of DNA replication), der pBR322 ori oder der P15A ori (Sambrook et al. : Molecular Cloning. A Laborätory Manual, 2nd ed. Cold Spring Harbor Laboratory Press, Cold Spring Harbor, NY, 1989).
v) Multiple Klonierungsregionen (MCS) erlauben und erleichtern die Insertion eines oder mehrerer Nukleinsäuresequenzen.
vi) Sequenzen, die eine homologe Rekombination bzw. Insertion in das Genom eines Wirtsorganismus ermöglichen.
vii) Elemente zum Beispiel "Bordersequenzen", die einen Agrobakterien-vermittelte Transfer in Pflanzenzellen für die Übertragung und Integration ins Pflanzengenom ermöglichen, wie zum Beispiel die rechte oder linke Begrenzung der T-DNA oder die vir-Region.

All die oben erwähnten Expressionskassetten oder weiteren Funktionselemente können, wie erwähnt, zwischen den Homolgiesequenzen A und B lokalisiert sein. Sie können aber auch außerhalb von diesen liegen. Dies ist vor allem bei "Bordersequenzen" vorteilhaft.

Die Einführung einer Rekombinationskassette oder eines Expressionskonstruktes für ein DSBI-Enzym in pflanzlichen Zellen kann vorteilhaft unter Verwendung von Vektoren realisiert werden, in die diese Konstrukte bzw. Kassetten insertiert werden. Vektoren können beispielhaft Plasmide, Cosmide, Phagen, Viren, Retroviren oder auch Agrobacterien sein.

Als Vektoren zur Expression in E. coli sind bevorzugt pQE70, pQE60 und pQE-9 (QIAGEN, Inc.); pBluescript Vektoren, Phagescript Vektoren, pNH8A, pNH16a, pNH18A, pNH46A (Stratagene Cloning Systems, Inc.); ptrc99a, pKK223-3, pKK233-3, pDR540, pRIT5 (Pharmacia Biotech, Inc.).

Bevorzugte Vektoren zur eukaryotischen Expression umfassen pWLNE0, pSV2CAT, pOG44, pXT1 und pSG (Stratagene Inc.) ; pSVK3, pBPV, pMSG und pSVL (Pharmacia Biotech, Inc.). Als induzierbare Vektoren seien pTet-Thia, Potter-Splice, pcDNA4/TO, pcDNA4/TO /LacZ, pcDNA6/TR, pcDNA4/TO/Myc-His /LacZ, pcDNA4/TO/Myc-His A, pcDNA4/TO/Myc-His B, pcDNA4/TO/Myc-His C, pVgRXR (Invitrogen, Inc.) oder die pMAM-Serie (Clontech, Inc.; GenBank Accession No. : U02443) zunennen. Diese stellen bereits das induzierbare regulatorische Kontrollelement beispielsweise für eine chemisch, induzierbare Expression eines DSBI-Enzyms zur Verfügung. In diese Vektoren kann die Nukleinsäuresequenz kodierend für ein DSBI-Enzym direkt insertiert werden.

Vektoren für die Expression in Hefe umfassen beispielhaft pYES2, pYD1, pTEE1/Zeo, pYES2/GS, pPICZ,pGAPZ, pGAPZalph, pPIC9, pPIC3.5, PHIL-D2, PHIL-S1, pPIC3SK, pPIC9K, und PA0815 (Invitrogen, Inc.).

In einer Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Ein anderer Gegenstand der Erfindung betrifft transgenen, pflanzliche Organismen, die das erfindungsgemäße Rekombinationssystem enthalten sowie Zellen, Zellkulturen, Gewebe, Teile oder Vermehrungsgut - wie zum Beispiel bei pflanzlichen Organismen Blätter, Wurzeln, Samen, Früchte, Pollen usw. - abgeleitet von solchen Organismen.

Eukaryotischer Organismus, Ausgangs- oder Wirtsorganismus meint niedere und höhere, einzellige und mehrzellige eukaryotischen Organismen. Umfasst sind auch eukaryotische Mikroorganismen wie beispielsweise Hefen, Algen oder Pilze.

Hefen sind beispielsweise Candida, Saccharomyces, Hansenula oder Pichia, besonders bevorzugt sind Saccharomyces cerevisiae oder Pichia pastoris (ATCC Accession No. 201178).

Pilze sind beispielsweise Aspergillus, Trichoderma, Ashbya, Neurospora, Fusarium, Beauveria oder weitere in Indian Chem Engr. Section B. Vol 37, No 1,2 (1995) auf Seite 15, Tabelle 6 beschriebene Pilze z.B. der filamentöse Hemiascomycet Ashbya gossypii.

Weiterhin sind tierische Organismen und von diesen abgeleitete Zellen oder Gewebebesderieben. Tierische Organismen umfasst bevorzugt Vertebraten und Invertebraten. Besonders bevorzugte Vertrebraten sind Säuger wie in Hund, Katze, Schaf, Ziege, Huhn, Maus, Ratte, Rind oder Pferd. Bevorzugte tierische Zellen umfassen CHO, COS, HEK293 Zellen. Bevorzugte Invertebraten umfassen Insektenzellen wie Drosophila S2 und Spodoptera Sf9 oder Sf21 Zellen.

Bevorzugte Wirts- oder Ausgangsorganismen sind vor allem transgene Pflanzen. Eingeschlossen sind im Rahmen der Erfindung alle Gattungen und Arten höherer und niedrigerer Pflanzen des Pflanzenreiches. Eingeschlossen sind ferner die reifen Pflanzen, Saatgut, Sprosse und Keimlinge, sowie davon abgeleitete Teile, Vermehrungsgut (zumBeispiel Samen oder Früchte) und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium.

Das erfindungsgemäße Rekombinationssystem ist bevorzugt für folgende Pflanzenfamilien verwendbar: Amaranthaceae, Brassicaceae, Carophyllaceae, Chenopodiaceae, Cömpositae, Cucurbitaceae, Labiatae, Leguminosae-Papilionoideae, Liliaceae, Linaceae, Malvaceae, Rosaceae, Saxifragaceae, Scrophulariaceae, Solanacea, Tetragoniacea.

Einjährige, mehrjährige, monocotyledone unddicotyledone Pflanzen sind bevorzugte Wirtsorganismen für die Herstellung transgener Pflanzen. Die Verwendung des erfindungsgemäßen Rekombinations systems bzw. Verfahrens ist ferner vorteilhaft bei allen Schmuckpflanzen, Nutz-oder Zierbäumen, Blumen, Schnittblumen, Sträuchern öder Rasen. Beispielhaft aber nicht einschränkend seien zu nennen Angiospermen, Bryophyten wie zum Beispiel Hepaticae (Leberblümchen) undMusci (Moose) ; Pteridophyten wie Farne, Schachtelhalm und Lycopoden; Gymnospermen wie Koniferen, Cycaden, Ginkgo und Gnetalen; Algen wie Chlorophyceae, Phaeophpyceae, Rhodophyceae, Myxophyceae, Xanthophyceae, Bacillariophyceae (Diatomeen) und Euglenophyceae.

Pflanzen im Rahmen der Beschreibung umfassen beispielhaft und nicht einschränkend die Familien der Rosaceae wie Rose, Ericaceae wie Rhododendrons und Azaleen, Euphorbiaceae wie Weihnachtssterne und Kroton, Caryophyllaceae wie Nelken, Solanaceae wie Petunien, Gesneriaceae wie das Usambaraveilchen, Balsaminaceae wie das Springkraut, Orchidaceae wie Orchideen; Iridaceae wie Gladiolen, Iris, Freesie und Krokus, Compositae wie Ringelblume, Geraniaceae wie Geranien, Liliaceae wie der Drachenbaum, Moraceae wie Ficus, Araceae. wie Philodendron und andere mehr.

Beispielhaft aber nicht einschränkend für Blütenpflanzen seien zu nennen die Familien der Leguminosae wie Erbse, Alfalfa und Soja; Gramineae wie Reis, Mais, Weizen; Solanaceae wie Tabak und andere mehr; die Familie der Umbelliferae, besonders die Gattung Daucus (ganz besonders die Art carota (Karrotte)) und Apium (ganz besonders die Art graveolens dulce (Selarie) und andere mehr; die Familie der Solanacea, besonders die Gattung Lycopersicon, ganz besonders die Art esculentum (Tomate) und die Gattung Solanum, ganz besonders die Art tuberosum (Kartoffel) und melongena (Aubergine) und andere mehr; und die Gattung Capsicum, ganz besonders die Art annum (Pfeffer) und andere mehr; die Familie der Leguminosae, besonders die Gattung Glycine, ganz besonders die Art max (Sojabohne) und andere mehr; und die Familie der Cruciferae, besonders die Gattung Brassica, ganz besonders die Arten napus (Raps), campestris (Rübe), oleraceä cv Tastie (Kohl), oleracea cv Snowball Y (Blumenkohl) und oleracea cv Emperor (Broccoli) ; und der Gattung Arabidopsis, ganz besonders die Art thaliana und andere mehr; die Familie der Compositae, besonders die Gattung Lactuca, ganz besonders die Art sativa (Salat) und andere mehr.

Die transgenen Pflanzen sind insbesondere ausgewählt unter monokotylen Kulturpflanzen, wie zum Beispiel Getreidearten wie Weizen, Gerste, Hirse, Roggen, Triticale, Mais, Reis oder Hafer sowie dem Zuckerrohr. Ferner sind die erfindungsgemäßen transgenen Pflanzen insbesondere ausgewählt unter dikotylen Kulturpflanzen, wie zum Beispiel
Brassicacae wie Raps (B.napus), Kresse, Arabidopsis, Kohlarten oder Canola, Leguminosae wie Soja, Alfalfa, Erbse, Bohnengewächsen oder Erdnuss
Solanaceae wie Kartoffel, Tabak, Tomate, Aubergine oder Paprika, Asteraceae wie Sonnenblume, Tagetes, Salat oder Calendula,
Cucurbitaceae wie Melone, Kürbis oder Zucchini,
sowie Lein, Baumwolle, Hanf, Flachs, Roter Pfeffer, Möhre, Karotte, Zuckerrübe und den verschiedenen Baum-, Nuss- und Weinspecies.

Besonders bevorzugt sind Arabidopsis thaliana, Nicotiana tabacum und Raps sowie alle Gattungen und Arten, die als Nahrungs-oder Futtermittel zum Einsatz kommen, wie die beschriebenen Getreidearten, oder sich zur Herstellung von Ölen eignen, wie Ölsaaten (wie zum Beispiel Raps), Nussarten, Soja, Sonnenblume, Kürbis und Erdnuss.

Pflanzliche Organismen im Sinne der Erfindung sind weiterhin weitere photosynthetisch aktive befähigte Organismen, wie zum Beispiel Algen oder Cyanobakterien, sowie Moose.

bevorzugte Algen sind Grünalgen, wie beispielsweise Algen der Gattung Haematococcus, Phaedactylum tricornatum, Volvox oder Dunaliella.

Die Herstellung eines transformierten pflanzlichen Organismus oder einer transformierten pflanzlichen Zelle erfordert, dass die entsprechende DNA in die entsprechende Wirtszelle eingebracht wird. Für diesen Vorgang, der als Transformation bezeichnet wird, steht eine Vielzahl von Methoden zur Verfügung (siehe auch Keown et al.1990 Methods in Enzymology 185:527-537) . So kann die DNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden. Als bevorzugte allgemeine Methoden seien zu nennen Calciumphosphat vermittelte Transfektion, DEAE-Dextran vermittelte Transfektion, kationische Lipid-vermittelte Transfektion, Elektroporation, Transduktion, Injektion. Derartige Verfahren sind dem Fachmann geläufig und beispielsweise beschrieben bei Davis et al., Basic Methods In Molecular Biolog (1986).

Es werden dem Fachmann geläufige Methoden der Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, biolistische Verfahren wie die Genkanone ("particle bombardment" Methode), die Elektroporation, die Inkubation trockener Embryonen in DNA-haltiger Lösung, die Sonikation und die Mikroinjektion sowie die Transformation intakter Zellen oder Gewebe durch Mikro- oder Makroinjektion in Gewebe oder Embryonen, Gewebeelektroporation, Inkubation trockener Embryonen in DNA-haltiger Lösung oder die Vakuuminfiltration von Samen. Im Falle von Injektion oder Elektroporation von DNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist es nützlich, das sich auf dem Plasmid ein zusätzliches selektionierbares Markergen befindet.

Jedes Pflanzengewebe kann als Zielmaterial dienen. Ebenso kann die Expression in Kallus, embryogenem Gewebe bzw. somatischen Embryonen erfolgen.

Neben diesen "direkten" Transformationstechniken kann eine Transformation auch durch bakterielle Injektion mittels Agrobacterium tumefaciens oder Agrobacterium rhizogenes durchgeführt werden. Diese Stämme enthalten ein Plasmid (Ti bzw. Ri Plasmid). Ein Teil dieses Plasmids, genannt T-DNA (transferred DNA), wird auf die Pflanze nach Agrobacterium-Infektion übertragen und in das Genom der Pflanzenzelle integriert.

Das Rekombinationskonstrukt oder die Expressionskassette für das DSBI-Enzym wird bevorzugt in spezielle Plasmide integriert, entweder in einen Zwischenvektor (englisch: shuttle or intermediate vector) oder einen binären Vektor. Wenn zum Beispiel ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden. Bevorzugt werden binäre Vektoren verwendet. Binäre Vektoren können sowohl in E. coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al., Mol. Gen. Genet. 163 (1978), 181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden.

Die Anwendung von Agrobakterium tumefaciens für die Transformation von Pflanzen unter Verwendung von Gewebekulturexplantaten wurde beschrieben von Horsch et al. (Horsch RB (1986) Proc Natl Acad Sci USA 83(8):2571-2575), Fraley et al. (Fraley et al. 1983, Proc. Natl. Acad. Sci. USA 80, 4803-4807) und Bevans et al. (Bevans et al. 1983, Nature 304, 184-187) . Viele Stämme von Agrobakterium tumefaciens sind in der Lage, genetisches Material - beispielsweise die erfindungsgemäßen Rekombinationskonstrukte - zu übertragen, wie z.B. die Stämme EHA101[pEHA101], EHA105[pEHA105], LBA4404[pAL4404], C58C1 [pMP90] und C58C1[pGV2260] . Der Stamm EHA101 [pEHA101] wurde von Hood et al. (Hood EE et al. (1996) J Bacteriol 168(3):1291-1301), der Stamm EHA105 [pEHA105] von Hood et al. (Hood et al. 1993, Transgenic Research 2, 208-218), der Stamm LBA4404[pAL4404] von Hoekema et äl. (Hoekema et al. 1983, Nature 303, 179-181), der Stamm C58C1[pMP90] von Koncz and Schell (Koncz and Schell 1986, Mol. Gen. Genet. 204, 383-396) und der Stamm C58C1[pGV2260] von Deblaere et al. (Deblaere et al. 1985, Nucl. Acids Res. 13, 4777-4788) beschrieben.

Der für die Transformation eingesetzte Agrobakterienstamm enthält zusätzlich zu seinem entwaffneten Ti-Plasmid ein binäres Plasmid mit der zu übertragenden T-DNA, die in der Regel ein Gen für die Selektion der transformierten Zellen und das zu übertragende Gen enthält. Beide Gene müssen mit transkriptiönalen und translationalen Initiations-und Terminationssignalen ausgestattet sein. Das Binärplasmid kann beispielsweise durch Elektroporation oder andere Transformationsmethoden in den Agrobakterienstamm übertragen werden (Mozo & Hooykaas 1991, Plant Mol. Biol. 16, 917-918). Die Cokultur der pflanzlichen Explantate mit dem Agrobakterienstamm findet in der Regel für zwei bis drei Tage statt.

Verschiedene Vektoren waren bzw. sind verwendbar. Grundsätzlich kann zwischen solchen Vektoren unterschieden werden, die für die Agrobakterien-vermittelte Transformation bzw. Agroinfektion eingesetzt werden können, d.h. die Rekombinationskonstrukte bzw, die Expressionskassette für die Expression des DSBI-Enzyms, innerhalb einer T-DNA enthalten, was sogar die stabile Integration der T-DNA ins Pflanzengenom zulässt. Außerdem können Bordersequenz-freie Vektoren eingesetzt werden, die beispielsweise durch Partikelbeschuss in die Pflanzenzellen transformiert werden können und dort sowohl zu einer transienten als auch zu einer stabilen Expression führen können.

Die Verwendung von T-DNA zur Transformation pflanzlicher Zellen ist intensiv untersucht und beschrieben (EP 120516: Hoekema, In: The Binary Plant Vector System, Offsetdrukkerij Kanters B. V., Alblasserdam, Chapter V; Fraley et al., Crit. Rev. Plant. Sci., 4:1-46 and An et al., EMBO J. 4 (1985), 277-287). Verschiedene binäre Vektoren sind bekannt und teilweise kommerziell erhältlich wie zum Beispiel pBIN19 (Clontech Laboratories, Inc. USA).

Für den Transfer der DNA auf die pflanzliche Zelle werden pflanzliche Explantate mit Agrobacterium tumefaciens oder Agrobacterium rhizogenes kokultiviert. Ausgehend von infiziertem Pflanzenmaterial (z.B.Blatt-, Wurzel- oder Stengelteile, aber auch Protoplasten oder Suspensionen von Pflanzenzellen) können ganze Pflanzen unter Verwendung eines geeigneten Mediums, dass zum Beispiel Antibiotika oder Biozide zur Selektion transformierten Zellen enthalten kann, regeneriert werden. Die erhaltenen Pflanzen können dann auf die Präsenz der eingeführten DNA, hier des erfindungsgemäßen Rekombinationskonstruktes oder der Expressionskassette für das DSBI-Enzym, durchmustert werden. Sobald die DNA in das Wirtsgenom integriert ist, ist der entsprechende Genotyp in der Regel stabil und die entsprechende Insertion wird auch in den Nachfolgegenerationen wiedergefunden. In der Regel enthält die integrierte Expressionskassette einen Selektionsmarker, der der transformierten Pflanze eine Resistenz gegen ein Biozid (zum Beispiel ein Herizid) oder ein Antibiotikum wie Kanamycin, G 418, Bleomycin, Hygromycin oder Phosphinotricin etc. verleiht. Der Selektionsmarker erlaubt die Selektion von transformierten Zellen von untransformierten (McCormick et al., Plant Cell Reports 5 (1986), 81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993), 128 - 143 sowie in Potrykus, Annu. Rev. Plant Physiol. Plant Molec. Biol. 42 (1991), 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984), 8711).

Die Agrobacterium-vermittelte Transformation ist am besten für dicotyledone Pflanzenzellen geeignet, wohingegen die direkten Transformationstechniken sich für jeden Zelltyp eignen.

Transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide zu verleihen vermag. Transformierte Zellen, die ein solches Markergen exprimieren, sind in der Lage in der Gegenwart von (Konzentrationen eines entsprechenden Antibiotikums oder Herbizides zu überleben, die einen untransformierten Wildtyp abtöten. Verschiedene positive und negative Selektionsmarker sind weiter oben beschrieben. Beispiel sind das bar Gen, dass Resistenz gegen das Herbizid Phosphinothricin verleiht (Rathore KS et al., Plant Mol Biol. 1993 Mar; 21 (5) : 871-884) das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht.

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Terner verden von den oben beschriebenen transgenen Organismen abgeleitete Zellen, Zellkulturen, Teile - wie zum Beispiel bei transgenen pflanzlichen Organismen Wurzeln, Blätter etc.-, und transgenes Vermehrungsgut (wie Samen oder Früchte).

Von Menschen und Tieren verzehrbare genetisch veränderte Pflanzen können auch beispielsweise direkt oder nach an sich bekannter Aufbereitung als Nahrungsmittel oder Futtermittel verwendet werden. Hier ist eine Deletion von beispielsweise Antibiotika- und/oder Herbizidresistenzen, wie sie oft bei der Erzeugung der transgenen Pflanzen eingeführt werden, aus Gründen der Kundenakzeptanz aber auch der Produktsicherheit sinnvoll.

Desseiteren wird die Verwendung der oben beschriebenen erfindungsgemäßen, transgenen Organismen und der von ihnen abgeleitete Zellen, Zellkulturen, Teile - wie zum Beispiel bei transgenen pflanzlichen Organismen Wurzeln, Blätter etc.-, und transgenes Vermehrungsgut wie Samen oder Früchte, zur Herstellung von Nahrungs- oder Futtermitteln, Pharmazeutika oder Feinchemikalien beschrieben. Auch hier ist eine Deletion von beispielsweise Antibiotika- und/oder Herbizidresistenzen aus Gründen der Kundenakzeptanz aber auch der Produktsicherheit vorteilhaft.

Feinchemikalien meint Enzyme, Vitamine, Aminosäuren, Zucker, Fettsäuren, natürliche und synthetische Geschmacks-, Aromaund Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakkzinen ist beschrieben (Hood EE, Jilka JM. (1999) Curr Opin Biotechnol. 10(4): 382-386; Ma JK und Vine ND (1999) Curr Top Microbiol Immunol.236:275-92).

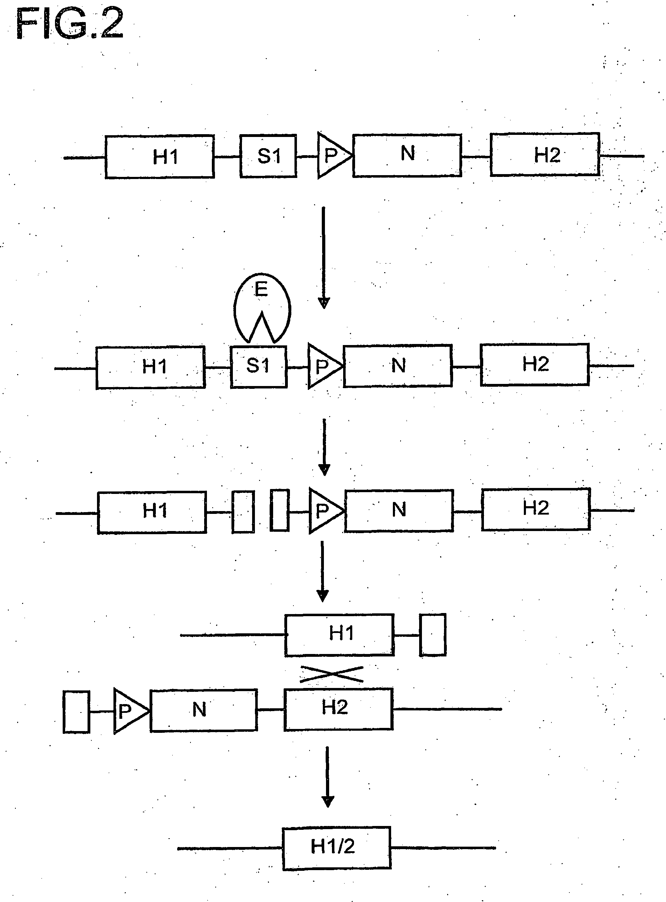

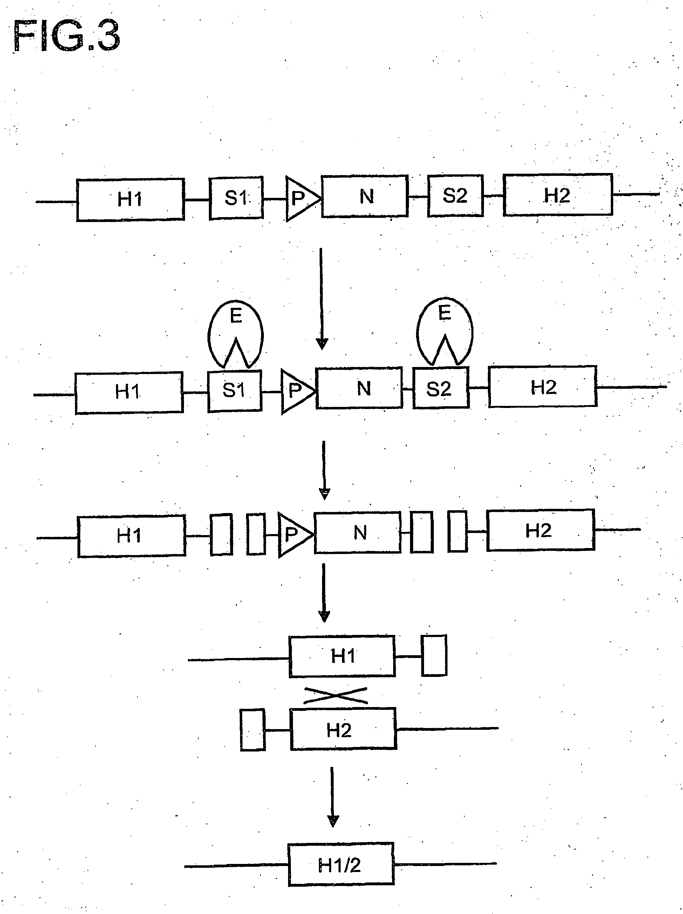

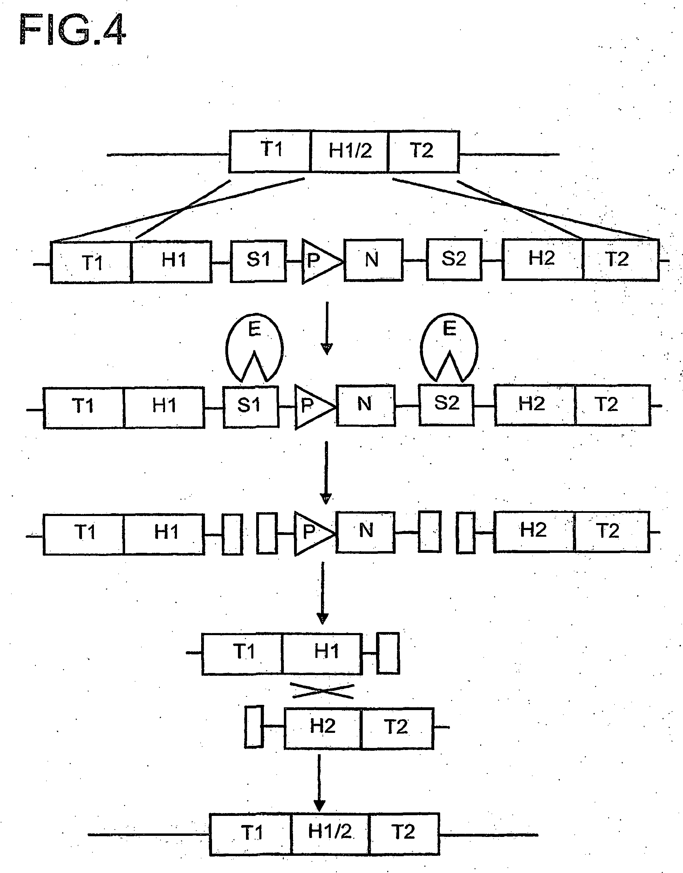

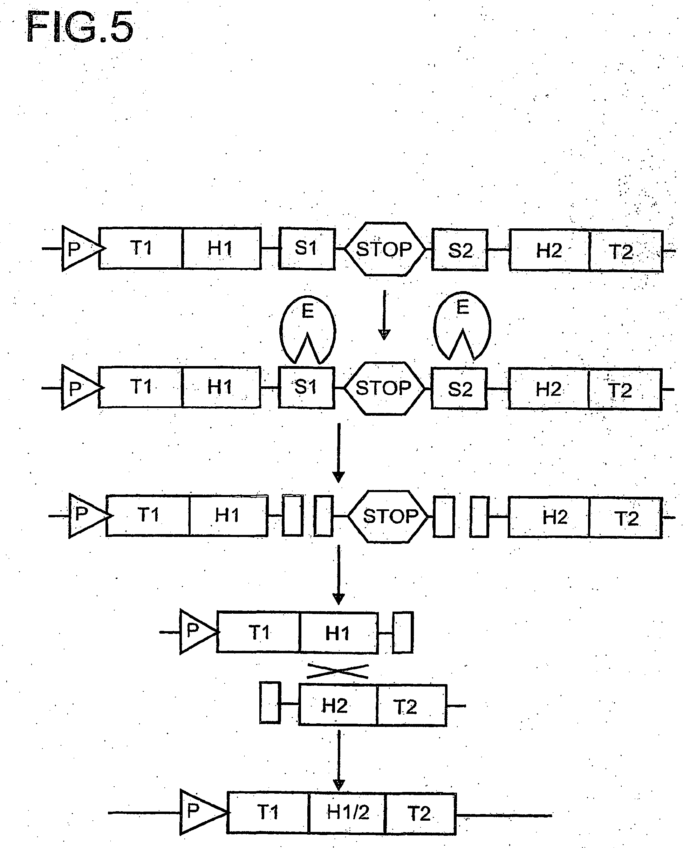

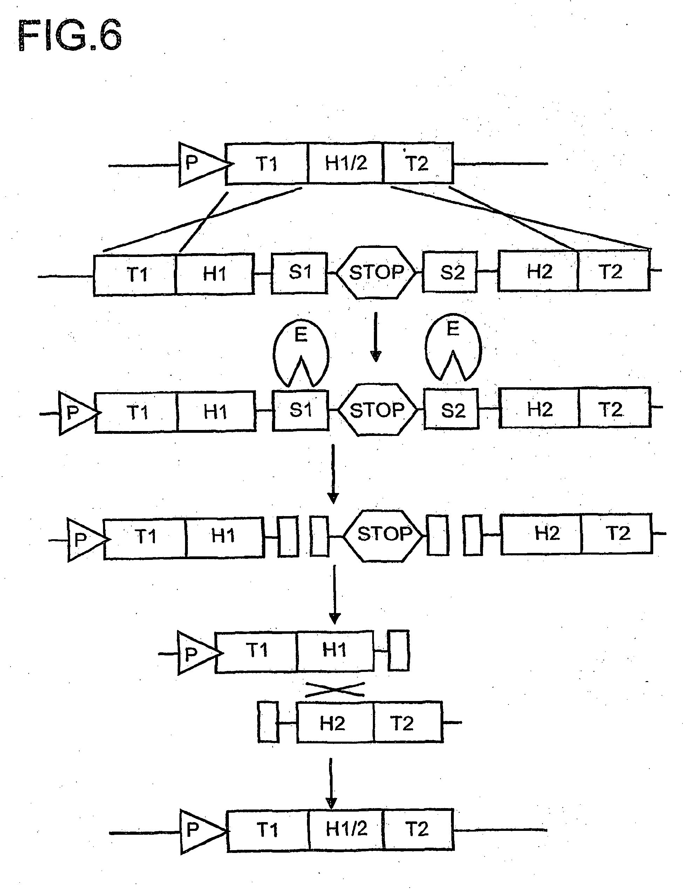

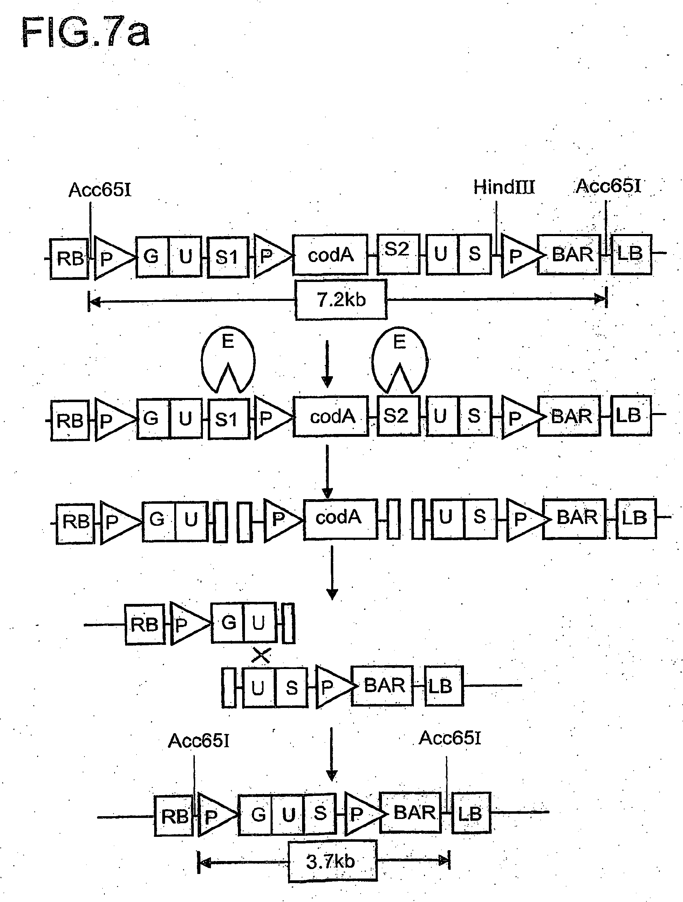

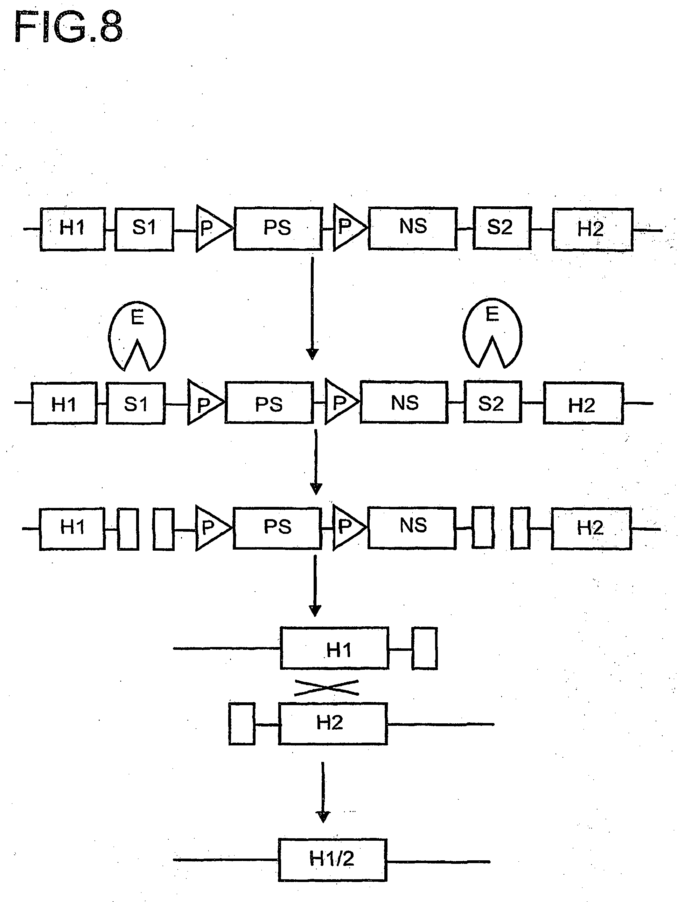

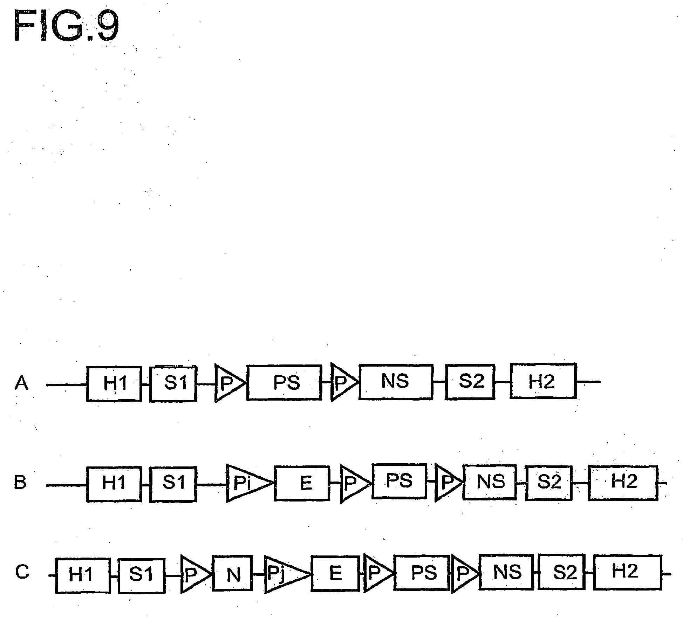

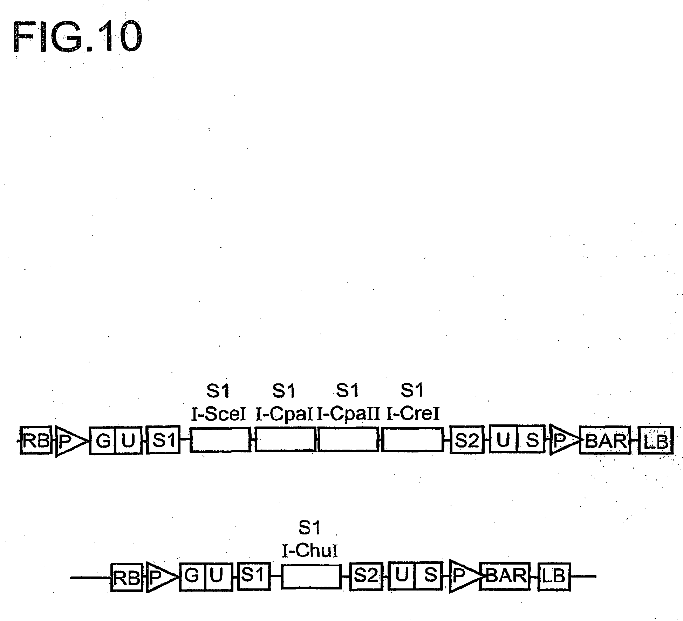

Ferner bietet das erfindungsgemäße Rekombinations system bzw. Verfahren verschiedene vorteilhafte Anwendungsmöglichkeiten, die sich mit den im Stand der Technik beschriebenen Deletionsverfahren nicht erreichen lassen. Verschiedene Anwendungsbeispiele sind nachfolgend beispielhaft, beschrieben:
1. Einfache Deletion einer Nukleinsäuresequenz aus der chromosomale DNA eines pflanzlichen Organismus:
   Unter Verwendung beliebiger Homologiesequenzen A und B können zwischen diesen lokalisierten Nukleinsäuresequenzen deletiert werden. Die aus den Homologiesequenzen A und B rekombinierte Sequenz verbleibt im Genom. Das Verfahren eignet sich beispielsweise, um Selektionsmarker nach der Herstellung eines transgenen pflanzlichen Organismus - beispielsweise einer transgenen Pflanze - wieder aus der chromosomalen DNA zu entfernen. Das Verfahren ist schematisch in Fig. 2 und 3 dargestellt, wobei in Fig. 2 die Variante mit einer Erkennungssequenz zur gezielten Induktion von DNA-Doppelstrangbrüchen und in Fig. 3 die Variante mit zwei Erkennungssequenzen zur gezielten Induktion von DNA-Doppelstrangbrüchen wiedergegeben ist.
2. Vollständige Deletion transgen eingeführter heterologer Nukleinsäuresequenzen aus der chromosomalen DNA eines pflanzlichen Organismus:
   Unter Verwendung von Homologiesequenzen A und B, die zu bestimmten Sequenzen des pflanzlichen Organismus homolog sind, kann das Expressionskonstrukt durch homologe Rekombination in den pflanzlichen Organismus eingeführt werden. Unter Verwendung des erfindungsgemäßen Rekombinationssystems bzw. Verfahrens würden die zwischen den Homologiesequenzen lokalisierten Nukleinsäuresequenzen deletiert werden. Die induzierte homologe Rekombination zwischen Homologiesequenzen A und B stellt die ursprüngliche Sequenz wieder her. Das Konstrukte wird rückstandslos aus der chromosomalen DNA entfernt. Das Verfahren eignet sich beispielsweise, um Selektionsmarker nach der Herstellung einer transgenen Pflanze wieder aus der chromosomalen DNA zu entfernen. Ferner ist das erfindungsgemäße System bzw. Verfahren dazu geeignet bestimmte Proteine zum Erreichen eines vorteilhaften Effektes vorübergehend zu exprimieren und - unter Verwendung einer induzierten DSBI-Enzym Expression oder Aktivität - wieder abzuschalten, indem das entsprechende Gen irreversibel wieder aus dem genom entfernt wird. Das Verfahren ist schematisch in Fig. 4 dargestellt, wobei hier die Variante mit zwei Erkennungssequenzen zur gezielten Induktion von DNA-Doppelstrangbrüchen wiedergegeben ist. Das System lässt sich auch mit einer Erkennungssequenz realisieren, bei größeren Insertionen zwischen den Homologiesequenzen A und B sind jedoch zwei Schnittstellen vorteilhaft, da so die Effizienz der Deletion und homologen Rekombination weiter gesteigert werden kann. (Innerhalb des zu deletierenden Sequenzbereiches können weitere Erkennungssequenzen lokalisiert sein.)
3. Induzierte Genaktivierung durch gezielte Deletion von Nukleinsäuresequenzen:
   Unter Verwendung von Homologiesequenzen A und B, deren homologe Rekombination beispielsweise ein vollständiges offenes Leseraster eines Proteins bzw. einen funktionsfähigen Promotors wiederherstellt, kann - abhängig von der Gegenwart des DSBI-Enzyms - dieinduzierbare Expression von Zielproteinen realisiert werden. Unter Verwendung des erfindungsgemäßen Rekombinationssystems bzw. Verfahrens würden die zwischen den Homologiesequenzen lokalisierten Nukleinsäuresequenzen deletiert werden. Das Verfahren ist schematisch in Fig. 5 und 6 dargestellt, wobei in Fig. 6 eine spezielle Ausführungsform von dem in Fig. 5 dargestellten allgemeinen Verfahren wiedergegeben wird, bei dem das Rekombinationskonstrukt zuvor durch homologe Rekombination in ein endogenes Gen insertiert wird und dieses dadurch - abhängig von der Gegenwart des DSBI-Enzyms - induzierbar aktiviert werden kann. Fig. 7a verdeutlicht das System der Genaktivierung an einem konkreten Ausführungsbeispiel, bei dem unter Verwendung des erfindungsgemäßen Systems bzw. Verfahrens das Gen der β-Glucuronidase (GUS) rekonstituiert wird, was eine Farbreaktion ermöglicht (s. Beschreibung zu Fig. 7a und Beispiele).
4. Leicht selektionierbares System zur Deletion einer Nukleinsäuresequenz aus der chromosomalen DNA eines pflanzlichen Organismus:
   In einer bevorzugten Ausführungsform enthält das Rekombinationskonstrukt einem positiven und einem negativen Selektionsmarker (und ggf. weitere zu deletierende Nukleinsäuresequenzen) derart, dass bei Induktion der Doppelstrangbrüche beide Marker deletiert werden. Ein entsprechendes System ist in Fig. 8 und 9 (A) dargestellt. Weiterhin kann auch die Expressionskassette für das DSBI-Enzym zwischen den Homologiesequenzen enthalten sein (Fig. 10 (B)), wobei die Expression bevorzugt unter der Kontrolle eines Induzierbaren Promotors (Pi) (z.B.: AoyamaTundChuaNH (1997) Plant J11:605-612; Caddick MX et al. (1998) Nat. Biotechnol 16:177-180) realisiert wird. Wie bereits beschrieben können weitere Nukleinsäuresequenzen enthalten sein (Fig. 9 (C)).
   Die Expression des DSBI-Enzyms führt in allen Fällen dazu, dass die DNA Sequenzen, die zwischen den beiden Erkennungssequenzen liegen, eliminiert werden und die homologen Sequenzen rekombinieren. Da die Zellen gleichzeitig einen negativen Selektionsmarker verlieren, können die Zellen mit einer erfolgreich realisierten Deletion durch Selektion identifiziert werden (Gleave AP et al.(1999) Plant Mol Biol. 40:223-235).
   Aus den so erhaltenen pflanzlichen Zellen können die entsprechenden vollständigen Pflanzen regeneriert und vermehrt werden, die nun keinerlei Markergene mehr enthalten.
5. Genetische Manipulation des Wirtsgenoms:
   Das erfindungsgemäße Rekombinationssystem bzw. Verfahren kann zu in situ Modifikationen des Wirtsgenoms verwendet werden. So kann beispielsweise eine Homologiesequenzen bereits endogen im Genom vorliegen. Nach Insertion der zweiten Homolögiesequenz verknüpft mit einer DSBI-Enzym-Erkennungssequenz werden etwaige zwischen den Homologiesequenzen A und B gelegene regulatorische oder codierende Sequenzen aus dem Genom entfernt.
   Gleichzeitig ist es denkbar, dass das Rekombinationskonstrukt regulatorische oder codierende Sequenzen umfaßt, die nach der Deletion wieder aus dem Organismus entfernt werden. So kann ein endogenes Gen beispielsweise vorübergehend gezielt reguliert werden.

In einer weiteren Ausführungsform wird die Effizienz des Rekombinationssystems gesteigert durch Kombination mit Systemen, die die homologe Rekombination fördern. Solche Systeme sind beschrieben und umfassen beispielhaft die Expression von Proteinen wie RecA oder die Behandlung mit PARP-Inhibitoren. Es konnte gezeigt werden, dass die intrachromosomale homologe Rekombination in Tabakpflänzen durch die Verwendung von PARP-Inhibitoren erhöht werden kann (Puchta H et al. (1995) Plant J. 7:203-210). Durch den Einsatz dieser Inhibitoren kann die Rate der homologen Rekombination in den Rekombinationskonstrukten nach Induktion des sequenz spezifischen DNA-Doppelstrangbruches und damit die Effizienz der Deletion der Transgensequenzen weiter erhöht werden. Verschiedene PARP Inhibitoren können dabei zum Einsatz kommen. Bevorzugt umfasst sind Inhibitoren wie 3-Aminobenzamid, 8-Hydroxy-2-methylquinazolin-4-on (NU1025), 1,11b-Dihydro-[2H]benzopyrano[4,3,2-de]isoqüinolin-3-on (GPI6150), 5-Aminoisoquinolinon, 3,4-Dihydro-5-[4-(1-piperidinyl)butoxy]-1 (2H)-isoquinolinon oder die in WO 00/26192, WO 00/29384, WO 00/32579, WO 00/64878, WO 00/68206, WO 00/67734, WO 01/23386 und WO 01/23390 beschriebenen Verbindungen.

Daneben konnten verschiedene homologe Rekombinationsreaktionen in Pflanzen durch die Expression des RecA Gens von E. coli in ihrer Frequenz erhöht werden (Reiss B et al. (1996) Proc Natl Acad Sci USA 93(7) :3094-3098) . Auch wird bei Anwesenheit des Proteins das Verhältnis von homologer zu illegitimer DSB Reparatur zugunsten der homologen Reparatur verschoben (Reiss B et al. (2000) Proc Natl Acad Sci USA 97(7):3358-3363). Verwiesen sei auch auf die in WO 97/08331 beschriebenen Verfahren zur Steigerung der homologen Rekombination in Pflanzen. Eine weitere Steigerung der Effizienz des Rekombinationssystem könnte durch die gleichzeitige Expression des RecA Gens oder anderer Gene die die homologe Rekombinationseffizienz erhöhen (Shalev G et al. (1999) Proc Natl Acad Sci USA 96 (13) : 7398-402) erreicht werden. Die oben angegebenen Systeme zur Förderung der homologen Rekombination können auch dort vorteilhaft eingesetzt werden, wo das Rekombinationskonstrukt durch homologe Rekombination gezielt in das Genom eines eukaryotischen Organismus eingeführt werden soll.

### Sequenzen

1. SEQ ID NO:1
   Nukleinsäuresequenz für die I-SceI Homing-Endonuklease.
2. SEQ ID NO:2
   Proteinsequenz für die I-SceI, Homing-Endonuklease.
3. SEQ ID NO:3
   Nukleinsäuresequenz für Fusionsprotein aus I-ChuI Homing-Endonuklease und N-terminaler Kernlokalisationssequenz.
4. SEQ ID NO:4
   Proteinsequenz für Fusionsprotein aus I-ChuI Homing-Endonuklease und N-terminaler Kernlokalisationssequenz.
5. SEQ ID NO:5
   Nukleinsäuresequenz für Fusionsprotein aus I-CreI Homing-Endonuklease und N-terminaler Kernlokalisationssequenz.
6. SEQ ID NO:6
   Proteinsequenz für Fusionsprotein aus I-CreI Homing-Endonuklease und N-terminaler Kernlokalisationssequenz.
7. SEQ ID NO:7
   Nukleinsäuresequenz für Fusionsprotein aus I-CpaI Homing-Endonuklease und N-terminaler Kernlokalisationssequenz.
8. SEQ ID NO:8
   Proteinsequenz für Fusionsprotein aus I-CpaI Homing-Endonuklease und N-terminaler Kernlokalisationssequenz.
9. SEQ ID NO:9
   Nukleinsäuresequenz für Fusionsprotein aus I-CpaII Homing-Endonuklease und N-terminaler Kernlokalisationssequenz.
10. SEQ ID NO:10
   Proteinsequenz für Fusionsprotein aus I-CpaII Homing-Endonuklease und N-terminaler Kernlokalisationssequenz.
11. SEQ ID NO: 11: Oligonukleotid-Primer OPN1
   5'-CGG CTC GAG CTA CGG GGA CGA TTT CTT TTT TTC AC-3'
12. SEQ ID NO: 12: Oligonukleotid-Primer OPN2
13. SEQ ID NO: 13: Oligonukleotid-Primer OPN3
14. SEQ ID NO: 14: Oligonukleotid-Primer OPN4 5'-CGG CTC GAG TTA CTC GCC AGT TTC TTC AAA ACG-3'
15. SEQ ID NO: 15: Oligonukleotid-Primer OPN5
16. SEQ ID NO: 16: Oligonukleotid-Primer OPN6
   5'-CGG CTC GAG CTA AAG GTG GCC TTT ATT GCC ATC AG-3'
17. SEQ ID NO: 17: Oligonukleotid-Primer OPN7
18. SEQ ID NO: 18: Oligonukleotid-Primer OPN8
   5'-CGG CTC GAG CTA AAG GTG GCC TTT ATT GCC ATC AG-3'
19. SEQ ID NO: 19: Oligonukleotid-Primer OPN9
20. SEQ ID NO: 20: Oligonukleotid-Primer OPN10
21. SEQ ID NO: 21: Oligonukleotid-Primer OPN11
   5'- CGG AAG CTT CGT CAC CAA TCC CAA TTC GAT CTA C - 3'
22. SEQ ID NO: 22: Oligonukleotid-Primer OPN12
   5'- CGG AAG CTT CCA CTT GCA AAG TCC CGC TAG TGC C - 3'
23. SEQ ID NO: 23: Oligonukleotid-Primer OPN13
   5'-CGG AAG CTT CGT CAC CAA TCC CAA TTC GAT CTA C - 3'
24. SEQ ID NO: 24: Oligonukleotid-Primer OPN14
   5`- CGG AAG CTT CCA CTT GCA AAG TCC CGC TAG TGC C - 3'
25. SEQ ID NO: 25: Oligonukleotid-Primer OPN15
26. SEQ ID NO: 26: Oligonukleotid-Primer OPN16
27. SEQ ID NO: 27: Oligonukleotid-Primer OPN17
28. SEQ ID NO: 28: Oligonukleotid-Primer OPN18
29. SEQ ID NO: 29: Kernlokalisationssequenz NLS1
   N-Pro-lys-Thr-Lys-Arg-Lys-Val-C
30. SEQ ID NO: 30: Kernlokalisationssequenz NLS2
   N-Pro-Lys-Lys-Lys-Arg-Lys-Val-C (SEQ ID NO: 30)

### Abbildungen

Für die Abbildungen gelten allgemein nachfolgende Abkürzungen:
H1: Homologiesequenz A
H2: Homologiesequenz B
H1/2: Sequenz als Ergebnis der homologen Rekombination aus H1 und H2
S1: erste Erkennungssequenz zur gezielten Induktion von DNA-Doppelstrangbrüchen
S2: zweite Erkennungssequenz zur gezielten Induktion von DNA-Doppelstrangbrüchen
E: DSBI-Enzym
P: Promotor oder anderes genetisches Kontrollelement
N: weitere Nukleinsäuresequenz
NS: Negativer Selektionsmarker
PS: Positiver Selektionsmarker
T1: Vorderer Teil beispielsweise eines Gens oder offenen Leserasters
T2: Hinterer Teil beispielsweise eines Gens oder offenen Leserasters
STOP: Unterbrechung eines Gens oder offenen Leserasters durch beispielsweise Stop-Kodons oder Verschiebung des Leseratsers.

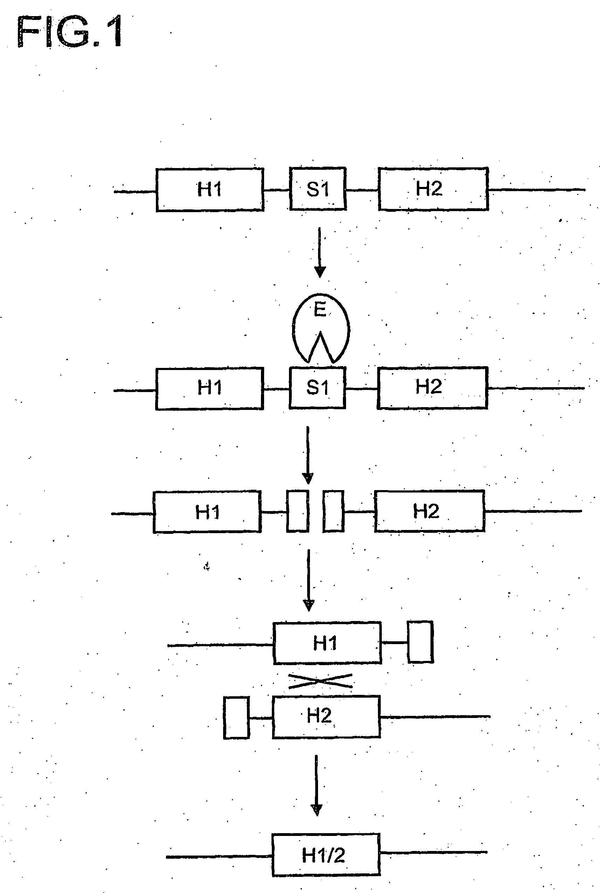

Fig. 1: Darstellung des Prinzips der Erfindung Sequenzen im Genom können effizient eliminiert werden, wenn sie von den Homologiesequenzen H1 und H2 flankiert sind und sich zwischen den Homologiesequenzen eine Schnittstelle (S1) für ein PSBI-Enzym befindet. Durch Einwirkung des DSBI-Enzyms (E) auf diese Rekombinationskassette (H1-S1-H2) kommt es nach Bildung von Doppelstrangbrüchen an der Schnittstelle S1 und zur Eliminierung der zwischen H1 und H2 gelegenen Sequenzen.
Fig. 2: Bevorzugte Ausführungsform Sequenzen - hier beispielsweise eine Expressionskassette bestehend aus einem Promotor (P) und einer zu exprimierenden weiteren Nukleinsäuresequenz (N) (beispielsweise einem Selektionsmarker) - können effizient aus der chromosomalen DNA eliminiert werden, wenn sie von den Homologiesequenzen H1 und H2 flankiert sind und sich zwischen den Homologiesequenzen eine Schnittstelle (S1) für ein DSBI-Enzym befindet. Durch Einwirkung des DSBI-Enzyms (E) auf diese Rekombinationskassette (H1-S1-P-N-H2) kommt es nach Bildung von Doppelstrangbrüchen an der Schnittstelle S1 und zur Eliminierung der zwischen H1 und H2 gelegenen Sequenzen. Die Schnittstelle S1 kann auch hinter oder in der Expressionskassette lokalisiert sein.
Fig. 3: Bevorzugte Ausführungsform Sequenzen - hier beispielsweise eine Expressionskassette bestehend aus einem Promotor (P) und einer zu exprimierenden weiteren Nukleinsäuresequenz (N) (beispielsweise einem Selektionsmarker) - können besonders effizient aus der chromosomalen DNA eliminiert werden, wenn sie von den Homologiesequenzen H1 und H2 flankiert sind und sich vor und hinter der zu deletierenden Nukleinsäuresequenz je eine Schnittstelle (S1 und S2) für ein DSBI-Enzym befindet. Durch Einwirkung des DSBI-Enzyms (E) auf diese Rekombinationskassette (H1-S1-P-N-S2-H2) kommt es nach Bildung von Doppelstrangbrüchen an den Schnittstellen S1 und S2 und zur Eliminierung der zwischen H1 und H2 gelegenen Sequenzen.
Fig. 4: Bevorzugte Ausführungsform Sequenzen - hier beispielsweise eine Expressionskassette bestehend aus einem Promotor (P) und einer zu exprimierenden weiteren Nukleinsäuresequenz (N) (beispielsweise einem Selektionsmarker) - können quasi spurenlos aus der chromosomalen DNA eliminiert werden, wenn das sie umfassende Rekombinationskonstrukt zuvor beispielsweise durch eine homologe Rekombination in das Wirtgenom insertiert wurde. Dabei wird das Gen, bestehend aus den Sequenzabschnittem T1, H1/2 und T2, unterbrochen. Das Rekombinationskonstrukt ist flankiert von zwei Teilen des unterbrochenen Gens (T1-H1 bzw. H2-T2), wobei der mittlere Teil (H1 oder H2) dupliziert wurde, um die homologe Rekombination zu erlauben. Durch Einwirkung des DSBI-Enzyms (E) auf die Schnittstellen (S1 und S2) kommt es zur Induktion von Doppelstrangbrüchen und zur Induktion der homologen Rekombination zwischen den Homologiesequenzen H1 und H2, wodurch zum einen die zwischen H1 und H2 gelegenen Sequenzen deletiert werden, zum anderen das Ausgangsgen wieder hergestellt wird.
Fig. 5: Bevorzugte Ausführungsform Nukleinsäuresequenzen (hier ein Genmit der Sequenz T1-H1/2-T2 unter Kontrolle eines Promotors P) können induzierbar exprimiert werden, indem das intakte Gen erst durch Anwendung des Rekombinationssystems rekonstituiert wird. Das Gen, bestehend aus den Sequenzabschnitten T1, H1/2 und T2, ist-beispielsweise durch Insertion von Stop-Kodons oder anderen Unterbrechungen des Leserasters im Rahmen des Rekombinationskonstruktes- inaktiviert. Das Rekombinationskonstrukt ist flankiert von zwei Teilen des unterbrochenen Gens (T1-H1 bzw. H2-T2), wobei der mittlere Teil (H1 oder H2) dupliziert wurde; um die homologe Rekombination zu erlauben. Durch Einwirkung des DSBI-Enzyms (E) auf die Schnittstellen (S1 und S2) kommt es zur Induktion von Doppelstrangbrüchen und zur Induktion der homologen Rekombination zwischen den Homologiesequenzen H1 und H2, wodurch zum einen die zwischen H1 und H2 gelegenen Sequenzen deletiert werden, zum anderen das intakte Gen hergestellt wird.
Fig. 6: Bevorzugte Ausführungsform Die Abbildung stellt ein Verfahren dar, das dem in Fig. 5 beschriebenen gleicht, nur das hier gezielte ein endogenes Gen aktiviert werden soll, indem das Rekombinationskonstrukt beispielsweise durch eine homologe Rekombination eingeführt wird.
Fig. 7a: Ausführungsbeispiel Die Abbildung verdeutlicht eine konkrete Ausführungsform des in Fig. 6 beschriebenen Verfahrens. Es wird ein Rekombinationskonstrukt über Agrobakterium vermittelte Transfektion eingeführt. Flankiert von der rechten und der linken "Bordersequenz" (RB bzw. LB) enthält das Konstrukt das unterbrochene Leseraster des GUS-Gens (β-Glucuronidase) unter der Kontrolle des 35S Promotors (P) und des Nopalinsythase (nos) Terminator. Die mittlere Region des GUS-Gens (U) wurde dupliziert und stellt die Homolgiesequenzen A undB dar. Zwischen diesen Sequenzen liegt als negative Selektionsmarker das codA-Gen unter Kontrolle des Cauliflower Mosaic Virus (CäMV) 35S Promoters und des Nopalinsynthase (nos) Terminators, flankiert von zwei Erkennungssequenzen des DSBI-Enzyms (S1 und S2). Ferner enthält das Rekombinationskonstrukt noch als positiven Selektionsmarker das BAR-Gen unter Kontrolle des 35S Promoters (P) und 35S Terminators.
Fig. 7a verdeutlicht das infolge der Einwirkung des DSBI-Enzyms Entstehen von Doppelstrangbrüchen und der homologen Rekombination zwischen den homologen U-Sequenzen, wodurch zum einen die zwischen den homologen U-Sequenzen gelegenen Sequenzen deletiert werden, zum anderen das GUS-Gen wieder hergestellt wird. Die Länge des Acc65I-Fragmentes wird dadurch von 7,3 kb auf 3,7 kb verkürzt.

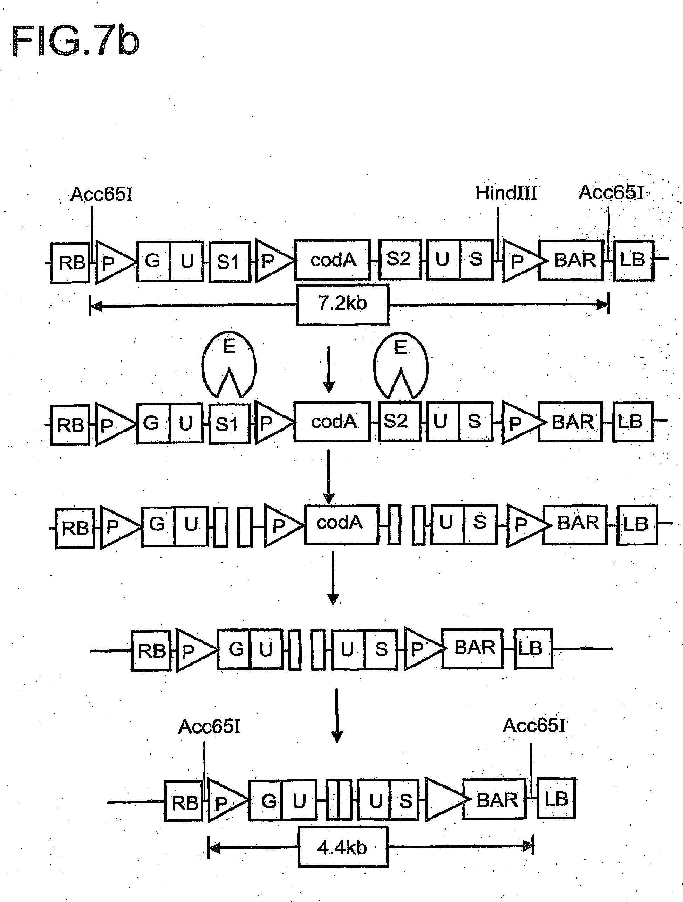

Fig.7b: Stellt das gleiche wie unter Fig. 7a beschriebene System dar. Fig. 7a verdeutlicht das infolge der Einwirkung des DSBI-Enzyms Entstehen von Doppelstrangbrüchen. Im Unterschied zu Fig. 7a erfolgt hier keine homologe Rekombination, sondern eine illegitime durch "non-homologous end-joining". Aufgrund der beiden Schnittstellen wird zwar der zwischen S1 und S2 gelegene Bereich deletiert, das GUS-Gen wird jedoch nicht wieder hergestellt. Die Länge des Acc65I-Fragmentes wird dadurch von 7,3 kb auf 4,4 kb verkürzt.

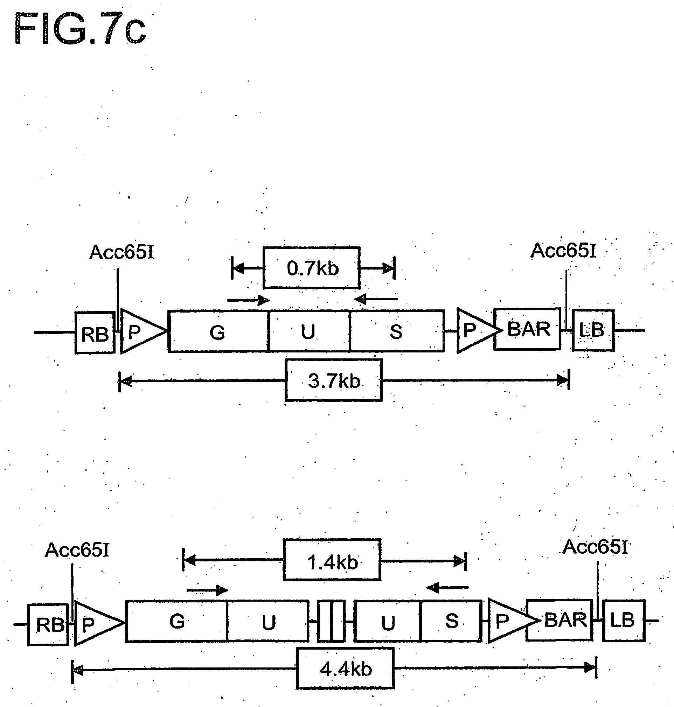

Fig. 7c: Die Abbildung stellt nochmals die beiden Endprodukte der unter den Fig.7a und Fig.7b beschriebenen Abläufe dar.
A: Ergebnis der homologen Rekombination; Acc65I-Fragment hat eine Länge von 3, 7 kb; das mit den Primern OPN13 und OPN14 (verdeutlicht durch die Pfeile) amplifizierte Fragment hat eine Größe von 0,7 kb.
B: Ergebnis der illegitimen Rekombination ("non-homologous end-joining"); Acc65I-Fragment hat eine Länge von 4,4 kb; das mit den Primern OPN13 und OPN14 (verdeutlicht durch die Pfeile) amplifizierte Fragment hat eine Größe von 1,4 kb.

Fig. 8: Bevorzugte Ausführungsform Vorteilhafterweise umfassen die Rekombinationskassetten sowohl einen positiven als auch einen negativen Selektionsmarker (PS bzw. NS) jeweils unter Kontrolle eines Promotors. Der positive Selektionsmarker ist nützlich, um die Einführung des Konstruktes in das Genom zu erleichtern und nachzuweisen. Der negative Selektionsmarker ist nützlich, um die Deletion des Konstruktes aus dem Genom nachzuweisen. Beide Marker werden effizient aus der chromosomalen DNA eliminiert, wenn sie von den Homologiesequenzen H1 und H2 flankiert sind und sich vor und/oder hinter der zu deletierenden Nukleinsäuresequenz je eine Schnittstelle (S1 und S2) für ein DSBI-Enzym befindet. Durch Einwirkung des DSBI-Enzyms (E) auf diese Rekombinationskassette kommt es nach Bildung von Doppelstrangbrüchen an den Schnittstellen S1 und/oder S2 und zur Eliminierung der zwischen H1 und H2 gelegenen Sequenzen. Die Einwirkung eines der genannten DSBI-Enzyms bewirkt. gezielte Doppelstrangbrüche und induziert die homologe Rekombination zwischen den homologen U-Sequenzen, wodurch zum einen die zwischen den homologen U-Sequenzen, gelegenen Sequenzen deletiert werden, zum anderen das GUS-Gen wieder hergestellt wird.
Fig.9: Leicht selektionierbare Systeme zur Deletion einer Nukleinsäuresequenz aus der chromosomalen DNA eines Organismus. Die Konstrukte enthalten einen positiven Selektionsmarker (PS) und negativen Selektionsmarker (NS) jeweils unter Kontrolle eines Promotors (P). (B) enthält zusätzlich eine Expressionskassette für das DSBI-Enzym, wobei die Expression bevorzugt unter der Kontrolle eines induzierbaren Promotors (Pi) realisiert wird. (C) Weitere Nukleinsäuresequenzen können enthalten sein. Die Expression des DSBI-Enzyms führt in allen Fällen dazu, dass die DNA Sequenzen, die zwischen den beiden Erkennungssequenzen liegen, eliminiert werden und die homologen Sequenzen rekombinieren. Da die Zellen gleichzeitig einen negativen Selektionsmarker verlieren, können die Zellen mit einer erfolgreich realisierten Deletion durch Selektion identifiziert werden (Gleave AP et al. (1999) Plant Mol Biol. 40:223-235).
Fig. 10: Die Abbildung verdeutlicht die beiden Konstrukte (SI-Konstrukt (A) und SD-Konstrukt (B)), die verwendet wurde, um den Nachweis zu führen, dass mit verschiedenen Restriktionsenzymen die homologe Rekombination durch Doppelstrangbrüche induziert werden kann. Die Konstrukte werden über Agrobakterium vermittelte Transfektion eingeführt. Flankiert von der rechten und der linken "Bordersequenz" (RB bzw. LB) enthalten die Konstrukte das unterbrochene Leseraster des GUS-Gens (β-Glucuronidase) unter der Kontrolle des 35S Promotors (P) und des Nopalinsynthase (nos) Terminator. Die mittlere Region des GUS-Gens (U) wurde dupliziert und stellt die Homologiesequenzen A und B dar. Zwischen diesen Sequenzen liegen im Falle des SI-Konstruktes (A) die Erkennungssequenzen der DSBI-Enzyme I-SceI; I-CpaI, I-CpaII und I-CreI, im Falle des SD-Konstruktes (B) die Erkennungssequenz des I-ChuI Enzyms. Ferner enthalten die Rekombinationskonstrukte noch als positiven Selektionsmarker das BAR-Gen unter Kontrolle eines Promotors (P).

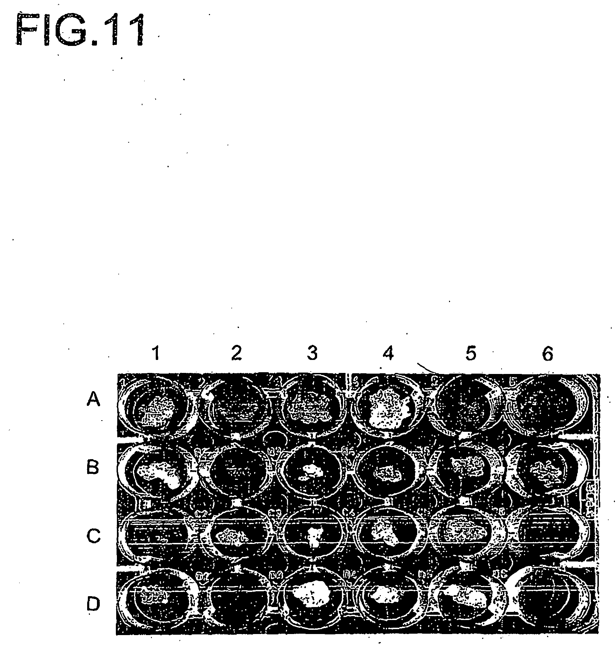

Fig. 11: Repräsentative histochemische Analyse von nach Induktion von Doppelstrangbrüchen erhaltenen Tabakkalli. Blaufärbung (hier Dunkelfärbung) zeigt Expression des ß-Glucuronidasegens und damit die Eliminierung des Selektionsmarkers durch homologe Rekombination an. Blau (Dunkelfärbungen) sind bei den Kalli in den Vertiefungen A2, A5, A6, B2, C1, C6 und D2 zu sehen.

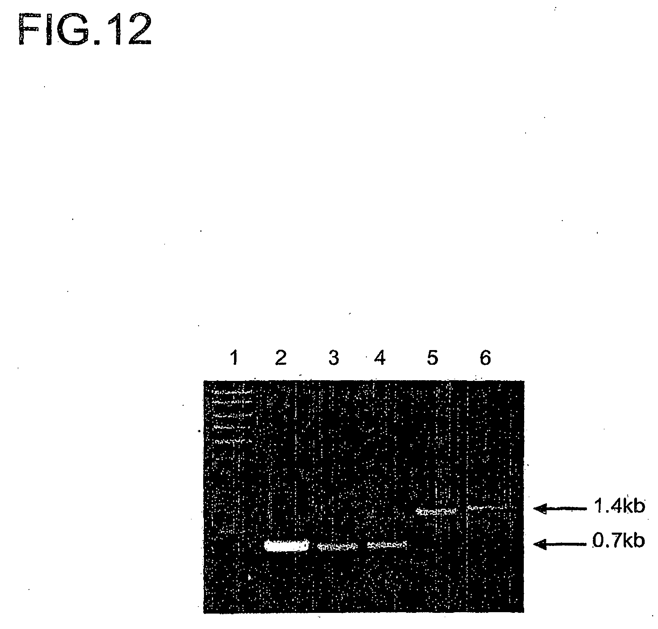

Fig. 12 : PCR Analyse zum Nachweis der homologen Rekombination. PCR mit den Primer OPN13 und OPN14 mit DNA aus Tabakkalli.
In den Spuren 1, 2 und 3 ist das PCR Produkt (Größe 0,7 kb), das homologe Rekombination anzeigt, zu sehen. Die entsprechenden Kalli waren nach histochemischer Färbung Blau, die entsprechenden PCRBanden wurden sequenziert, um zu zeigen, dass das offene Leseraster (ORF) der ß-Glucuronidase tatsächlich durch homologe Rekombination restauriert wurde.
Spur 4 und 5: PCR Produkte (1,4 kb) von nicht blauanfärbaren Kalli bei dem das Transgen durch "non-homologous end-joning" eliminiert wurde.

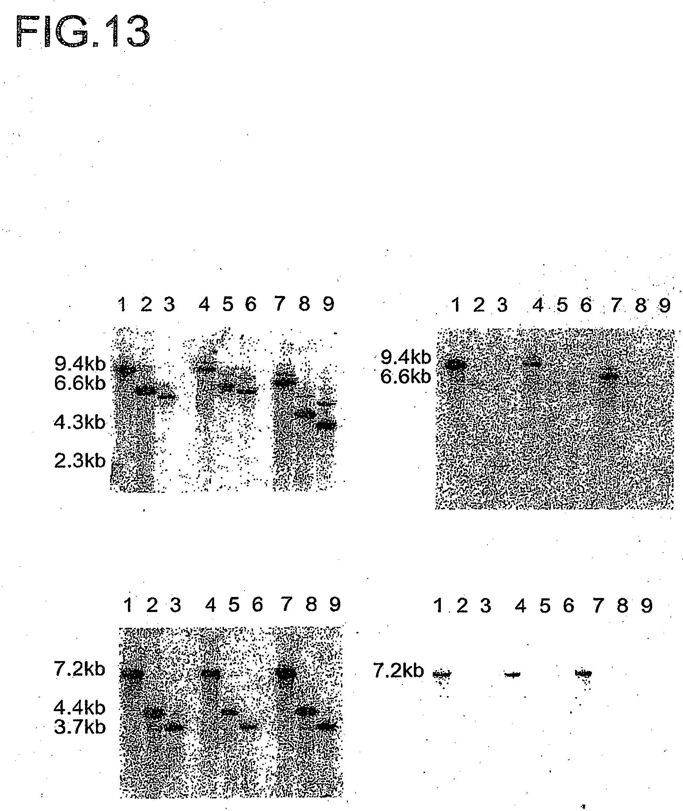

Fig. 13: Southern Blots, die die vollständige Elimination der entsprechenden Transgensequenz anzeigen. Die Spuren der Blots A bis D beinhalten jeweils:

| Spur | Linie | Beschreibung |
|---|---|---|
| 1 | GU.C.USB 1 | Ausgangslinie |
| 2 | GU.C.USB 1-61 | "non-homologous end-joning" |
| 3 | GU.C.USB 1-83 | homologe Rekombination |
| 4 | GU.C.USB 3 | Ausgangslinie |
| 5 | GU.C.USB 3-1 | "non-homologous end-joning" |
| 6 | GU.C.USB 3-3 | homologe Rekombination |
| 7 | GU.C.USB 7 | Ausgangslinie |
| 8 | GU.C.USB 7-14 | "non-homologous end-joning" |
| 9 | GU.C.USB 7-34 | homologe Rekombination |

A: HindIII-verdaute DNA, die mit einer ß-Glucuronidase spezifischen Probe hybridisiert wurde.
B: HindIII-verdaute DNA, die mit einer codA spezifischen Probe hybridisiert wurde.
C: Acc65I-verdaute DNA, die mit einer ß-Glucuronidase spezifischen Probe hybridisiert wurde.
D: Acc65I-verdaute DNA, die miteiner codA spezifischen Probe hybridisiert wurde.
Die Analyse zeigt, dass nach Induktion von DNA-Doppelstrangbrüchen mittels Expression des Restriktionsenzyms sowohl mit homologe Rekombination (Spuren 3, 6 und 9) als auch mit illegitime (Spuren 2, 5 und 8) auftreten kann, wobei stets die zwischen den Restriktionsschnittstellen liegende Transgensequenz (codA) aus dem Pflanzengenom eliminiert wurde.

### Beispiele

### Allgemeine Methoden:

Die chemische Synthese von Oligonukleotiden kann beispielsweise, in bekannter Meise, nach der Phosphoamiditmethode (Voet, Voet, 22. Auflage, Wiley Press New York, Seite 896-897) erfolgen. Die im Rahmen der vorliegenden Erfindung durchgeführten Klonierungsschritte wie z.B. Restriktionsspaltungen, Agorosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer ALF-Express (Pharmacia, Upsala, Schweden) nach der Methode von Sanger (Sanger et al., Proc. Natl. Acad. Sci. USA 74 (1977), 5463-5467).

### Beispiel 1: Klonierung der Homing-Endonukleasen

Die offenen Leseraster (ORFs) der Homing Endonukleasen I-CreI (Wang J et al. (1997) Nucleic Acids Res 25: 3767-3776), I**-**ChuI (Cote V et al. (1993) Gene 129:69-76), I-CpaI (Turmel M et al. (1995a) Nucleic Acids Res 23:2519-2525) und I-CpaII (Turmel M et al. (1995b) Mol. Biöl. Evol. 12, 533-545) wurden aus den entsprechenden Chlamydomonas Stämmen kloniert.

Um die optimale Translation des Gens zu gewährleisten wurden die ORFs der Endonuklaesen mit der "Leader"-Sequenz eines Pflanzenvirus verbunden (CaMV Gen V, wie es sich bei I-SceI bewährt hat; Puchta H (1993) Nucl Acids Res 21:5034-5040). Auch wurde den ORFs eine Kernlokalisationssequenz (NLS2; SEQ ID NO: 30) vorangestellt um das Protein effizient an den beabsichtigten Wirkungsort zu bringen. Beide Elemente (Leader-Sequenz und Kernlokalisationssequenz) wurden über die PCR durch die verwendeten Oligonukleotid-Primer eingebracht.

Zur Isolierung der offenen Leseraster (ORFs) der Endukleasen aus Chlamydomonas wurden von der Sammlung für Algenkultur in Göttingen (Universität Göttingen. Experimentelle Phykologie und Sammlung von Algenkulturen, Albrecht-von-Haller-Institut für Pflanzenwissenschaften, Untere Karspüle 2, D-37073 Göttingen) die Algenkulturen Chlamydomonas reinhardtii/Smith (Stamm Nr. 11-32b); Chlamydomonas applanata/Lucksch (Stamm Nr.: 11-9) und Chlamydomonas segris/King (Stamm Nr. : 9.83) bezogen. Die Kulturen wurden mit Hilfe einer Schüttelkultur in MS Medium angezogen und DNA mit Hilfe des DNeasy Plant Maxi Kit (Qiagen, Hilden) gewonnen.

Aus einer Probe der Algenkultur 11-32b Chlamydomonas reinhardtii/Smith wurde mit Hilfe der Oligonukleotide OPN1 und OPN2 (SEQ ID NO: 11 und 12) der ORF von I-CreI (GenBank Acc.-No.: X01977) amplifiziert.
OPN1 (SEQ ID NO: 11):
   5'-CGG CTC GAG CTA CGG GGA CGA TTT CTT TTT TTC AC- 3'
OPN2 (SEQ ID NO: 12):

Für die PCR-Reaktion wurden 2 ∝l (entsprechend ungefähr 100 ng DNA) der DNA-Präparation eingesetzt. In einem Gesamtvolumen von 50 ∝l werden gemäß den Angaben des Herstellers (Life Technologies) zusammengegeben:
5 ocl 10X PCR Buffer [200 mM Tris-HCl (pH 8,4), 500 mM KCl]
1, 5 ∝l 50 mM MgCl₂
1 ∝l 10 mM dNTP Mix (jeweils 10 mM dATP, dCTP, dGTP und dTTP)
1 ocl Primer OPN1 (10 ∝M)
1 ocl Primer OPN2 (10 ∝M)
0,4 ∝l Taq DNA polymerase (5 U/∝l)
2 ∝l DNA-Präparation
38,1 ∝l autoklaviertes, distilliertes Wasser

Das Reaktionsgemisch wird mit ca. 50 ocl Silikonöl überschichtet und nachfolgendem Temperaturprogramm ausgesetzt (Thermocycler: MWG Biotech Primus HT; MWG Biotech, Deutschland):

| | |
|---|---|
| 1 | Zyklus mit 180 sec bei 9S_C |
| 30 | Zyklen mit 92_C für 60 sec, 54_C für 60 sec und 72_C für 3 min. |
| 1 | Zyklus mit 72_C für 5 min. |

Das PCR-Fragment wurde über Agarosegelelektrophorese und unter Verwendung des QIAquickR Gel Extraction Kits (Qiagen, Hilden, Deutschland) aufgereinigt und in den pGEM-T Easy Vector (Promega, Madison, USA) kloniert. Anschließendend wurde eine Sequenzanalyse mit dem DNA-Sequenzierungsgerät ALF-Express (Pharmacia, Upsala, Schweden) durchgeführt. Die Sequenz ist in SEQ ID NO: 5 dargestellt.

Analog wie für I-CreI wurde auch die Klonierung des ORF von I-CpaI aus der Algenkultur 9.83 Chlamydomonas segris/King (Genbank Acc.-No. : L36830) durchgeführt. Für die PCR wurden die Oligonukleotiden OPN3 und OPN4 verwendet. Die Sequenz ist in SEQ ID NO: 7 dargestellt.
OPN3 (SEQ ID NO: 13):
OPN4 (SEQ ID NO: 14):
   5'-CGG CTC GAG TTA CTC GCC AGT TTC TTC AAA ACG-3'

Analog wie für I-CreI wurde auch die Klonierung des ORF von I-CpaII durchgeführt (Genbank Acc.-No: L39865). Dazu wurde eine Probe der Algenkultur 9.83 Chlamydomonas segris/King verwendet. Für die PCR wurden die Oligonukleotiden OPN5 und OPN6 verwendet. Die Sequenz ist in SEQ ID NO: 9 dargestellt.
OPN5 (SEQ ID NO: 15):
OPN6 (SEQ ID NO: 16):
   5'-CGG CTC GAG CTA AAG GTG GCC TTT ATT GCC ATC AG-3'

Analog wie für I-CreI wurde auch die Klonierung des ORF von I-ChuI aus der Algenkultur Nr. 11-9 Chlamydomonas applanata/Lucksch (Genbank Acc.-No. : L06107) durchgeführt. Für die PCR wurden die Oligonukleotiden OPN7 und OPN8 verwendet. Die Sequenz ist in SEQ ID NO: 3 dargestellt.
OPN7 (SEQ ID NO: 17):
OPN8 (SEQ ID NO: 18):
   5'-CGG CTC GAG CTA AAG GTG GCC TTT ATT GCC ATC AG-3')

Der ORF der einzelnen Homing-Endonukleasen (mit dem Kernlokalisationssignal) wurde jeweils aus den jeweiligen pGEM-T Easy Vector durch SalI Restriktionsverdaus herausgeschnitten, gelelektrophoretisch aufgereinigt und jeweils in die SalI Restriktionsschnittstelle des binären Vektors pBinAR (Höfgen und Willmitzer (1990) Plant Science 66:221-230) kloniert. Die Expression der einzelnen Enzyme erfolgt unter Kontrolle des 35S Promotors und des Octopine Synthase Terminators.

Der binäre I-*Sce*I Expression Vector pCISceI (Puchta H et al. (1996) Proc. Natl. Acad. Sci. USA 93:5055-5060) enthält einen synthetischen I-*Sce*I ORF unter der Kontrolle des CaMV 35S Promotors (Puchta H et al. (1993) Nucl Acids Res 21: 5034-5040) zwischen den T-DNA "Borders".

Alle fünf Plasmide wurden in *E*. coli vermehrt, mit dem QIAfilter Plasmid Midi Kit (Qiagen, Hilden) aufgereinigt und mittels Elektroporation in den Agrobakteriumstamm C58 überführt.

### Beispiel 2: Herstellung des Konstruktes pGU.I.USB

Zur Konstruktion der Rekombinationssubstrate wurde das Plasmid pGU. US (Tinland B et al. (1994) Proc. Natl. Acad. Sci. USA 91:8000-8004) verwendet. Das Plasmid enthält im Bereich der T-DNA zwei überlappende Hälften des ß-Glucuronidase (GUS) Gens, die eine Überlappung von 557 bp aufweisen. Zwischen den GUS Sequenzen ist in eine unikale XbaI Schnittstelle ein Hygromycin-Gen integriert.

In einem ersten Schritt wurde das BAR Gen mit Promotor und Terminatorsequenzen als isoliertes HindIII Fragment aus dem Vektor pRC (Puchta H et al. (1996) Proc Natl Acad Sci USA 93:5055-5060) herausgeschnitten, über Agarosegelelektrophorese vom der Vektorsequenz abgetrennt, aus dem Gel ausgeschnitten, mit Hilfe des QIAquickR Gel Extraction Kits (Qiagen, Hilden, Deutschland) isoliert und danach in die unikale HindIII Schnittstelle von pGU.US inseriert. Dazu wurde zuvor der Vektor pGU.US mit HindIII geschnitten und mit Alkalischer Phosphatase (Calf Intestinal Alkaline Phosphatase (CIP), New England Biolabs, Frankfurt, Deutschland) zur Verhinderung der Rezirkularisierung dephosphoryliert. Der entstehende Vektor trägt die Bezeichnung pGU.US-BAR.

In dem Vektor pNE3 (Stougaard J (1993) Plant J 3:755-761) wurdezunächst die XbaI Schnittstelle durch eine "Klenow-filling-in" Reaktion entfernt. Aus dem resultierenden Vektor pNE3-XBA wurde mittels PCR unter Verwendung der Oligonukleotidprimer ONP9 (SEQ ID NO: 16) und ONP10 (SEQ ID NO: 17) das offene Leseraster (ORF) des negativen Selektionsmarkergens Cytosindeaminase (*cod*A) unter der Kontrolle des Cauliflower Mosaic Virus (CaMV) 35S Promoters und des Nopalinsythase (nos) Terminator ampifiziert. Durch die verwendeten Oligonukleotidprimer OPN9 und OPN10 wurde an beiden Enden des Amplifikats je eine I-*Sce*I Schnittstelle (in Fettdruck in den unten angegebenen Sequenzen hervorgehoben) und eine NotI bzw. XbaI Schnittstelle angefügt.
OPN9 (SEQ ID NO: 19):
OPN10 (SEQ ID NO: 20):

Für die PCR-Reaktion wurden 2 ocl (entsprechend ungefähr 100 ng) einer Plasmidpräparation von pNE3-XBA eingesetzt. In einem Gesamtvolumen von 50 ∝l wurden gemäß den Angaben des Herstellers (Life Technologies) zusammengegeben:
5 ∝l 10X PCR Buffer [200 mM Tris-HCl (pH 8, 4), 500 mM KCl]
1,5 ocl 50 mM MgCl₂
1 ∝l 10 mM dNTP Mix (jeweils 10 mM dATP, dCTP, dGTP und dTTP)
1 ocl Primer OPN1 (10 ∝M)
1 ocl Primer OPN2 (10 ocM)
0,4 ∝l Taq DNA Polymerase (5 U/∝l)
2 ∝l Plasmidpräparation von pNE3-XBA
38,1 ∝l autoklaviertes, destilliertes Wasser

Das Reaktionsgemisch wurde mit ca. 50 ∝l Silikonöl überschichtet und nachfolgendem Temperaturprogramm ausgesetzt (Thermocycler: MWG Biotech Primus HT; MWG Biotech, Deutschland):

Das PCR-Produkt wurde mit XbaI und NotI verdaut. Der Vektor pGU-US-BAR wurde ebenfalls mit XbaI und NotI verdaut (was zur Deletion des Hygomycin Markergen führte), das Vektorfragment über Agarosegelelektrophorese und unter Verwendung des QIAquickR Gel Extraction Kits (Qiagen, Hilden, Deutschland) aufgereinigt. Die Ligation von verdautem PCR-Fragment und Vektor führte zu dem binären Vektor pGU.C.USB (siehe Fig. 7a). Der Vektor enthält auf einer T-DNA zwischen zwei I-SceI Schnittstellen ein Markeregen (die Cytosindeminase (codA)). Die I-SceI Schnittstellen werden nach außen hin von homologen Sequenzbereichen von 557 bp des ß-Glucuronidasegens (GUS) flankiert. Das GUS-Gen dient als Marker der homologen Restauration (Swoboda P et al. (1994) EMBO J 13:481 - 489). Wird das Gen durch homologe Rekombination restauriert, kann die Expression histochemisch nachgewiesen werden. Die Elimination des Markergens führt zu 5-FC (Fluorocytosin) resistenten Tabakzellen die dann zu Kalli regeneriert werden können (Salomon S und Puchta H (1998) EMBO J 17:6086-6095).

### Beispiel 3: Pflanzentransformation mit pGU.I.USB

Nicotiana tabacum L. cv. Petite Havana Line SR1 Sämlinge wurden mit dem Agrobacterium Stamm C58 transformiert, der den binären Vector pGU.C.USB enthielt.

Dazu wurden, wie bei Puchta H. (1999) Methods Mol Biol 113:
447-451, beschrieben, Samen unter sterilen Bedingungen auf angefeuchteten Filterpapier ausgebracht und die Sämlinge nach 2 Wochen geerntet (25°C, 16 Stunden Licht/8 Stunden Dunkel Rhythmus).

Zur Inokulation wurde der Agrobakterienstamm, der das binäre Plasmid zur Transformation enthielt, zuerst über Nacht in einer Schüttelkultur bei 28°C in YEB Medium angezogen. Die Agrobakteriensuspension wurde dann für 10 Minuten bei 15.000 g abzentrifugiert und die Zellen in 10 mM MgSO₄ aufgenommen, so dass die endgültige optische Dichte der Suspension einem Wert von ungefähr 0,5 entsprach. In einem Reaktionsgefäß wurden dann unter sterilen Bedingungen die Sämlinge in die Bakteriensuspension geben und in einem sterilen Exsikkator ein Vakuum von 0, 15 at angelegt. Nach 10 Minuten wurden die Sämlinge dann auf MS Platten ausgebracht, die BAP (6-Benzylaminopurin 5 ocg/ml) und NAA (1-Naphthalenessigsäure 0,5 ∝g/ml) enthielten, und für 3 Tage in einer Wachstumskammer (25°C, 16 Stunden Licht/8 Stunden Dunkel Rhythmus) belassen. Die Sämlinge wurden danach auf MS Medium gebracht, das neben NAA und BAP noch Phosphinotricin (100 ∝g/ml), Vancomycin (1 ocg/ml) und Cefotaxin (0, 5 ∝g/ml) enthielt. Alle 10 Tage wurden die Sämlinge auf frisch-bereite Platten übertragen. Aus den entstehenden Kalli bildeten sich mit der Zeit Sprösslinge. Diese Sprösslinge wurden - sobald sie eine gewisse Größe erreicht hatten (1 bis 2 cm) - vom Kallusmaterial abgeschnitten und in Magentaboxen eingesetzt, die MS Medium mit Phosphinotricin, Vancomycin und Cefotaxin enthielten (Konzentrationen wie oben) . Die Sprösslinge bildeten nach kurzer Zeit Wurzeln und wurden nach 2 bis 4 Wochen in Erde umgesetzt. Im Gewächshaus wurden die Pflanzen zum Blühen gebracht, geselbstet und gewartet bis die entstandenen Samen in den Kapseln reiften. Danach wurden die Samen für die Segregationsanalysen auf MS Medium, das 300 ∝g Phosphinotricin (zur positiven Selektion) bzw. 500 ∝g 5-FC (Fluorocytosin; zur negativen Selektion) per ml enthielt, ausgebracht. Durch Ermittlung des Verhältnis der resistenten zu sensitiven Sämlingen (3:1 bei positiver Selektion bzw. 1:3 bei negativer Selektion) konnte gezeigt werden, dass bei den drei ausgewählten Linien die Rekombinationskonstrukte an einem Locus insertiert waren.

### Beispiel 5: Induktion der Gendeletion durch Einführung des DSBI-Enzyms I-SceI

In den Experimenten wurden F1 Sämlinge der transgenen Linien GU.C.USB 1, 3 and 7, die je eine Kopie der im Fig. 2 dargestellten T-DNA GU.C.USB enthielten, mit einem I-SceI transient exprimierenden Agrobakterienstamm, der das Plasmid pCISceI enthielt (Puchta H et al. (1996) Proc Natl Acad Sci USA 93, 5055-5060), auf die oben beschriebene Art (siehe auch Puchta, 1999b) inokuliert. Die Sämlinge wurden nach 3 Tagen auf MS Medium mit BAP und NAA (Konzentrationen wie oben) Medium auf das gleichen Medium zusätzlich in Gegenwart von 100 ∝g 5-FC and 100 ∝g Phosphinotricin per ml inkubiert, um Pflanzenzellen zu detektieren, bei denen das zu eliminierende Markergen (in diesem Falle das codA Gen) deletiert wurde. Die auf dem Medium wachsenden Kalli wurden nach 6 Wochen in zwei Teile geteilt, ein Teil wurde zur Regeneration von Sprossachsen verwendet der andere wurde zur Isolierung von DNA und zum ß-Glucuronidase Assay verwendet. Die erhaltenen 5-FC resistenten transgenen Kalli wurden auf homologe Rekombinationsereignisse hin mittels histochemischer Färbung untersucht. Dabei deutet eine Blaufärbung eine Restauration des Kallus an (s. Fig. 11).

Die histochemische Färbung der Kalli wurde wie bei Swoboda et al., 1994 beschrieben durchgeführt. Dazu wurden die Kalli in Färbelösung (0.3 mg X-Gluc [Duchefa, Harlem, Nl] pro ml 100 mM Natriumphosphatpuffer pH 7,0; 0,1 % triton; 0,05 % NaN₃) gebracht. Es wurde im Exsikkator für 15 Minuten Vakuum angelegt und anschließend wurden die Kalli in der Lösung für 48 Stunden bei 37°C inkubiert. Nach Abgießen der Färbelösung wurde durch mehrmaliges Schütteln in 80% Ethanol das verbleibende Chlorophyll aus dem Pflanzenmaterial entfernt. Die erhaltene Blaufärbung zeigte die Aktivität der ß-Glucuronidase an.

Bei ungefähr einem Viertel der Fälle wurde das Markergen durch homologe Rekombination erfolgreich eliminiert (Fig. 11, Tabelle 2).

**Tabelle 2. Zahl der 5-FC resistenten Tabak Kalli nach transienter DSB Induktion**

| Transgene Line | Sämlinge | resist. Kalli | GUS positiv | GUS positiv (% von resist. Kalli) |
|---|---|---|---|---|
| GU.C.USB 1 | 290 | 56 | 22 | 39 |
| GU.C.USB 3 | 490 | 90 | 24 | 27 |
| GU.C.USB 7 | 370 | 59 | 11 | 19 |

Molekulare Analysen bestätigten den Sachverhalt: Da die Linie GU.C.USB 1 eine einzelne Kopie des Transgens enthielt, wurden die Kalli direkt mittels PCR auf Rekombinationsereignisse hin untersucht.

Eine statistisch ausgewählter Teil der Kalli wurde dann mittels PCR auf der molekularen Ebene untersucht. Durch die molekulare Analyse mit dem Primerpaaren
OPN11 (SEQ ID NO: 21)
   5'- CGG AAG CTA CGT CAC CAA TCC CAA TTC GAT CTA C - 3' und
OPN12 (SEQ ID NO: 22)
   5'- CGG AAG CTT CCA CTT GCA AAG TCC CGC TAG TGC C - 3'
konnten die neugebildeten Verknüpfungsstellen aus dem Tabakgenom isoliert werden (Fig. 12; Tabelle 3).

**Tabelle 3. Molekulare Analyse von Rekombinationsereignissen mittels PCR**

| Transgene Linie | Kalli | PCR Fragment(e) | | |
|---|---|---|---|---|
| | | 0,7 kb | 1,4 kb | Keines/anders |
| GU.C.USB 1 | 30 | 10 | 12 | 7 |

Es wurden drei 0,7 kb PCR Fragmente ausgewählt und sequenziert. Die Sequenzierung ergab in allen drei Fällen die funktionelle Sequenz des ß-Glucuronidasegens, d.h. die Restaurierung des Gens ist tatsächlich durch homologe Rekombination fehlerfrei erfolgt.

Bei der Sequenzierung von fünf 1, 4 kb PCR Banden ergab sich, dass diese Banden nach Ausschneiden des codA Genes durch Reparatur der beiden I-SceI Schnittstellen entstanden sind (durch "non-homologous end-joining" NHEJ), ohne dass homologe Rekombination erfolgte. Dabei kam es meist zu kleineren Deletionen an der I-SceI Schnittstelle.

Per Southern Blot wurde gezeigt, dass es bei den Rekombinanten mit den 0, 7 bzw. 1, 9 kb Banden - wie erwartet zur vollständigen Eliminierung der zwischen den I-SceI Schnittstellen liegenden Sequenz kam. Es konnte keinerlei codA spezifische DNAmehr im Genom der regenerierten Pflanzen nachgewiesen werden (Fig. 13 B und D Spuren 2 und 3).

Die DNA wurde mit Hilfe des DNeasy Plant Mini Kit (Quiagen, Hilden) isoliert. Zur Detektion der Rekombinationsprodukte wurde genomische DNA mittels PCR unter Verwendung der Oligonukleotide OPN13 und OPN14 analysiert.
OPN13 (SEQ ID NO: 23):
   5'- CGG AAG CTT CGT CAC CAA TCC CAA TTC GAT CTA C - 3'
OPN14 (SEQ ID NO: 24):
   5'- GGG AAG CTT CCA CTT GCA AAG TCC CGC TAG TGC C - 3'
   5 ocl 10X PCR Buffer [200 mM Tris-HCl (pH 8,4), 500 mM KCl)
   1,5 ocl 50 mM MgCl₂
   1 ∝l 10 mM dNTP Mix (jeweils 10 mM dATP, dCTP, dGTP und dTTP)
   1 ∝l Primer OPN1 (10 ∝M)
   1 ∝l Primer OPN2 (10 ∝M)
   0,4 4 ∝l Taq DNA polymerase (5 U/ocl)
   2 ∝l DNA-präparation
   38,1 ∝l autoklaviertes, destilliertes Wasser

Das Reaktionsgemisch wird mit ca. 50 ∝l Silikonöl überschichtet und nachfolgendem Temperaturprogramm ausgesetzt (Thermocycler: MWG Biotech Primus HT; MWG Biotech, Deutschland):

| | |
|---|---|
| 1 | Zyklus mit 180 sec bei 95_C |
| 30 | Zyklen mit 92_C für 60 sec, 54_C für 60 sec und 72_C für 3 min. |
| 1 | Zyklus mit 72_C für 5 min. |

Die Sequenzierung der PCR Produkte wurde mit dem "ABI Prism Dye Terminator Cycle Sequencing Reaction Kit" (PE Applied Biosystems, Weiterstadt) durchgeführt.

Zum Southern Blotting wurde die DNA mit HindIII oder Acc65I geschnitten und einer Elektrophorese in einem 0.8 % Agarosegel unterworfen. Die im Gel befindliche DNA wurde dann mittels Kapillarblottings, wie in der Anleitung des Herstellers beschrieben, auf die Hybridisierungsmembran 'Hybond N' (Amersham, Little Chalfont, UK) übertragen. Für die molekulare Hybridisierung wurden codA bzw. GUS spezifische Genfragmente aus den Ausgangsplasmiden isoliert (XbaI/XhoI Fragment als PNE3; Stpugaard, 1993 und KpnI/SacI Fragment aus pGUS23, Puchta and Hohn, 1991, isoliert mit dem QIAquick Gel Extraction Kits [Qiagen, Hilden]) und mit Hilfe eines "Random Priming Labeling Kit" (Megaprime DNA labeling system RPN1607, Amersham, Little Chalfont, UK) und [α-32P] dATP (Amersham, Little Chalfont, UK) markiert. Die Hybridisierungen wurden bei 65° C durchgeführt.

Da bei den Linien GU.C.USB 3 und GU.C.USB 7 jeweils 2 genetisch gelinkte Transgenkopien integriert waren, wurden bei diesen Linien eine repräsentative Anzahl von Pflanzen aus Kallus regeneriert, DNA gewonnen und dann per Southern Blot untersucht (Tabelle 4).

Bei Acc65I deutet die Anwesenheit einer GUS-spezifischen Bande von 3,7 kb auf eine homologe Rekombination, die einer 4,4 kb Bande auf ein NHEJ-Ereignis ("non-homologous end-joining"; NHEJ) hin (Fig. 7b und c; Fig. 13 C).

**Tabelle 4. Molekulare-Analyse von Rekombinationsereignissen mittels Southern Blots**

| Transgene Linie | Kalli | Acc65I-Fragment (kb) | | |
|---|---|---|---|---|
| | | 3,7 | 4,4 | Deletion |
| GU.C.USB 3 | 39 | 6 | 18 | 15 |
| GU.C.USB 7 | 14 | 2 | 5 | 7 |

Interessanterweise wurde in allen Fällen die gleiche Art von Verknüpfung bei beiden Transgenkopien gefunden. Es traten also - mit anderen Worten - entweder nur homologe Rekombinationen oder nur NHEJ-Ereignisse auf. In keinem Fall lagen beide Möglichkeiten parallel vor, d.h. beispielsweise eine homologe Rekombination an dem einem Transgen und ein NHEJ-Ereignis an dem anderen.

Es wurden bei beiden Linien auch PCR Analysen durchgeführt und jeweils drei 0,7 kb große PCR Fragmente ausgewählt und sequenziert. Die Sequenzierung ergab in allen drei Fällen die funktionelle Sequenz des ß-Glucuronidasegens, d.h. die Restaurierung des Gens ist tatsächlich durch homologe Rekombination erfolgt.

Bei der Sequenzierung von insgesamt neun 1, 4 kb langer PCR Banden der zwei Linien ergab sich weiterhin, dass diese Banden tatsächlich nach Ausschneiden des codA Genes durch Reparatur der beiden 1-SceI Schnittstellen entstanden sind (durch "non-homologous end-joining" NHEJ). Dabei kam es wiederum meist zu kleineren Deletionen an der I-SceI Schnittstelle.

Per Southern Blot wurde gezeigt, dass es bei dem Rekombinanten wie erwartet zur vollständigen Eliminierung der zwischen den I-SceI Schnittstellen liegenden Sequenz kam. Es konnte keinerlei codA spezifische DNA mehr im Genom der regenerierten Pflanzen nachgewiesen werden (Fig. 13 B und D Spuren 5, 6 und 8, 9).

### Beispiel 5:

Es wurden verschiedene transgene Tabakpflanzenlinien hergestellt, die zwischen den Hälften des ß-Glucuronidasegens (Anordnung wie oben beschrieben) neben einer I-SceI Schnittstelle mittels Klonierung synthetischer Oligonukleotide auch Schnittstellen für die oben aufgeführten Restriktionsenzyme aufwiesen (Fig. 10). Sämlinge dieser Tabaklinie wurden dann jeweils im direkten Vergleich mit Agrobakterien inokkuliert, die entweder I-SceI oder das entsprechende Enzym in Pflanzenzellen exprimieren können. Die entstehenden Kalli wurden dann nach 2 Wochen histochemisch gefärbt. Die Ergebnisse sind in (Tabelle 4 dargestellt.

Das Plasmid pGU.C.US.B wurde mit I-SceI geschnitten, so dass das codA Gen aus dem Plasmid herausgeschnitten wurde. Die verdaute DNA wurde mittels Agarosegelelektrophorese aufgetrennt, die größere Bande wurde ausgeschnitten und mittels des QIAquick Gel Extraction Kits (Qiagen, Hilden) aufgereinigt und anschließend ligiert und in E. coli transformiert. Das erhalten Plasmid wurde dann mit XbaI geschnitten.

Die komplementären einzelsträngigen Oligonukleotide OPN25 und OPN26 wurden durch kurzes Erhitzen auf 92°C und abschließendes Abkühlen in eine doppelsträngige Form gebracht und daran anschließend mit dem XbaI geschnittenen Plasmid ligiert. Das erhaltene SI-Konstrukt (pSI) enthält die Schnittstellen für I-SceI, I-CpaI, I-CpaII und I-CreI ((s. Fig. 10 (A)).
OPN15 (SEQ ID NO: 25):
OPN16 (SEQ ID NO: 26):

Die komplementären einzelsträngigen Oligonukleotide OPN27 und OPN28 wurden durch kurzes Erhitzen auf 92°C und abschließendes Abkühlen in eine doppelsträngige Form gebracht und daran anschließen mit dem XbaI geschnittenen Plasmid ligiert. Das erhaltene SD-Konstrukt (pSD) enthält die Schnittstellen für I-SceI und I-ChuI (s. Fig. 10 (B)).
OPN17 (SEQ ID NO: 27):
OPN118 (SEQ ID NO: 28):

Es wurden wie bereits weiter oben beschrieben mittels Agrobacterium Transformation transgene Tabakpflanzen mit beiden Konstrukte hergestellt. Dabei wurden für die weiteren Versuche Linien verwendet, die an nur einem Locus transgene Sequenzen enthielten. Diese Linien wurden durch die 3:1 Segregation in Phosphinotricin- resistente und nicht resistente Pflanzen ermittelt. Die geselbsteten Sämlinge würden dann mit Agrobakteriumstämmen inokuliert, die eines der vier Konstrukte zur Expression der Restriktionsendonukleasen bzw. als Vektorkontrolle das Plasmid BinAR oder als positiv Kontrolle eine 1:1 Mischung aus BinAR und CISce-I enthielten. Die Inokulationen wurden wie oben beschrieben durchgeführt (Puchta H (1999) Methods Mol. Biol. 113:447-451) und zur Selektion wurden die Sämlinge auf MS Medium mit 100 ∝g Kanamycin pro ml, das auch BAP und AAA, Vancomycin und Cefotaxin (Konzentrationen wie oben) enthielt über mehrere Wochen kultiviert. Die entstehenden Kalli wurden dann, wie oben beschrieben einer histochemischen ß-Glucuronidasefärbung unterworfen.

Mit allen vier getesteten Restriktionsenzymen konnte eine Induktion der homologen Rekombination in der gleichen Größenordnung hervorgerufen werden wie mit I-SceI (das hier in einer Coinokulation mit dem Selektionsvektor pBinAR [AR] eingesetzt wurde) (Tabelle 5). Damit ist gezeigt, dass bei Verwendung von beliebigen Restriktionsendonukleasen effizient homologer Rekombination induziert werden kann.

**Tabelle 5. Induktion der homologen Rekombination in Pflanzen mittels verschiedener Endonukleasen I-CreI, I-CpaI, I-CpaII und I-ChuI. [Sektoren/Kalli] meint die Anzahl von blaugefärbten Arealen in den resistenten Kalli.**

| Transgene Linie | Enzym | Sektoren/Kalli | Verhältnis |
|---|---|---|---|
| SI5 | I-SceI/AR | 42/31 | 1.35 |
| | I-CreI | 77/50 | 0.54 |
| | I-CpaII | 51/50 | 1.02 |
| SI2 | I-SceI/AR | 8/9 | 0.89 |
| | I-CreI | 40/18 | 2.22 |
| | I-CpaII | 9/20 | 0.45 |
| SI2 | I-CpaI | 144/106 | 1.36 |
| SD2 | I-ChuI | 166/100 | 1.66 |

## Patentansprüche

1. Rekombinationssystem, **dadurch gekennzeichnet, dass**
I) ein transgenes Rekombinationskonstrukt insertiert in die chromosomale DNA einer pflanzlichen Zelle oder einespflanzlichen Organismus, daseine Sequenz enthält, aufgebaut in 5'/3'-Richtung aus
a1) einer ersten Homologiesequenz A und
b1) mindestens einer Erkennungssequenz zur gezielten Induktion von DNA-Doppelstrangbrüchen und
c) einer Expressionskassette als weitere Nukleinsäuresequenz und
a2) einer zweiten Homologiesequenz B, wobei die Homologiesequenzen A und B eine ausreichende Länge und ausreichende Homologie aufweisen, um eine homologe Rekombination zwischen den Homologiesequenzen A und B zu gewährleisten,
und wobei besagte Expressionskassette und besagte Erkennungssequenz(en) zur gezielten Induktion von DNA-Doppelstrangbrüchen infolge einer homologen Rekombination zwischen A und B aus der chromosomalen DNA entfernt werden,
und
II) ein Enzym geeignet zur Induktion von DNA-Doppelstrangbrüchen an der (den) Erkennungssequenz(en) (b1) zur gezielten Induktion von DNA-Doppelstrangbrüchen oder eine Nukleinsäuresequenz kodierend für ein Enzym geeignet zur Induktion von DNA-Doppelstrangbrüchen an der (den) Erkennungssequenz(en) (b1)
in einer pflanzlichen Zelle oder einem pflanzlichen Organismus zusammen vorliegen und das zur Induktion von DNA-Doppelstrangbrüchen geeignete Enzym ausgewählt ist aus der Gruppe bestehend aus Homing-Endonukleasen, Gruppe II Intron Endonukleasen, Rekombinasen, Transposasen, Chimäre Nukleasen.

2. Rekombinationssystem gemäß Anspruch 1, **dadurch gekennzeichnet, dass** das Rekombinationskonstrukt wie folgt aufgebaut ist
a1) einer ersten Homologiesequenz A und
b1) einer ersten Erkennungssequenz zur gezielten Induktion von DNA-Doppelstrangbrüchen und
c) einer weiteren Nukleinsäuresequenz umfassend mindestens eine Expressionskassette und
b2) einer zweiten Erkennungssequenz zur gezielten Induktion von DNA-Doppelstrangbrüchen und
a2) einer zweiten Homologiesequenz B, wobei die Homologiesequenzen A und B eine ausreichende Länge und ausreichende Homologie aufweisen, um eine homologe Rekombination zu gewährleisten.

3. Rekombinationssystem nach einem der Ansprüche 1 oder 2, **dadurch gekennzeichnet, dass** das Rekombinationskonstrukt oder die weitere Nukleinsäuresequenz mindestens eines der Elemente beinhaltet ausgewählt aus der Gruppe bestehend aus
i) Positiven Selektiönsmarkern
ii) Negativen Selektionsmarkern
iii) Reportergenen
iv) Replikationsursprüngen
v) Multiple Klonierungsregionen
vi) "Border"-Sequenzen für Agrobakterium-Transfektion
vii) Sequenzen, die eine homologe Rekombination bzw. Insertion in das Genom eines Wirtsorganismus ermöglichen
viii) Expressionskassette für ein Enzym geeignet zur Induktion von DNA-Doppelstrangbrüchen an der Erkennungsseqüenz zur gezielten Induktion von DNA-Doppelstrangbrüchen

4. Rekombinationssystem nach einem der Ansprüche 1 bis 3, **dadurch gekennzeichnet, dass** das Enzym geeignet zur Induktion von DNA-Doppelstrangbrüchen an der Erkennungssequenz zur gezielten Induktion von DNA-Doppelstrangbrüchen ausgewählt ist aus der Gruppe der Homing-Endonukleasen bestehend aus F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, 1-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI und PI-TliII.

5. Rekombinationssystem nach einem der Ansprüche 1 bis 4, **dadurch gekennzeichnet, dass** das Enzym geeignet zur Induktion von DNA-Doppelstrangbrüchen an der Erkennungssequenz zur gezielten Induktion von DNA-Doppelstrangbrüchen ausgewählt ist aus der Gruppe der Homing-Endonukleasen bestehend aus den Enzymen gemäß SEQ ID NO: 2, 4, 6, 8 und 10.

6. Rekombinationssystem nach einem der Ansprüche 1 bis 5, **dadurch gekennzeichnet, dass** das Enzym geeignet zur Induktion von DNA-Doppelstrangbrüchen an der Erkennungssequenz zur gezielten Induktion von DNA-Doppelstrangbrüchen unter Verwendung einer Expressionskassette realisiert wird, die eine für das besagte Enzym kodierende Nukleinsäuresequenz beinhaltet.

7. Rekombinationssystem nach einem der Ansprüche 1 bis 6, **dadurch gekennzeichnet, dass** das Enzym geeignet zur Induktion von DNA-Doppelstrangbrüchen an der Erkennungssequenz zur gezielten Induktion von DNA-Doppelstrangbrüchen unter Verwendung einer Expressionskassette realisiert wird, die eine für das besagte Enzym kodierende Nukleinsäuresequenz gemäß SEQ ID NO: 1, 3, 5, 7 oder 9 beinhaltet.

8. Verfahren zum Entfernen einer DNA-Sequenz aus der chromosomalen DNA einer pflanzlichen Zelle oder pflanzlichen Organismus, **dadurch gekennzeichnet, dass**
I) ein transgenes Rekombinationskonstrukt insertiert in die chromosomale DNA eines eukaryotischen Organismus, das eine Sequenz enthält aufgebaut in 5'/3'-Richtung aus
a1) einer ersten Homologiesequenz A und
b1) mindestens einer Erkennungssequenz zur gezielten Induktion von DNA-Doppelstrangbrüchen und
c) einer Expressionskassette als weitere Nukleinsäuresequenz und
a2) einer zweiten Homologiesequenz B, wobei die Homologiesequenzen A und B eine ausreichende Länge und ausreichende Homologie aufweisen, um eine homologe Rekombination zu gewährleisten,
und
II) ein Enzym geeignet zur Induktion von DNA-Doppelstrangbrüchen an der (den) Erkennungssequenz (en) (b1) zur gezielten Induktion von DNA-Doppelstrangbrüchen
in einer pflanzlichen Zelle oder einem pflanzlichen Organismus zusammengebracht werden, und die Induktion von DNA-Doppelstrangbrüchen an der (den) Erkennungssequenz(en) zur gezielten Induktion von DNA-Doppelstrangbrüchen sowie die homologe Rekombination zwischen den Homologiesequenzen A und B erfolgt, und wobei besagte Expressionskassette und besagte Erkennungssequenz(en) zur gezielten Induktion von DNA-Doppelstrangbrüchen infolge einer homologen Rekombination zwischen A und B aus der chromosomalen DNA entfernt werden und das zur Induktion von DNA-Doppelstrangbrüchen geeignete Enzym ausgewählt ist aus der Gruppe bestehend aus Homing-Endonukleasen, Gruppe II Intron Endonukleasen, Rekombinasen, Transposasen, Chimäre Nukleasen.

9. Verfahren gemäß Anspruch 8, **dadurch gekennzeichnet, dass** das Rekombinationskonstrukt wie folgt aufgebaut ist
a1) einer ersten Homologiesequenz A und
b1) einer ersten Erkennungssequenz zur gezielten Induktion von DNA-Doppelstrangbrüchen und
c) einer weiteren Nukleinsäuresequenz umfassend mindestens eine Expressionskassette und
b2) einer zweiten Erkennungssequenz zur gezielten Induktion von DNA-Doppelstrangbrüchen und
a2) einer zweiten Homolögiesequenz B, wobei die Homologiesequenzen A und B eine ausreichende Länge und ausreichende Homologie aufweisen, um eine homologe Rekombination zu gewährleisten.

10. Verfahren nach einem der Ansprüche 8 oder 9, **dadurch gekennzeichnet, dass** das Rekombinationskonstrukt oder die weitere Nukleinsäuresequenz mindestens eines der Elemente beinhaltet ausgewählt aus der Gruppe bestehend aus
i) Positiven Selektionsmarkern
ii) Negativen Selektionsmarkern
iii) Reportergenen
iv) Replikationsursprüngen
v) Multiple Klonierungsregionen
vi) "Border"-Sequenzen für Agrobakterium-Transfektion
vii) Sequenzen, die eine homologe Rekombination bzw. Insertion in das Genom eines Wirtsorganismus ermöglichen
viii) Expressionskassette für ein Enzym geeignet zur Induktion von DNA-Doppelstrangbrüchen an der Erkennungssequenz zur gezielten Induktion von DNA-Doppelstrangbrüchen

11. Verfahren nach einem der Ansprüche 8 bis 10, **dadurch gekennzeichnet, dass** das Enzym geeignet zur Induktion von DNA-Doppelstrangbrüchen an der Erkennungssequenz zur gezielten Induktion von DNA-Doppelstrangbrüchen ausgewählt ist aus der Gruppe der Homing-Endonukleasen bestehend aus F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, 1-PorIIP, I-PpbIP, I-PpoI, I-SPBetaIP, I-ScaI, I-SceI, I-SceII, I-SceIII , I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI und PI-TliII.

12. Verfahren nach einem der Ansprüche 8 bis 11, **dadurch gekennzeichnet, dass** das Enzym geeignet zur Induktion von DNA-Doppelstrangbrüchen an der Erkennungssequenz zur gezielten Induktion von DNA-Doppelstrangbrüchen ausgewählt ist aus der Gruppe der Homing-Endonukleasen bestehend aus den Enzymen gemäß SEQ ID NO: 2, 4, 6, 8 und 10.

13. Verfahren nach einem der Ansprüche 8 bis 12, **dadurch gekennzeichnet, dass** das Enzym geeignet zur Induktion von DNA-Doppelstrangbrüchen an der Erkennungssequenz zur gezielten Induktion von DNA-Doppelstrangbrüchen unter Verwendung einer Expressionskassette realisiert wird, die eine für das besagte Enzym kodierende Nukleinsäuresequenz beinhaltet.

14. Verfahren nach einem der Ansprüche 8 bis 13, **dadurch gekennzeichnet, dass** das Enzym geeignet zur Induktion von DNA-Doppelstrangbrüchen an der Erkennungssequenz zur gezielten Induktion von DNA-Doppelstrangbrüchen unter Verwendung einer Expressionskassette realisiert wird, die eine für das besagte Enzym kodierende Nukleinsäuresequenz gemäß SEQ ID NO: 1, 3, 5, 7 oder 9 beinhaltet.

15. Pflanzlicher Organismus, enthaltend ein Rekominationssystem gemäß einem der Ansprüche 1 bis 8.

16. Pflanzlicher Organismus, nach Anspruch 15, ausgewählt aus der Gruppe bestehend aus Arabidopsis thaliana, Tabak, Weizen, Roggen, Gerste, Hafer, Raps, Mais, Kartoffel, Zuckerrübe, Soja, Sonnenblume, Kürbis oder Erdnuss.

17. Zellkulturen, Organe, Gewebe, Teile oder transgenes Vermehrungsgut enthaltend ein Rekombinationssystem gemäß einem der Ansprüche 1 bis 8.

18. Verwendung eines pflanzlichen Organismus enthaltend ein Rekombinationssystem gemäß einem der Ansprüche 1 bis 8 oder von diesem abgeleitete Zellkulturen, Organe, Gewebe, Teile oder transgenes Vermehrungsgut nach Anspruch 17 als Nahrungs-, Futtermittel oder Saatgut oder zur Herstellung von Pharmazeutika oder Feinchemikalien.

## Claims

1. A recombination system which comprises, in a plant cell or a plant organism,
I) a transgenic recombination construct inserted into the chromosomal DNA of a plant cell or of a plant organism comprising a sequence composed, in the 5'/3'-direction, of
a1) a first homology sequence A and
b1) at least one recognition sequence for the targeted induction of DNA double-strand breaks and
c) an expression cassette as further nucleic acid sequence and
a2) a second homology sequence B, the homology sequences A and B having a sufficient length and sufficient homology in order to ensure homologous recombination between the homology sequences A and B,
and said expression cassette and said recognistion sequences(s) for the targeted induction of DNA double-strand breaks being removed from the chromosomal DNA as a result of homologous recombination between A and B,
together with
II) an enzyme suitable for inducing DNA double-strand breaks at the recognition sequences (b1) for the targeted induction of DNA double-strand breaks or a nucleic acid sequence encoding an enzyme suitable for inducing DNA double-strand breaks at the recognition sequences (b1),
and the enzyme suitable for inducing DNA double-strand breaks is selected from the group consisting of homing endonucleases, group II intron endonucleases, recombinases, transposases and chimeric nucleases.

2. A recombination system according to claim 1, wherein the recombination construct is constructed as follows:
a1) a first homology sequence A and
b1) a first recognition sequence for the targeted induction of DNA double-strand breaks and
c) a further nucleic acid sequence comprising at least one expression cassette and
b2) a second recognition sequence for the targeted induction of DNA double-strand breaks and
a2) a second homology sequence B, the homology sequences A and B having a sufficient length and sufficient homology in order to ensure homologous recombination.

3. A recombination system according to either of claims 1 and 2, wherein the recombination construct or the further nucleic acid sequence encompasses at least one of the elements selected from the group consisting of
i) positive selection markers
ii) negative selection markers
iii) reporter genes
iv) replication origins
v) multiple cloning regions
vi) border sequences for Agrobacterium transfection
vii) sequences which enable homologous recombination or insertion into the genome of a host organism
viii) expression cassette for an enzyme suitable for inducing DNA double-strand breaks at the recognition sequence for the targeted induction of DNA double-strand breaks

4. A recombination system according to any of claims 1 to 3, wherein the enzyme suitable for inducing DNA double-strand breaks at the recognition sequence for the targeted induction of DNA double-strand breaks is selected from the group of the homing endonucleases consisting of F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII , I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPAIP, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI and PI-TliII.

5. A recombination system according to any of claims 1 to 4, wherein the enzyme suitable for inducing DNA double-strand breaks at the recognition sequence for the targeted induction of DNA double-strand breaks is selected from the group of the homing endonucleases consisting of the enzymes as shown in SEQ ID NO: 2, 4, 6, 8 and 10.

6. A recombination system according to any of claims 1 to 5, wherein the enzyme suitable for inducing DNA double-strand breaks at the recognition sequence for the targeted induction of DNA double-strand breaks is realized using an expression cassette encompassing a nucleic acid sequence encoding said enzyme.

7. A recombination system according to any of claims 1 to 6, wherein the enzyme suitable for inducing DNA double-strand breaks at the recognition sequence for the targeted induction of DNA double-strand breaks is realized using an expression cassette encompassing a nucleic acid sequence encoding said enzyme as shown in SEQ ID NO: 1, 3, 5, 7 or 9.

8. A method for removing a DNA sequence from the chromosomal DNA of a plant cell or plant organism, which comprises combining, in a plant cell or a plant organism,
I) a transgenic recombination construct inserted into the chromosomal DNA of a eukaryotic organism comprising a sequence composed, in the 5'/3'-direction, of
a1) a first homology sequence A and
b1) at least one recognition sequence for the targeted induction of DNA double-strand breaks and
c) an expression cassette as further nucleic acid sequence and
a2) a second homology sequence B, the homology sequences A and B having a sufficient length and sufficient homology in order to ensure homologous recombination,
together with
II) an enzyme suitable for inducing DNA double-strand breaks at the recognition sequences (b1) for the targeted induction of DNA double-strand breaks,
and the induction of DNA double-strand breaks at the recognition sequences for the targeted induction of DNA double-strand breaks and the homologous recombination taking place between the homology sequences A and B, and said expression cassette and said recognistion sequences(s) for the targeted induction of DNA double-strand breaks being removed from the chromosomal DNA as a result of homologous recombination between A and B,and the enzyme suitable for inducing DNA double-strand breaks is selected from the group consisting of homing endonucleases, group II intron endonucleases, recombinases, transposases and chimeric nucleases.

9. A method according to claim 8, wherein the recombination construct is constructed as follows:
a1) a first homology sequence A and
b1) a first recognition sequence for the targeted induction of DNA double-strand breaks and
c) a further nucleic acid sequence comprising at least one expression cassette and
b2) a second recognition sequence for the targeted induction of DNA double-strand breaks and
a2) a second homology sequence B, the homology sequences A and B having a sufficient length and sufficient homology in order to ensure homologous recombination.

10. A method according to either of claims 8 and 9, wherein the recombination construct or the further nucleic acid sequence encompasses at least one of the elements selected from the group consisting of
i) positive selection markers
ii) negative selection markers
iii) reporter genes
iv) replication origins
v) multiple cloning regions
vi) border sequences for Agrobacterium transfection
vii) sequences which enable homologous recombination or insertion into the genome of a host organism
viii) expression cassette for an enzyme suitable for inducing DNA double-strand breaks at the recognition sequence for the targeted induction of DNA double-strand breaks

11. A method according to any of claims 8 to 10, wherein the enzyme suitable for inducing DNA double-strand breaks at the recognition sequence for the targeted induction of DNA double-strand breaks is selected from the group of the homing endonucleases consisting of F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII , I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI and PI-TliII.

12. A method according to any of claims 8 to 11, wherein the enzyme suitable for inducing DNA double-strand breaks at the recognition sequence for the targeted induction of DNA double-strand breaks is selected from the group of the homing endonucleases consisting of the enzymes as shown in SEQ ID NO: 2, 4, 6, 8 and 10.

13. A method according to any of claims 8 to 12, wherein the enzyme suitable for inducing DNA double-strand breaks at the recognition sequence for the targeted induction of DNA double-strand breaks is realized using an expression cassette encompassing a nucleic acid sequence encoding said enzyme.

14. A method according to any of claims 8 to 13, wherein the enzyme suitable for inducing DNA double-strand breaks at the recognition sequence for the targeted induction of DNA double-strand breaks is realized using an expression cassette encompassing a nucleic acid sequence encoding said enzyme as shown in SEQ ID NO: 1, 3, 5, 7 or 9.

15. A plant organism comprising a recombination system according to any of claims 1 to 8.

16. A plant organism according to claim 15 selected from the group consisting of Arabidopsis thaliana, tobacco, wheat, rye, barley, oats, oilseed rape, maize, potato, sugar beet, soybean, sunflower, pumpkin/squash or peanut.

17. A cell culture, organ, tissue, part or transgenic propagation material comprising a recombination system according to any of claims 1 to 8.

18. The use of a plant organism comprising a recombination system according to any of claims 1 to 8 or of a cell culture, organ, tissue, part or transgenic propagation material comprising a recombination system according to any of claims 1 to 8 according to claim 17 derived therefrom as foodstuff, feedstuff or seed or for the production of pharmaceuticals or fine chemicals.

## Revendications

1. Système recombinant, **caractérisé en ce que**
I) une construction recombinante transgénique insérée dans l'ADN chromosomique d'une cellule végétale ou d'un organisme végétal, qui contient une séquence constituée par, dans le sens 5'/3',
a1) une première séquence d'homologie A et
b1) au moins une séquence de reconnaissance pour l'induction ciblée de ruptures de double brin d'ADN et
c) une cassette d'expression comme autre séquence d'acide nucléique et
a2) une deuxième séquence d'homologie B, les séquences d'homologie A et B présentant une longueur suffisante et une homologie suffisante pour assurer une recombinaison homologue entre les séquences d'homologie A et B,
et ladite cassette d'expression et ladite/lesdites séquence(s) de reconnaissance pour l'induction ciblée de ruptures de double brin d'ADN étant éliminées de l'ADN chromosomique suite à une recombinaison homologue entre A et B,
et
II) une enzyme appropriée pour l'induction de ruptures de double brin d'ADN au niveau de la/des séquence(s) (b1) de reconnaissance pour l'induction ciblée de ruptures de double brin d'ADN ou une séquence d'acide nucléique codant pour une enzyme appropriée pour l'induction de ruptures de double brin d'ADN au niveau de la/des séquence(s) de reconnaissance (b1)
se trouvent ensemble dans une cellule végétale ou dans un organisme végétal et l'enzyme appropriée pour l'induction de ruptures de double brin d'ADN étant choisie dans le groupe formé par les endonucléases de ciblage (homing), les endonucléases d'introns de groupe II, les recombinases, les transposases, les nucléases chimères.

2. Système recombinant selon la revendication 1, **caractérisé en ce que** la construction recombinante est constituée, comme suit, par
a1) une première séquence d'homologie A et
b1) une première séquence de reconnaissance pour l'induction ciblée de ruptures de double brin d'ADN et
c) une autre séquence d'acide nucléique comprenant au moins une cassette d'expression et
b2) une deuxième séquence de reconnaissance pour l'induction ciblée de ruptures de double brin d'ADN et
a2) une deuxième séquence d'homologie B, les séquences d'homologie A et B présentant une longueur suffisante et une homologie suffisante pour assurer une recombinaison homologue.

3. Système recombinant selon l'une quelconque des revendications 1 ou 2, **caractérisé en ce que** la construction recombinante ou l'autre séquence d'acide nucléique comprend au moins un des éléments choisis dans le groupe constitué par
i) les marqueurs de sélection positifs
ii) les marqueurs de sélection négatifs
iii) les gènes rapporteurs
iv) les origines de réplication
v) les régions de clonage multiple
vi) les séquences limites (Border) pour la transfection d'Agrobacterium
vii) les séquences qui permettent une recombinaison homologue ou, selon le cas, une insertion dans le génome d'un organisme hôte
viii) des cassettes d'expression pour une enzyme appropriée pour l'induction de ruptures de double brin d'ADN au niveau de la séquence de reconnaissance pour l'induction ciblée de ruptures de double brin d'ADN

4. Système recombinant selon l'une quelconque des revendications 1 à 3, **caractérisé en ce que** l'enzyme appropriée pour l'induction de ruptures de double brin d'ADN au niveau de la séquence de reconnaissance pour l'induction ciblée de ruptures de double brin d'ADN est choisie dans le groupe des endonucléase de ciblage (Homing) constitué par F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI et PI-TliII.

5. Système recombinant selon l'une quelconque des revendications 1 à 4, **caractérisé en ce que** l'enzyme appropriée pour l'induction de ruptures de double brin d'ADN au niveau de la séquence de reconnaissance pour l'induction ciblée de ruptures de double brin d'ADN est choisie dans le groupe des endonucléases de ciblage (Homing) constitué par les enzymes selon les séquences SEQ ID NO : 2, 4, 6, 8, et 10.

6. Système recombinant selon l'une quelconque des revendications 1 à 5, **caractérisé en ce que** l'enzyme appropriée pour l'induction de ruptures de double brin d'ADN au niveau de la séquence de reconnaissance pour l'induction ciblée de ruptures de double brin d'ADN est réalisée en utilisant une cassette d'expression qui comporte une séquence d'acide nucléique codant pour ladite enzyme.

7. Système recombinant selon l'une quelconque des revendications 1 à 6, **caractérisé en ce que** l'enzyme appropriée pour l'induction de ruptures de double brin d'ADN au niveau de la séquence de reconnaissance pour l'induction ciblée de ruptures de double brin d'ADN est réalisée en utilisant une cassette d'expression qui comporte une séquence d'acide nucléique codant pour ladite enzyme selon les séquences SEQ ID NO : 1, 3, 5, 7 ou 9.

8. Procédé pour éliminer une séquence d'ADN de l'ADN chromosomique d'une cellule végétale ou d'un organisme végétal, **caractérisé en ce que**
I) une construction recombinante transgénique insérée dans l'ADN chromosomique d'un organisme eucaryote qui contient une séquence, constituée par, dans le sens 5'/3',
a1) une première séquence d'homologie A et
b1) au moins une séquence de reconnaissance pour l'induction ciblée de ruptures de double brin d'ADN et
c) une cassette d'expression comme autre séquence d'acide nucléique et
a2) une deuxième séquence d'homologie B, les séquences d'homologie A et B présentant une longueur suffisante et une homologie suffisante pour assurer une recombinaison homologue,
et
II) une enzyme appropriée pour l'induction de ruptures de double brin d'ADN au niveau de la/des séquence(s) de reconnaissance (b1) pour l'induction ciblée de ruptures de double brin d'ADN
sont rassemblées dans une cellule végétale ou dans un organisme végétal et l'induction de ruptures de double brin d'ADN au niveau de la/des séquence(s) de reconnaissance pour l'induction ciblée de ruptures de double brin d'ADN ainsi que la recombinaison homologue entre les séquences d'homologie A et B s'effectuent et ladite cassette d'expression et ladite/lesdites séquence(s) de reconnaissance pour l'induction ciblée de ruptures de double brin d'ADN étant éliminées de l'ADN chromosomique suite à une recombinaison homologue entre A et B et l'enzyme appropriée pour l'induction de ruptures de double brin d'ADN étant choisie dans le groupe formé par les endonucléases de ciblage (homing), les endonucléases d'introns de groupe II, les recombinases, les transposases, les nucléases chimères.

9. Procédé selon la revendication 8, **caractérisé en ce que** la construction recombinante est constituée comme suit par
a1) une première séquence d'homologie A et
b1) une première séquence de reconnaissance pour l'induction ciblée de ruptures de double brin d'ADN et
c) une autre séquence d'acide nucléique comprenant au moins une cassette d'expression et
b2) une deuxième séquence de reconnaissance pour l'induction ciblée de ruptures de double brin d'ADN et
a2) une deuxième séquence d'homologie B, les séquences d'homologie A et B présentant une longueur suffisante et une homologie suffisante pour assurer une recombinaison homologue.

10. Procédé selon l'une quelconque des revendications 8 ou 9, **caractérisé en ce que** la construction recombinante ou l'autre séquence d'acide nucléique comprend au moins un des éléments choisis dans le groupe constitué par
i) les marqueurs de sélection positifs
ii) les marqueurs de sélection négatifs
iii) les gènes rapporteurs
iv) les origines de réplication
v) les régions de clonage multiple
vi) les séquences limites (Border) pour la transfection d'Agrobacterium
vii) les séquences qui permettent une recombinaison homologue ou, selon le cas, une insertion dans le génome d'un organisme hôte
viii) des cassettes d'expression pour une enzyme appropriée pour l'induction de ruptures de double brin d'ADN au niveau de la séquence de reconnaissance pour l'induction ciblée de ruptures de double brin d'ADN.

11. Procédé selon l'une quelconque des revendications 8 à 10, **caractérisé en ce que** l'enzyme appropriée pour l'induction de ruptures de double brin d'ADN au niveau de la séquence de reconnaissance pour l'induction ciblée de ruptures de double brin d'ADN est choisie dans le groupe des endonucléase de ciblage (Homing) constitué par F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI et PI-TliII.

12. Procédé selon l'une quelconque des revendications 8 à 11, **caractérisé en ce que** l'enzyme appropriée pour l'induction de ruptures de double brin d'ADN au niveau de la séquence de reconnaissance pour l'induction ciblée de ruptures de double brin d'ADN est choisie dans le groupe des endonucléases de ciblage (Homing) constitué par les enzymes selon les séquences SEQ ID NO : 2, 4, 6, 8, et 10.

13. Procédé selon l'une quelconque des revendications 8 à 12, **caractérisé en ce que** l'enzyme appropriée pour l'induction de ruptures de double brin d'ADN au niveau de la séquence de reconnaissance pour l'induction ciblée de ruptures de double brin d'ADN est réalisée en utilisant une cassette d'expression qui comporte une séquence d'acide nucléique codant pour ladite enzyme.

14. Procédé selon l'une quelconque des revendications 8 à 13, **caractérisé en ce que** l'enzyme appropriée pour l'induction de ruptures de double brin d'ADN au niveau de la séquence de reconnaissance pour l'induction ciblée de ruptures de double brin d'ADN est réalisée en utilisant une cassette d'expression qui comporte une séquence d'acide nucléique codant pour ladite enzyme selon les séquences SEQ ID NO : 1, 3, 5, 7 ou 9.

15. Organisme végétal, contenant un système recombinant selon l'une quelconque des revendications 1 à 8.

16. Organisme végétal selon la revendication 15, choisi dans le groupe constitué par Arabidopsis thaliana, le tabac, le blé, le seigle, l'orge, l'avoine, le colza, le maïs, la pomme de terre, la betterave sucrière, le soja, le tournesol, la courge ou l'arachide.

17. Cultures cellulaires, organes, tissus, parties ou matériau de reproduction transgénique contenant un système recombinant selon l'une quelconque des revendications 1 à 8.

18. Utilisation d'un organisme végétal contenant un système recombinant selon l'une quelconque des revendications 1 à 8, ou de cultures cellulaires, d'organes, de tissus, de parties ou de matériau de reproduction transgénique, dérivés de celui-ci, contenant un système recombinant selon l'une quelconque des revendications 1 à 8, selon la revendication 17 comme produit alimentaire, aliment pour animaux ou semence ou pour la préparation de produits pharmaceutiques ou de produits chimiques fins.
